(19) Europäisches Patentamt
European Patent Office
Office européen des brevets

(11) **EP 4 122 948 A1**

(12) **EUROPEAN PATENT APPLICATION**
published in accordance with Art. 153(4) EPC


(43) Date of publication:
**25.01.2023 Bulletin 2023/04**

(21) Application number: **21772323.8**

(22) Date of filing: **16.03.2021**

(51) International Patent Classification (IPC):
***C07K 14/435*** (2006.01) ***C08G 81/00*** (2006.01)
***C08H 1/00*** (2006.01) ***C08L 89/00*** (2006.01)
***C08L 101/16*** (2006.01) ***C12N 15/12*** (2006.01)
***C12P 21/02*** (2006.01)

(52) Cooperative Patent Classification (CPC):
**C07K 14/435; C08G 81/00; C08H 1/00; C08L 89/00; C08L 101/16; C12P 21/02**

(86) International application number:
**PCT/JP2021/010699**

(87) International publication number:
**WO 2021/187502 (23.09.2021 Gazette 2021/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2020 JP 2020045850**
**07.08.2020 JP 2020135124**



(71) Applicant: **Spiber Inc.**
**Tsuruoka-shi, Yamagata 997-0052 (JP)**

(72) Inventors:
- **SAKATA, Kazuki**
**Tsuruoka-shi, Yamagata 997-0052 (JP)**
- **KITAHARA, Nao**
**Tsuruoka-shi, Yamagata 997-0052 (JP)**
- **SHIRAKAWA, Seiji**
**Nagasaki-shi, Nagasaki 852-8521 (JP)**
- **TAKAHASHI, Kentaro**
**Tsuruoka-shi, Yamagata 997-0052 (JP)**
- **MUTO, Leona**
**Tsuruoka-shi, Yamagata 997-0052 (JP)**
- **SHAFAAT, Oliver Syed**
**Tsuruoka-shi, Yamagata 997-0052 (JP)**
- **KAGATA, Hideki**
**Tsuruoka-shi, Yamagata 997-0052 (JP)**

(74) Representative: **Handley, Matthew Edward et al**
**Venner Shipley LLP**
**200 Aldersgate**
**London EC1A 4HD (GB)**


Remarks:
The complete document including Reference Table(s) and the Sequence Listing(s) can be downloaded from the EPO website

(54) **SYNTHETIC POLYMER AND METHOD FOR PRODUCING SAME, MOLDING MATERIAL, AND MOLDED BODY**


(57) An aspect of the present disclosure provides a synthetic polymer containing a first segment containing a polypeptide skeleton and one or a plurality of second segments bonded directly to the first segment, in which the second segment contains a molecular group having a plasticizing function for the polypeptide skeleton.


EP 4 122 948 A1

## Description

### Technical Field

The present disclosure relates to a synthetic polymer, a production method of the same, a molding material, and a molded body.

### Background Art

Structural proteins such as silk fibroin and spider silk fibroin are attracting attention as alternatives to structural materials composed of synthetic resins and the like because they can exhibit excellent robustness. In recent years, the development of recombinant technology has led to the development of mass production technology of recombinant structural proteins imitating the structural proteins described above (for example, Patent Literature 1) .

Since a protein has a number of polar groups such as an amide group in the main chain and a carboxy group, an amino group, a hydroxyl group, and a thiol group in the side chain, a large number of hydrogen bonds are formed between molecules. For this reason, it is considered that a molded body obtained by subjecting a structural protein to heat and pressure molding has excellent mechanical strength, but the molded body also has a brittle side surface in which cracks and brokenness are generated by receiving an impact or the like. The circumstances described above are an obstacle to using proteins as alternatives to general-purpose plastics.

### Citation List

### Patent Literature

Patent Literature 1: WO 2015/178466 A

### Summary of Invention

### Technical Problem

When a molded body is formed using a structural protein, it is also conceivable to improve flexibility of the molded body by adding a plasticizer such as a general-purpose plastic. However, the plasticizer is generally hydrophobic and has low compatibility with proteins having a number of polar groups as described above. According to the studies conducted by the present inventors, it has been found that when a plasticizer is mixed with a structural protein to form a molded body, phase separation occurs between the structural protein and the plasticizer, and an expected effect cannot be obtained. In addition, it is desirable to have high flexibility also in a protein molded body produced by a molding technique other than heat and pressure molding of a film, a fiber, or the like using a protein or a structural protein.

An object of the present disclosure is to provide a synthetic polymer capable of producing a molded body which is a polymer material having a peptide skeleton and has excellent flexibility, and a production method of the same. Another object of the present disclosure is to provide a molding material and a molded body each containing the synthetic polymer described above.

### Solution to Problem

An aspect of the present disclosure provides a synthetic polymer containing a first segment containing a polypeptide skeleton and one or a plurality of second segments bonded directly to the first segment, in which the second segment contains a molecular group having a plasticizing function for the polypeptide skeleton.

Since the synthetic polymer contains the second segment, the synthetic polymer as a whole has excellent flexibility while containing a polypeptide skeleton similarly to a protein. In addition, in the synthetic polymer, since the first segment and the second segment are bonded directly to each other, occurrence of macro phase separation as in a case where a plasticizer is simply mixed with a protein is reduced. Therefore, a molded body having excellent flexibility can be produced.

The polypeptide skeleton may be a hydrophobic polypeptide skeleton. An average hydropathy index (degree of hydrophobicity of the hydrophobic polypeptide skeleton may be 0.22 or more. In a case where the polypeptide skeleton is the hydrophobic polypeptide skeleton as described above, the affinity of the molecular group having the plasticizing function with the first segment is improved, and a molded body having more excellent flexibility can be produced.

A molecular weight of the second segment may be a value within a range of 1 to 70 when a molecular weight of the first segment is 100. Therefore, in the molded body obtained using the synthetic polymer, the flexibility of the entire

molded body can be advantageously enhanced while maintaining the characteristics (for example, high mechanical strength) of the first segment.

The second segment may contain a skeleton derived from at least one selected from the group consisting of polyether, polyester, polycarbonate, polyamide, polyol, polyolefin, polyacetal, polyketal, poly(meth)acrylate, silicone, polyurethane, polyalkyleneimine, a phenolic resin, a urea resin, a melamine resin, and polysaccharides.

The second segment may include at least one selected from the group consisting of a polyether group, a polyester group, a polycarbonate group, a polyamide group, a polyol group, and a modified polysaccharide group.

A plurality of first segments may be contained, and some of the second segments may be bonded to two or more first segments to form a network structure. By having the network structure, for example, a molded body formed of the synthetic polymer can exhibit a large elongation. In addition, by having the network structure, for example, a molded body formed of the synthetic polymer can be imparted with a function as an elastic body.

A plurality of first segments may be contained, and the first segment and the second segment may be alternatively bonded. By alternately bonding the first segment and the second segment, for example, a molded body formed of the synthetic polymer can exhibit a large elongation.

The second segment may contain a plurality of molecular groups, and the plurality of molecular groups may be linked to each other. A designed width of the second segment can be further expanded, and the flexibility of the synthetic polymer can be more easily adjusted.

The second segment may further contain a linker (reactive functional group), and the molecular group and the polypeptide may be bonded via the linker.

The linker may include at least one selected from the group consisting of a structural unit represented by the following Formula (1), structural units represented by the following General Formulas (2a) to (6), a structural unit represented by the following Formula (7), structural units represented by the following General Formulas (8a) to (9), structural units represented by the following General Formulas (10) to (11b), and structural units represented by the following General Formulas (13) to (16).

[Chem. 1]

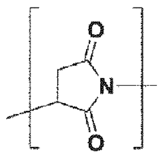

(1)

[Chem. 2]

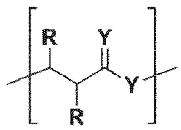

(2a)

[Chem. 3]

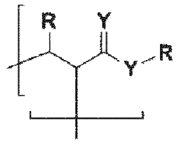

(2b)

[Chem. 4]

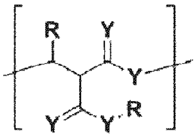

(3a)

[Chem. 5]

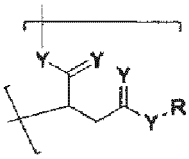

(4a)

[Chem. 6]

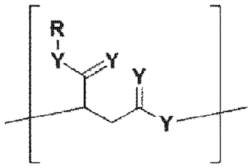

(4b)

[Chem. 7]

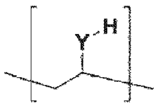

(5)

[Chem. 8]

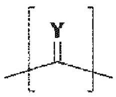

(6)

[Chem. 9]

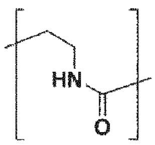

(7)

[Chem. 10]

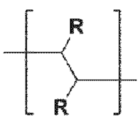

(8a)

[Chem. 11]

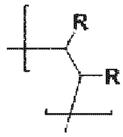

(8b)

[Chem. 12]

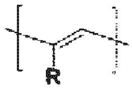

(9)

[Chem. 13]

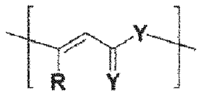

(10)

[Chem. 14]

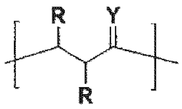

(11a)

[Chem. 15]

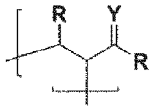

(11b)

[Chem. 16]

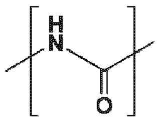

(13)

[Chem. 17]

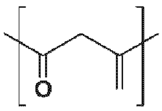

(14)

[Chem. 18]

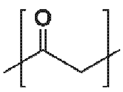

(15)

[Chem. 19]

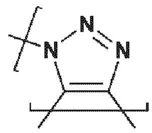

(16)

In General Formulas (2a), (2b), (3a), (4a), (4b), (5), (6), (10), (11a), and (11b), Y's each independently represent an oxygen atom, a sulfur atom, or $NR^1$, and $R^1$ represents hydrogen, a hydrocarbon group, an aromatic group, a carbonyl group, or a sulfonyl group, and in General Formulas (2a), (2b), (3a), (4a), (4b), (8a), (8b), (9), (10), (11a), and (11b), R's each independently represent hydrogen, a hydrocarbon group, or an aromatic group.

The linker may be at least one selected from the group consisting of a structural unit represented by Formula (1) and structural units represented by General Formulas (2a), (3a), (4a), (4b), and (10).

The polypeptide skeleton may include a skeleton derived from an artificial protein.

The artificial protein may include an artificial structural protein.

The artificial structural protein may include artificial fibroin.

The artificial fibroin may include artificial modified spider silk fibroin.

An aspect of the present disclosure provides a molding material containing the synthetic polymer described above.

Since the molding material contains the synthetic polymer described above, a molded body having excellent flexibility can be produced.

An aspect of the present disclosure provides a molded body containing the synthetic polymer described above.

The molded body has excellent flexibility because it contains the synthetic polymer described above.

An aspect of the present disclosure provides a production method of a synthetic polymer, the method including a step of obtaining a synthetic polymer by reacting a compound containing a polypeptide skeleton with a compound represented by any one of the following General Formulas (1A) to (16A), in which the polypeptide skeleton includes at least one selected from the group consisting of a thiol group, an amino group, a hydroxy group, a guanidino group, a carboxy group, a phenoxy group, an indole group, an amide group, an azide group, and an alkynyl group.

[Chem. 20]

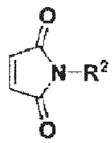

(1A)

[Chem. 21]

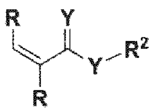

(2A)

[Chem. 22]

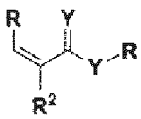

(2B)

[Chem. 23]

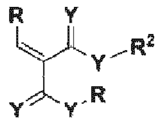

(3A)

[Chem. 24]

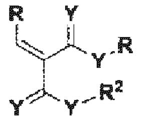

(3B)

[Chem. 25]

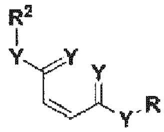

(4A)

[Chem. 26]

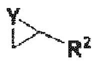

(5A)

[Chem. 27]

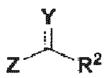

(6A)

[Chem. 28]

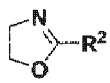

(7A)

[Chem. 29]

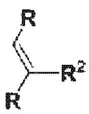

(8A)

[Chem. 30]

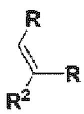

(8B)

[Chem. 31]

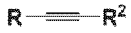

(9A)

[Chem. 32]

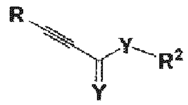

(10A)

[Chem. 33]

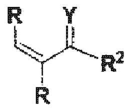

(11A)

[Chem. 34]

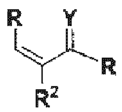

(11B)

[Chem. 35] **Z-$R^2$ (12A)**

[Chem. 36] **OCN-$R^2$ (13A)**

[Chem. 37]

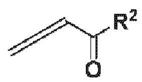

(14A)

[Chem. 38]

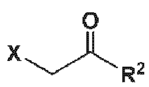

(15A)

[Chem. 39] **$N_3$-$R^2$ (16A)**

In General Formulas (1A), (2A), (2B), (3A), (3B), (4A), (5A), (6A), (7A), (8A), (8B), (9A), (10A), (11A), (11B), and (12A), $R^2$ represents a molecular group having a plasticizing function for the polypeptide skeleton, in General Formulas (2A), (2B), (3A), (3B), (4A), (5A), (6A), (8B), (9A), (10A), (11A), and (11B), Y's each independently represent an oxygen atom, a sulfur atom, or $NR^1$, and $R^1$ represents hydrogen, a hydrocarbon group, an aromatic group, a carbonyl group, or a sulfonyl group, in General Formulas (2A), (2B), (3A), (3B), (4A), (5A), (8A), (8B), (9A), (10A), (11A), and (11B), R's each independently represent hydrogen, a hydrocarbon group, or an aromatic group, in General Formulas (6A) and (12A), Z represents a halogen atom, a sulfonic acid ester group, or a fluorine-containing carboxylic acid ester group, and in General Formula (15A), X represents a halogen atom.

In the production method of a synthetic polymer, a synthetic polymer is prepared by reacting a compound containing a polypeptide skeleton containing a specific functional group with a specific compound containing a molecular

group having a plasticizing function for the polypeptide skeleton and represented by any one of the following General Formulas (1A) to (16A). By doing so, the obtained synthetic polymer is formed of a material that has a molecular group having a plasticizing function for the polypeptide skeleton, has a polypeptide skeleton similarly to a protein, and has excellent flexibility as a whole synthetic polymer. Therefore, a molded body having excellent flexibility can be produced. In addition, in the synthetic polymer, since the first segment and the second segment are bonded to each other, occurrence of macro phase separation as in a case where a plasticizer is simply mixed with a protein is reduced.

The reaction between the compound containing the polypeptide skeleton and the compound represented by any one of the following General Formulas (1A) to (12A) and (14A) may be a Michael addition reaction.

The polypeptide skeleton may be a hydrophobic polypeptide skeleton. An average hydropathy index of the hydrophobic polypeptide skeleton may be 0.22 or more.

The molecular group having the plasticizing function for the polypeptide skeleton may have a value within a range of 1 to 70 when a molecular weight of the polypeptide skeleton is 100.

The compound containing the polypeptide skeleton may contain an artificial protein.

The artificial protein may include an artificial structural protein.

An aspect of the present disclosure provides a production method of a synthetic polymer, the method including a step of obtaining a synthetic polymer by reacting a compound containing a polypeptide skeleton with a compound containing a molecular group having a plasticizing function for the polypeptide skeleton and at least a compound having two or more structural units selected from the group consisting of structural units represented by the following General Formulas (2-1A) to (2-16A), in which the polypeptide skeleton includes at least one selected from the group consisting of a thiol group, an amino group, a hydroxy group, a guanidino group, a carboxy group, a phenoxy group, an indole group, an amide group, an azide group, and an alkynyl group, and the compound containing a molecular group includes at least one selected from the group consisting of a thiol group, an amino group, a hydroxy group, a guanidino group, a carboxy group, a phenoxy group, an indole group, an amide group, an azide group, and an alkynyl group.

[Chem. 40]

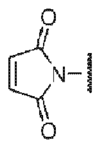

(2-1A)

[Chem. 41]

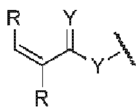

(2-2A)

[Chem. 42]

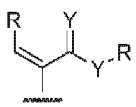

(2-2B)

[Chem. 43]

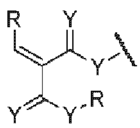

(2-3A)

[Chem. 44]

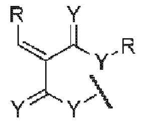

(2-3B)

[Chem. 45]

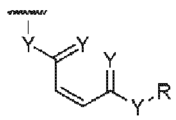

(2-4B)

[Chem. 46]

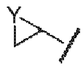

(2-5A)

[Chem. 47]

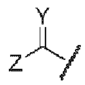

(2-6A)

[Chem. 48]

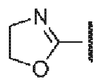

(2-7A)

[Chem. 49]

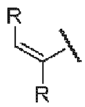

(2-8A)

[Chem. 50]

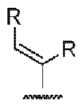

(2-8B)

[Chem. 51]

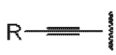

(2-9A)

[Chem. 52]

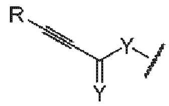 (2-10A)

[Chem. 53]

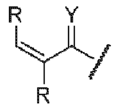 (2-11A)

[Chem. 54]

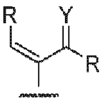 (2-11B)

[Chem. 55]

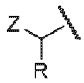 (2-12A)

[Chem. 56]

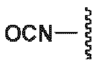 (2-13A)

[Chem. 57]

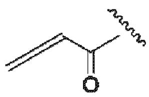 (2-14A)

[Chem. 58]

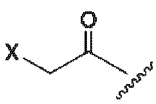 (2-15A)

[Chem. 59]

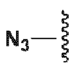 (2-16A)

In General Formulas (2-2A), (2-2B), (2-3A), (2-3B), (2-4A), (2-5A), (2-6A), (2-10A), (2-11A), and (2-11B), Y's each independently represent an oxygen atom, a sulfur atom, or $NR^1$, and $R^1$ represents hydrogen, a hydrocarbon group, an aromatic group, a carbonyl group, or a sulfonyl group, in General Formulas (2-2A), (2-2B), (2-3A), (2-3B), (2-4A), (2-8A), (2-8B), (2-9A), (2-10A), (2-11A), (2-11B), and (2-12A), R's each independently represent hydrogen, a hydrocarbon group, or an aromatic group, in General Formulas (2-6A) and (2-12A), Z represents a halogen atom, a sulfonic acid ester group, or a fluorine-containing carboxylic acid ester group, and in General Formula (2-15A), X represents a halogen atom.

In the production method of a synthetic polymer, a synthetic polymer is prepared by reacting a compound containing a polypeptide skeleton having a specific functional group with a compound containing a molecular group having a plasticizing function for the polypeptide skeleton and having a specific functional group, and at least a compound having two or more structural units selected from the group consisting of structural units represented by General Formulas (2-1A) to (2-16A). By doing so, the obtained synthetic polymer is formed of a material that has a molecular group having a plasticizing function for the polypeptide skeleton, has a polypeptide skeleton similarly to a protein, and has excellent flexibility as a whole synthetic polymer. Therefore, a molded body having excellent flexibility can be produced. In addition, in the synthetic polymer, since the first segment and the second segment are bonded to each other, occurrence of macro phase separation as in a case where a plasticizer is simply mixed with a protein is reduced.

The polypeptide skeleton may be a hydrophobic polypeptide skeleton. An average hydropathy index of the hydrophobic polypeptide skeleton may be 0.22 or more.

A molecular weight of the molecular group having the plasticizing function for the polypeptide skeleton may be a value within a range of 1 to 70 when a molecular weight of the polypeptide skeleton is 100.

## Advantageous Effects of Invention

According to the present disclosure, it is possible to provide a synthetic polymer capable of producing a molded body having excellent flexibility as a polymer material having a peptide skeleton. According to the present disclosure, it is possible to provide a molding material and a molded body each containing the synthetic polymer.

## Brief Description of Drawings

Fig. 1 is a schematic view illustrating an example of a domain sequence of artificial fibroin.
Fig. 2 is a view illustrating a distribution of values of z/w (%) in naturally derived fibroin.
Fig. 3 is a view illustrating a distribution of values of x/y (%) in naturally derived fibroin.
Fig. 4 is a schematic view illustrating an example of a domain sequence of artificial fibroin.
Fig. 5 is a schematic view illustrating an example of a domain sequence of fibroin.
Fig. 6 is a view illustrating measurement results of SDS-PAGE for molding materials prepared in Examples.
Fig. 7 is a view illustrating measurement results of SDS-PAGE for molding materials prepared in Examples.
Fig. 8 is a schematic cross-sectional view of a pressure molding machine used in Examples.
Fig. 9 is a schematic view illustrating a method of using the pressure molding machine illustrated in Fig. 8, in which (a), (b), and (c) are schematic cross-sectional views of the pressure molding machine before introduction of a composition, after the introduction of the composition, and in a state where the composition is heated and pressurized, respectively.
Fig. 10 is a photograph showing an appearance of a molded body molded using a synthetic polymer of Example 1.
Fig. 11 is a photograph showing an appearance of a molded body molded using a mixture of Comparative Example 2.
Fig. 12 is a photograph showing an appearance of a molded body molded using a mixture of Comparative Example 3.
Fig. 13 is a photograph showing an appearance of fibers prepared in Example 12.
Fig. 14 is a graph showing results of GPC measurement for a synthetic polymer prepared in Example 13.
Fig. 15 is a graph showing results of GPC measurement for a synthetic polymer prepared in Example 14.
Fig. 16 is a graph showing results of GPC measurement for a synthetic polymer prepared in Example 15.
Fig. 17 is a graph showing results of GPC measurement for a synthetic polymer prepared in Example 16.
Fig. 18 is a graph showing results of GPC measurement for a synthetic polymer prepared in Example 17.
Fig. 19 is a graph showing results of GPC measurement in Reference Example 2.
Fig. 20 is a photograph showing results of contact angle measurement in Reference Example 3.

## Description of Embodiments

Hereinafter, modes for carrying out the present disclosure will be described in detail with reference to the drawings in some cases. However, the following embodiments are examples for describing the present disclosure, and are not intended to limit the present disclosure to the following contents.

The materials exemplified in the present specification can be used alone or in combination of two or more thereof unless otherwise specified. Contents of the respective components in a composition mean the total amount of a plurality of substances present in the composition, unless otherwise specified, when a plurality of substances corresponding to the respective components in the composition are present.

In the present specification, a molding material refers to a material used for producing a molded body. The molding material according to the present disclosure can also be a protein material obtained by chemically modifying a recombinant structural protein. In the present specification, the shape of the molded body is not particularly limited, and may be, for example, a film shape, a plate shape, a block shape, a sponge shape, a fiber shape, or the like. In the present specification, the form of the molding material is not limited at all, and may be, for example, a powder, a particle, a liquid, a gel, or the like. In addition, the molding material according to the present disclosure can be formed into molded bodies having various shapes by, for example, heat-and-pressure molding, cast molding, spinning, and the like. At the time of molding, a mold may be used as necessary.

[Synthetic polymer]

An embodiment of the synthetic polymer contains a first segment containing a polypeptide skeleton and one or a plurality of second segments bonded to the first segment. The second segment contains a molecular group having a plasticizing function for the polypeptide skeleton. The number of second segments is one or plural, preferably 2 or more, more preferably 2 to 10, still more preferably 2 to 8, still more preferably 2 to 6, and most preferably 2 to 4, with respect to one first segment.

The synthetic polymer may contain a first segment and a second segment. For example, one or a plurality of second segments may be bonded to one first segment, or a plurality of blocks containing a first segment and a second segment bonded to the first segment may be connected. That is, the synthetic polymer may be a polymer (for example, a graft polymer or the like) containing a first segment containing a polypeptide skeleton as a main chain and a second segment as a side chain, or may be a block polymer containing a first segment and a second segment. That is, a plurality of first segments may be contained, and the first segment and the second segment may be alternatively bonded. By alternately bonding the first segment and the second segment, for example, a molded body formed of the synthetic polymer can exhibit a large elongation.

In addition, the synthetic polymer may contain a plurality of first segments, and some of the second segments may be bonded to two or more first segments to form a network structure. By having the network structure, for example, a molded body formed of the synthetic polymer can exhibit a large elongation. In addition, by having the network structure, for example, a molded body formed of the synthetic polymer can be imparted with a function as an elastic body.

The first segment and the second segment may be bonded directly to each other, or may be bonded by a structure in which the first segment can be bonded to the second segment. Note that in any of these bonding forms, the bonding between the first segment and the second segment may be covalent bonding.

(First segment)

The first segment may be bonded to the second segment by a functional group (for example, a thiol group of cysteine or the like) in an amino acid sequence constituting the polypeptide skeleton. In other words, any polypeptide having a functional group capable of binding to the second segment can be employed as the polypeptide skeleton. At this time, the number of the functional groups of the polypeptide may be, for example, 1 or more, 2 or more, or 4 or more. The number of the functional groups of the polypeptide may be, for example, 30 or less, 25 or less, 20 or less, 15 or less, 10 or less, or 8 or less. The number of the functional groups of the polypeptide can be adjusted within the above range, and may be, for example, 1 to 30, 1 to 25, 1 to 20, 1 to 15, 1 to 10, 1 to 8, or 2 to 8. The first segment may also be bonded to the second segment by a functional group introduced to a functional group in an amino acid sequence constituting the polypeptide skeleton.

The functional group as described above may be, for example, at least one selected from the group consisting of a thiol group, an amino group, a hydroxy group, a guanidino group, a carboxy group, a phenoxy group, an indole group, an amide group, an azide group, and an alkynyl group, preferably at least one selected from the group consisting of a thiol group, an amino group, a hydroxy group, a guanidino group, a carboxy group, a phenoxy group, an indole group, and an amide group, more preferably at least one selected from the group consisting of a thiol group, an amino group, a hydroxy group, a guanidino group, a carboxy group, a phenoxy group, and an indole group, still more preferably at least one selected from the group consisting of a thiol group, an amino group, a hydroxy group, a guanidino group, a carboxy group, and a phenoxy group, still more preferably at least one selected from the group consisting of a thiol group, an amino group, a hydroxy group, a guanidino group, and a carboxy group, still more preferably at least one selected from the group consisting of a thiol group, an amino group, a hydroxy group, and a guanidino group, particularly preferably at least one selected from the group consisting of a thiol group, an amino group, and a hydroxy group, particularly preferably at least one selected from the group consisting of a thiol group and an amino group, and most preferably a thiol group. The amino group may be, for example, an amino group of lysine. The hydroxy group may be, for example, a hydroxy group of serine, a hydroxy group of threonine, or the like. The guanidino group may be, for example, a guanidino group of arginine. The carboxy group may be, for example, a carboxy group of glutamic acid, a

carboxy group of aspartic acid, or the like. The phenoxy group may be, for example, a phenoxy group of tyrosine. The indole group may be, for example, an indole group of tryptophan. The amide group may be, for example, an amide group of glutamine, an amide group of asparagine, or the like. The alkynyl group may be an alkynyl group introduced by reacting halogenated acetylene with a thiol group of cysteine.

The first segment contains a polypeptide skeleton, and may, for example, consist only of the polypeptide skeleton. The polypeptide skeleton may include a skeleton derived from an artificial protein, or may be composed only of a skeleton derived from an artificial protein. The artificial protein may include artificial fibroin or may consist only of artificial fibroin. When the polypeptide skeleton includes a skeleton derived from a recombinant protein, the number and position of functional groups in the amino acid sequence constituting the polypeptide skeleton can be arbitrarily adjusted. Therefore, the structures and characteristics of the synthetic polymer and the molding material can be designed. In addition, since the recombinant protein includes artificial fibroin, fibrillation of the synthetic polymer is facilitated. Therefore, it is possible to obtain advantages such as an improvement in fiber forming ability when the synthetic polymer is used as a molding material.

A molecular weight of the first segment is, for example, preferably 200 to 1,000,000, more preferably 300 to 900,000, still more preferably 400 to 800,000, still more preferably 500 to 700,000, still more preferably 600 to 600,000, still more preferably 1,000 to 600,000, still more preferably 3,000 to 600,000, still more preferably 5,000 to 600,000, still more preferably 10,000 to 600,000, and still more preferably 5,000 to 100,000. Note that when the molecular weight of the first segment is less than 200, the first segment that functions as a hard segment may become too small with respect to the second segment (soft segment) containing a molecular group having a plasticizing function. In such a case, the rigidity of the molded body molded using the synthetic polymer (molding material) may be reduced, and it may be difficult to use the molded body as, for example, a structural body. On the other hand, when the molecular weight of the first segment is more than 1,000,000, the reactivity of the coupling reaction is reduced, such that the reaction cannot be completed in a time when the first segment can be chemically produced as a material in a commercial manner, and as a result, the unreacted second segment remaining in the molded body may be localized. In addition, the molecular weight of the first segment and a molecular weight of the polypeptide skeleton contained in the first segment may be, for example, 1,000 or more, 2,000 or more, 3,000 or more, 4,000 or more, 5,000 or more, 6,000 or more, 7,000 or more, 8,000 or more, 9,000 or more, 10,000 or more, 20,000 or more, 30,000 or more, 40,000 or more, 50,000 or more, 60,000 or more, 70,000 or more, 80,000 or more, 90,000 or more, or 100,000 or more. Further, the molecular weight of the first segment and the molecular weight of the polypeptide skeleton contained in the first segment may be 400,000 or less, less than 360,000, 300,000 or less, or 200,000 or less. The solubility of the first segment or the polypeptide skeleton in the solvent tends to increase as the molecular weight of each of the first segment or the polypeptide skeleton is decreased. Therefore, in a case where the molecular weight of the polypeptide skeleton or the first segment is, for example, 200,000 or less or 100,000 or less, when a compound containing a polypeptide skeleton is dissolved in a solvent and is reacted with a compound containing a molecular group having a plasticizing function for the polypeptide skeleton to obtain a synthetic polymer, it can be desired to improve generation efficiency of the synthetic polymer to be targeted. In addition, in a molded body molded using the synthetic polymer obtained as described above, improvement in flexibility can be expected while securing a certain degree of strength. In addition, when the molecular weight of the polypeptide skeleton or the first segment is, for example, 200,000 or less or 100,000 or less, it is expected that the amount of the second segment used can be suppressed as much as possible in order to enhance the flexibility of the synthetic polymer. Note that the molecular weight of the first segment and the molecular weight of the polypeptide skeleton are weight average molecular weights.

In the present specification, the molecular weight is a value measured by sodium dodecyl sulfate-polyacrylamide gel electrophoresis (SDS-PAGE). Note that the electrophoresis is performed in the following procedure. That is, first, 200 μL of 2 M lithium chloride DMSO (manufactured by FUJIFILM Wako Pure Chemical Corporation) is added to 2 mg of a powder sample, and the mixture is stirred while being heated at 80°C for 60 minutes and further at 95°C for 10 minutes to dissolve the sample. Thereafter, the sample is diluted 50 times with a 10 M urea solution, further diluted twice with a sample buffer (manufactured by FUJIFILM Wako Pure Chemical Corporation), and heated at 95°C for 5 minutes to denaturalize the protein. Next, a gel for SDS-PAGE (manufactured by Bio-lad) is attached to an electrophoresis device (manufactured by Bio-lad), the device is filled with an SDS buffer, and the electrophoresis device is connected to a power supply device (manufactured by Bio craft). 10 μL of the denatured sample is added to each well of the gel for SDS-PAGE, and a current of 30 mA/1 sheet is allowed to flow for 30 minutes. After completion of the electrophoresis, the gel for SDS-PAGE is taken out from the device, immersed in Oriole fluorescent gel stain (manufactured by Bio-lad), and shaken for 1 hour. Thereafter, the gel is placed on a UV sample tray (manufactured by Bio-lad), and a stained image is acquired with a Gel Doc EZ gel imaging device (manufactured by Bio-lad) .

<Artificial protein>

An example of the artificial protein can include any protein that can be produced in an industrial scale, and an

example thereof can include a protein that can be used for industrial purposes. The term "can be used for industrial purposes" means, for example, that it can be used for various general-purpose materials used indoors or outdoors. A specific example of the protein that can be used for industrial purposes can include a structural protein. In addition, specific examples of the structural protein can include spider silk, silkworm silk, keratin, collagen, elastin, resilin, and proteins derived from them. As the protein to be used, artificial fibroin is preferable, and artificial spider silk fibroin (artificial modified spider silk fibroin) is more preferable.

Note that the artificial protein includes a recombinant protein and a synthetic protein. That is, in the present specification, the "artificial protein" means a protein artificially produced. The artificial protein may be a protein having a domain sequence different from an amino acid sequence of a naturally derived protein, or may be a protein having a domain sequence identical to an amino acid sequence of a naturally derived protein. In addition, the "artificial protein" may be a protein obtained by using an amino acid sequence of a naturally derived protein as it is, a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence). Note that since the artificial protein can freely design an amino acid sequence unlike a natural protein, when the artificial protein is contained in a synthetic polymer contained in a molding material or a molded body of the present embodiment described below, the function, characteristics, physical properties, and the like of the molding material or the molded body can be arbitrarily controlled by appropriately designing the amino acid sequence of the artificial protein. In addition, since uniform molecular design is always possible, a protein that has high homology with a target protein and is suitable for a purpose can be stably obtained. As a result, it is possible to advantageously stabilize the quality of a target synthetic polymer, and eventually, a molding material or a molded body obtained using the synthetic polymer.

The polypeptide according to the present embodiment may contain 50 or more amino acid residues. The number of amino acid residues is, for example, 100 or more, 150 or more, 200 or more, 250 or more, 300 or more, 350 or more, 400 or more, 450 or more, or 500 or more. The number of amino acid residues is, for example, 5,000 or less, 4,500 or less, 4,000 or less, 3,500 or less, 3,000 or less, 2,500 or less, 2,000 or less, 1,500 or less, or 1,000 or less. As the number of amino acid residues is smaller, the solubility in the solvent tends to be increased. Therefore, in a case where the number of amino acid residues of the polypeptide according to the present embodiment is, for example, 5,000 or less or 2,500 or less, when a compound containing a polypeptide skeleton is dissolved in a solvent and is reacted with a compound containing a molecular group having a plasticizing function for the polypeptide skeleton to obtain a synthetic polymer, it can be desired to improve generation efficiency of the synthetic polymer to be targeted. In addition, in a molded body formed using the synthetic polymer obtained as described above, improvement in flexibility can be expected while securing a certain degree of strength.

(Polypeptide skeleton)

The polypeptide skeleton may be, for example, a hydrophobic polypeptide skeleton. In a case where the polypeptide skeleton is the hydrophobic polypeptide skeleton, the affinity of the molecular group having the plasticizing function with the first segment is improved, and a molded body having more excellent flexibility can be produced. In addition, the water resistance of such a molded body is improved, and for example, in a case where the molded body is used as a general-purpose material for industrial purposes, the use life can be advantageously extended. In addition, in the synthetic polymer according to the present embodiment, for example, the hydrophobicity or hydrophilicity of the entire synthetic polymer can be arbitrarily adjusted by controlling the hydrophobicity or hydrophilicity of the molecular group having the plasticizing function for the polypeptide skeleton contained in the second segment. However, in a case where the polypeptide skeleton is a hydrophobic polypeptide skeleton, the entire synthetic polymer can be shifted to the hydrophobic side as compared with a case where the polypeptide skeleton is a hydrophilic polypeptide skeleton, and thus the hydrophobicity or hydrophilicity of the entire synthetic polymer can be controlled over a wider range.

The hydrophobicity of the hydrophobic polypeptide skeleton can be determined using a value of the average hydropathy index described below as an index. The value of the average hydropathy index of the hydrophobic polypeptide skeleton may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. In addition, an upper limit value thereof is not particularly limited, and may be, for example, 1.00 or less or 0.7 or less.

The hydrophobic polypeptide skeleton preferably has low solubility in a lithium bromide aqueous solution (concentration: 9 M) at 60°C. This solubility can be evaluated using a compound (polypeptide) corresponding to the hydrophobic polypeptide skeleton or a polypeptide obtained by decomposing a synthetic polymer and isolating only a hydrophobic polypeptide skeleton. In a case where the polypeptide is dissolved in a lithium bromide aqueous solution (concentration: 9 M) at 60°C, a maximum concentration may be, for example, less than 30 mass%, less than 25 mass%, less than 20 mass%, less than 15 mass%, less than 10 mass%, less than 5 mass%, or less than 1 mass%. Note that

the hydrophobic polypeptide skeleton may be completely insoluble in a lithium bromide aqueous solution (concentration: 9 M) at 60°C.

The hydrophobic polypeptide skeleton preferably has a large water contact angle. The water contact angle can be evaluated using a compound (polypeptide) corresponding to the hydrophobic polypeptide skeleton on a substrate or using a membrane, the membrane being formed of a polypeptide obtained by decomposing a synthetic polymer and isolating only a hydrophobic polypeptide skeleton. The hydrophobic polypeptide skeleton is preferably a polypeptide constituting a membrane with a contact angle of 55° or more after 5 seconds of water being added dropwise to the membrane. The contact angle may be, for example, 60° or more, 65° or more, or 70° or more.

The hydrophobic polypeptide skeleton preferably has excellent hot water resistance. The hot water resistance can be evaluated using a compound (polypeptide) corresponding to the hydrophobic polypeptide skeleton or a polypeptide obtained by decomposing a synthetic polymer and isolating only a hydrophobic polypeptide skeleton. The hydrophobic polypeptide skeleton is preferably a polypeptide that is not decomposed even when a dispersion in which a content of the polypeptide is 5 mass% is prepared, the dispersion containing the polypeptide and water, and the dispersion is heated at 100°C for 5 hours.

The polypeptide skeleton according to the present embodiment may be a structural protein. The structural protein means a protein related to the structure of a living body, a protein included in a structure created by a living body, or a protein derived from those proteins. The structural protein also refers to a protein that self-aggregates under predetermined conditions to form a structure such as a fiber, a film, a resin, a gel, a micelle, or a nanoparticle. Examples of the structural protein in nature include fibroin, keratin, collagen, elastin, and resilin.

The structural protein may be an artificial structural protein. In the present specification, the "artificial structural protein" means a structural protein artificially produced. The artificial structural protein may be a structural protein produced microbially by genetic recombination, and also includes a structural protein obtained by modifying an amino acid sequence from the viewpoint of moldability and productivity. The artificial structural protein is not limited to a sequence of a naturally derived structural protein.

When an artificial structural protein is artificially molded, an amino acid having a smaller side chain is more likely to be hydrogen-bonded, and a molded body having higher strength is more likely to be obtained. In addition, since the alanine residues and the glycine residues are amino acids having nonpolar side chains, the alanine residues and the glycine residues are arranged to face inward in a folding process during the polypeptide production and tend to have $\alpha$-helix structure or $\beta$-sheet structure. Therefore, it is desirable that a proportion of amino acids such as a glycine residue, an alanine residue, and a serine residue is high. From the viewpoint of obtaining a molded body having more excellent strength, a content of the alanine residues may be, for example, 10 to 40%, and may be 12 to 40%, 15 to 40%, 18 to 40%, 20 to 40%, or 22 to 40%. From the viewpoint of obtaining a molded body having more excellent strength, a content of the glycine residues may be, for example, 10 to 55%, and may be 11 to 55%, 13 to 55%, 15 to 55%, 18 to 55%, 20 to 55%, 22 to 55%, or 25 to 55%.

Note that in the present specification, the "content of the alanine residues" is a value represented by the following Formula.

Content of alanine residues = (number of alanine residues contained in polypeptide/number of all amino acid residues of polypeptide) × 100 (%)

In addition, the content of the glycine residues, the content of the serine residues, the content of the threonine residues, the content of the proline residues, and the content of the tyrosine residues have the same meanings as those obtained by replacing the alanine residue with the glycine residue, the serine residue, the threonine residue, the proline residue, and the tyrosine residue, respectively, in the above equation.

From the viewpoint of improving processability, it is important to inhibit strong hydrogen bonding between molecules at the time of processing, and from this viewpoint, it is desirable that an amino acid having a large side chain or an amino acid having flexibility is uniformly contained in the entire sequence to a certain extent. Specifically, a motif containing a tyrosine residue, a threonine residue, or a proline residue may be repeated and inserted in a cycle. For example, a total content of the proline residues, the threonine residues, and the tyrosine residues in arbitrary 20 consecutive amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. In addition, for example, the total content of the proline residues, the threonine residues, and the tyrosine residues in arbitrary 20 consecutive amino acid residues may be 50% or less, 40% or less, 30% or less, or 20% or less.

In the artificial structural protein according to an embodiment, a total of the content of the serine residues, the

content of the threonine residues, and the content of the tyrosine residues may be 4% or more, 4.5% or more, 5% or more, 5.5% or more, 6% or more, 6.5% or more, or 7% or more. The total of the content of the serine residues, the content of the threonine residues, and the content of the tyrosine residues may be, for example, 35% or less, 33% or less, 30% or less, 25% or less, or 20% or less.

The artificial structural protein according to an embodiment may have a repeating sequence. That is, the polypeptide according to the present embodiment may have a plurality of amino acid sequences (repeating sequence units) having high sequence identity in the polypeptide. The number of amino acid residues of the repeating sequence units is preferably 6 to 200. In addition, a sequence identity between the repeating sequence units may be, for example, 85% or more, 90% or more, 95% or more, 96% or more, 97% or more, 98% or more, or 99% or more. In addition, a degree of hydrophobicity (hydropathy index) of the repeating sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. Note that an upper limit value of the degree of hydrophobicity of the repeating sequence unit is not particularly limited, and may be, for example, 1.0 or less or 0.7 or less.

The artificial structural protein according to an embodiment may include an (A)n motif. In the present specification, the (A)n motif means an amino acid sequence mainly containing alanine residues. The number of amino acid residues in the (A)n motif may be 2 to 27 and may be an integer of 2 to 20, 2 to 16, or 2 to 12. In addition, a proportion of the number of alanine residues to the total number of amino acid residues in the (A)n motif is 40% or more and may also be 60% or more, 70% or more, 80% or more, 83% or more, 85% or more, 86% or more, 90% or more, 95% or more, or 100% (which means that the (A)n motif consists of alanine residues).

The artificial structural protein preferably has an amino acid sequence corresponding to an amino acid sequence in which a cysteine residue is inserted at a position adjacent to a glycine residue, a serine residue, or an alanine residue, and more preferably has an amino acid sequence corresponding to an amino acid sequence in which a cysteine residue is inserted at a position adjacent to a glycine residue. The cysteine residue has a mercapto group (also referred to as a thiol group or a sulfhydryl group), and the selectivity of the mercapto group can facilitate binding to the second segment. Therefore, depending on the insertion position of the cysteine residue in the artificial structural protein, the binding position of the second segment to the polypeptide skeleton of the first segment can be set to any position. Note that the cysteine residue may be located between the glycine residue, the serine residue, or the alanine residue and the glycine residue, the serine residue, or the alanine residue, or may be located between the serine residue and the glycine residue.

The artificial structural protein preferably has an amino acid sequence corresponding to an amino acid sequence in which a cysteine residue is inserted at a position adjacent to a hydrophobic amino acid residue. In this case, the hydrophobic amino acid residues are immobilized by a hydrophobic interaction between molecules. The cysteine residue may be located next to the hydrophobic amino acid residue, may be located between the hydrophobic amino acid residue and an amino acid residue other than the hydrophobic amino acid residue, may be located between the hydrophobic amino acid residue and the glycine residue, the serine residue, or the alanine residue, or may be located between the hydrophobic amino acid residue and the glycine residue. The hydrophobic amino acid residue may be one selected from the group consisting of an isoleucine residue, a valine residue, a leucine residue, a phenylalanine residue, a methionine residue, and an alanine residue.

As the artificial structural protein, artificial fibroin is preferable. An example of the fibroin includes naturally derived fibroin. Examples of the naturally derived fibroin include fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

A more specific example of the fibroin produced by insects includes a silkworm fibroin L chain (GenBank Accession No. M76430 (base sequence), AAA27840.1 (amino acid sequence)).

Examples of the fibroin produced by arachnids include spider silk proteins produced by spiders belonging to the genus *Araneus,* such as *Araneus ventricosus*, *Araneus diadematus*, *Araneus pinguis*, *Araneus pentagrammicus,* and *Araneus nojimai*, spiders belonging to the genus *Neoscona*, such as *Neoscona scylla*, *Neoscona nautica*, *Neoscona adianta*, and *Neoscona scylloides*, spiders belonging to the genus *Pronus*, such as *Pronous minutus*, spiders belonging to the genus *Cyrtarachne*, such as *Cyrtarachne bufo* and *Cyrtarachne inaequalis*, spiders belonging to the genus *Gasteracantha,* such as *Gasteracantha kuhlii* and *Gasteracantha mammosa*, spiders belonging to the genus *Ordgarius*, such as *Ordgarius hobsoni* and *Ordgarius sexspinosus*, spiders belonging to the genus *Argiope*, such as *Argiope amoena*, *Argiope minuta*, and *Argiope bruennichi,* spiders belonging to the genus *Arachnura*, such as *Arachnura logio*, spiders belonging to the genus *Acusilas*, such as Acusilas coccineus, spiders belonging to the genus *Cytophora*, such as *Cyrtophora moluccensis*, *Cyrtophora exanthematica*, and *Cyrtophora unicolor*, spiders belonging to the genus *Poltys*, such as *Poltys illepidus*, spiders belonging to the genus *Cyclosa*, such as *Cyclosa octotuberculata*, *Cyclosa sedeculata*, *Cyclosa vallata*, and *Cyclosa atrata*, and spiders belonging to the genus *Chorizopes*, such as *Chorizopes nipponicus*,

and spider silk proteins produced by spiders belonging to the family *Tetragnathidae*, such as spiders belonging to the genus *Tetragnatha*, such as *Tetragnatha praedonia*, *Tetragnatha maxillosa*, *Tetragnatha extensa*, and *Tetragnatha squamata*, spiders belonging to the genus *Leucauge*, such as *Leucauge magnifica*, *Leucauge blanda*, and *Leucauge subblanda*, spiders belonging to the genus *Nephila*, such as *Nephila clavata* and *Nephila pilipes*, spiders belonging to the genus *Menosira*, such as *Menosira ornata*, spiders belonging to the genus *Dyschiriognatha*, such as *Dyschiriognatha tenera*, spiders belonging to the genus *Latrodectus,* such as *Latrodectus mactans, Latrodectus hasseltii, Latrodectus geometricus,* and *Latrodectus tredecimguttatus*, and spiders belonging to the genus *Euprosthenops*. Examples of the spider silk proteins include dragline silk proteins such as MaSps (MaSp1 and MaSp2) and ADFs (ADF3 and ADF4), and MiSps (MiSp1 and MiSp2).

More specific examples of the fibroin produced by spiders include fibroin-3 (adf-3) [derived from Araneus diadematus] (GenBank Accession Number AAC47010 (amino acid sequence), U47855 (base sequence)), fibroin-4 (adf-4) [derived from Araneus diadematus] (GenBank Accession Number AAC47011 (amino acid sequence), U47856 (base sequence)), dragline silk protein spidroin 1 [derived from Nephila clavipes] (GenBank Accession Number AAC04504 (amino acid sequence), U37520 (base sequence)), major angu11ate spidroin 1 [derived from Latrodectus hesperus] (GenBank Accession Number ABR68856 (amino acid sequence), EF595246 (base sequence)), dragline silk protein spidroin 2 [derived from Nephila clavata] (GenBank Accession Number AAL32472 (amino acid sequence), AF441245 (base sequence)), major anpullate spidroin 1 [derived from Euprosthenops australis] (GenBank Accession Number CAJ00428 (amino acid sequence), AJ973155 (base sequence)) and major ampullate spidroin 2 [Euprosthenops australis] (GenBank Accession Number CAM32249.1 (amino acid sequence), AM490169 (base sequence)), minor ampullate silk protein 1 [Nephila clavipes] (GenBank Accession Number AAC14589.1 (amino acid sequence), minor ampullate silk protein 2 [Nephila clavipes] (GenBank Accession Number AAC14591.1 (amino acid sequence)), and minor ampullate spidroin-like protein [Nephilengys cruentata] (GenBank Accession Number ABR37278.1 (amino acid sequence)).

A more specific example of the naturally derived fibroin includes fibroin having sequence information registered in NCBI GenBank. Among sequence information registered in NCBI GenBank, for example, the sequence information of the fibroin is checked by extracting sequences with a keyword of spidroin, ampullate, fibroin, "silk and polypeptide", or "silk and protein" in DEFINITION from sequences containing INV as DIVISION, by extracting sequences having a specific product character string from CDS, and by extracting sequences having a character string specific to TISSUE TYPE from SOURCE.

The "artificial fibroin" in the present specification means artificially produced fibroin (artificial fibroin). The artificial fibroin may be fibroin having an amino acid sequence different from an amino acid sequence of naturally derived fibroin or may be fibroin having an amino acid sequence identical to an amino acid sequence of naturally derived fibroin.

The artificial fibroin may be a fibrous protein having a structure similar to that of naturally derived fibroin, or may be fibroin having a sequence similar to a repeating sequence of naturally derived fibroin. The "sequence similar to the repeating sequence of fibroin" may actually be a sequence included in naturally derived fibroin, or may be a sequence similar thereto.

The "artificial fibroin" may be fibroin whose amino acid sequence has been modified based on naturally derived fibroin (for example, fibroin whose amino acid sequence has been modified by altering a cloned gene sequence of naturally derived fibroin) or fibroin obtained by artificially designing an amino acid sequence independently of naturally derived fibroin (for example, fibroin having a desired amino acid sequence by chemically synthesizing a nucleic acid encoding the designed amino acid sequence), as long as it has the amino acid sequence specified in the present disclosure. Note that a modified amino acid sequence of artificial fibroin is also included in artificial fibroin when the amino acid sequence thereof is different from the amino acid sequence of naturally derived fibroin. Examples of the artificial fibroin include artificial silk fibroin (fibroin obtained by modifying an amino acid sequence of a silk protein produced by silkworms), and artificial spider silk fibroin (fibroin obtained by modifying an amino acid sequence of a spider silk protein produced by spiders). In a case where the synthetic polymer according to the present disclosure is used as a molding material, artificial fibroin preferably may include artificial spider silk fibroin, and more preferably may be formed of artificial spider silk fibroin, because spider silk fibroin is relatively easily fibrillated and has a high fiber forming ability.

Specific examples of the artificial fibroin according to the present embodiment can include artificial fibroin derived from a major dragline silk protein produced in a major ampullate gland of a spider (first artificial fibroin), artificial fibroin having a domain sequence in which the content of glycine residues is reduced (second artificial fibroin), artificial fibroin having a domain sequence in which the content of an $(A)_n$ motif is reduced (third artificial fibroin), artificial fibroin in which the content of glycine residues and the content of an $(A)_n$ motif are reduced (fourth artificial fibroin), artificial fibroin having a domain sequence including a region locally having a high hydropathy index (fifth artificial fibroin), and artificial fibroin having a domain sequence in which the content of glutamine residues is reduced (sixth artificial fibroin).

An example of the first artificial fibroin can include a protein having a domain sequence represented by Formula 1: $[(A)_n \text{ motif-REP}]_m$. In the first artificial fibroin, the number of amino acid residues in the $(A)_n$ motif is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16,

and most preferably an integer of 10 to 16. The number of amino acid residues constituting REP in Formula 1 in the first artificial fibroin is preferably 10 to 200 residues, more preferably 10 to 150 residues, even more preferably 20 to 100 residues, and still more preferably 20 to 75 residues. In the first artificial fibroin, the total number of glycine residues, serine residues, and alanine residues included in the amino acid sequence represented by Formula 1: $[(A)_n$ motif-REP$]_m$ is preferably 40% or more, more preferably 60% or more, and still more preferably 70% or more, relative to the total number of amino acid residues.

The first artificial fibroin may be a polypeptide having an amino acid sequence unit represented by Formula 1: $[(A)_n$ motif-REP$]_m$, and including a C-terminal sequence which is an amino acid sequence set forth in any one of SEQ ID NOS: 1 to 3 or a C-terminal sequence which is an amino acid sequence having 90% or more homology with the amino acid sequence set forth in any one of SEQ ID NOS: 1 to 3.

The amino acid sequence set forth in SEQ ID NO: 1 is identical to an amino acid sequence consisting of 50 amino acid residues at the C-terminus of the amino acid sequence of ADF3 (GI: 1263287, NCBI), the amino acid sequence set forth in SEQ ID NO: 2 is identical to an amino acid sequence obtained by removing 20 amino acid residues from the C-terminus of the amino acid sequence set forth in SEQ ID NO: 1, and the amino acid sequence set forth in SEQ ID NO: 3 is identical to an amino acid sequence obtained by removing 29 residues from the C-terminus of the amino acid sequence set forth in SEQ ID NO: 1.

More specific examples of the first artificial fibroin can include artificial fibroin having (1-i) an amino acid sequence set forth in SEQ ID NO: 4 (recombinant spider silk protein ADF3KaiLargeNRSH1) and (1-ii) an amino acid sequence having a sequence identity of 90% or higher with the amino acid sequence set forth in SEQ ID NO: 4. The sequence identity is preferably 95% or more.

The amino acid sequence of SEQ ID NO: 4 is obtained by the following mutation. That is, in the amino acid sequence of ADF3 having the N-terminus to which an amino acid sequence including a start codon, a His10 tag, and a human rhinovirus 3C protease (HRV3C protease) recognition site (SEQ ID NO: 5) are added, the first to thirteenth repetitive regions are roughly doubled and the translation ends at the 1154th amino acid residue. The C-terminal amino acid sequence of the amino acid sequence of SEQ ID NO: 4 is identical to the amino acid sequence of SEQ ID NO: 3.

The artificial fibroin of (1-i) may consist of the amino acid sequence set forth in SEQ ID NO: 4.

The domain sequence of the second artificial fibroin has an amino acid sequence in which the content of glycine residues is reduced compared to the naturally derived fibroin. The second artificial fibroin can be defined as fibroin having an amino acid sequence corresponding to an amino acid sequence in which at least one or a plurality of glycine residues in REP are substituted by other amino acid residues, compared to the naturally derived fibroin.

The domain sequence of the second artificial fibroin may have an amino acid sequence corresponding to an amino acid sequence in which one glycine residue in at least one or a plurality of motif sequences is substituted by another amino acid residue, compared to the naturally derived fibroin, the motif sequence being at least one motif sequence selected from GGX and GPGXX (where G represents a glycine residue, P represents a proline residue, and X represents an amino acid residue other than glycine) in REP.

In the second artificial fibroin, a proportion of the motif sequence in which a glycine residue is substituted by another amino acid residue described above to the total motif sequences may be 10% or higher.

The second artificial fibroin has a domain sequence represented by Formula 1: $[(A)_n$ motif-REP$]_m$, and may have an amino acid sequence in which z/w is 30% or more, 40% or more, 50% or more, or 50.9% or more in a case where the total number of amino acid residues in the amino acid sequence consisting of XGX (where X represents an amino acid residue other than glycine) included in all REPs in a sequence excluding the sequence from the $(A)_n$ motif located at the most C-terminal side to the C-terminus of the domain sequence from the domain sequence is defined as z, and the total number of amino acid residues in the sequence excluding the sequence from the $(A)_n$ motif located at the most C-terminal side to the C-terminus of the domain sequence from the domain sequence is defined as w. The number of alanine residues with respect to the total number of amino acid residues in the $(A)_n$ motif is 83% or more, preferably 86% or more, more preferably 90% or more, still more preferably 95% or more, and even still more preferably 100% (meaning that the $(A)_n$ motif consists of only alanine residues).

It is preferable that a content proportion of the amino acid sequence consisting of XGX in the second artificial fibroin is increased by substituting one glycine residue in the GGX motif with another amino acid residue. In the second artificial fibroin, a content proportion of amino acid sequences consisting of GGX in the domain sequence is preferably 30% or less, more preferably 20% or less, still more preferably 10% or less, still more preferably 6% or less, still more preferably 4% or less, and particularly preferably 2% or less. A content proportion of amino acid sequences consisting of GGX in the domain sequence is calculated by a method similar to the following method for calculating a content proportion of amino acid sequences consisting of XGX (z/w).

The method for calculating z/w will be described in more detail. First, the amino acid sequence consisting of XGX is extracted from all REPs included in a sequence excluding the sequence from the $(A)_n$ motif located at the most C-terminal side to the C-terminus of the domain sequence from the domain sequence in the fibroin having a domain sequence represented by Formula 1: $[(A)_n$ motif-REP$]_m$ (artificial fibroin or naturally derived fibroin). The total number

of amino acid residues included in XGX is represented by "z". For example, when 50 amino acid sequences consisting of XGX are extracted (no overlap), "z" is 50 × 3 = 150. In addition, for example, in an amino acid sequence consisting of XGXGX, one X (X in the center) is included in two XGXs, and the overlapping portion is subtracted to calculate "z" (that is, there are 5 amino acid residues in XGXGX). w is a total number of amino acid residues contained in a sequence excluding the sequence from the $(A)_n$ motif located at the most C-terminal side to the C-terminus of the domain sequence from the domain sequence. For example, in a case of the domain sequence shown in Fig. 1, w is 4 + 50 + 4 + 100 + 4 + 10 + 4 + 20 + 4 + 30 = 230 (excluding the $(A)_n$ motif located at the most C-terminal side). Next, "z" is divided by "w" to calculate z/w (%).

Here, z/w in the naturally derived fibroin will be described. First, fibroin of which the amino acid sequence information is registered in NCBI GenBank was verified using the method exemplified above, and as a result, 663 types of fibroin (among these, 415 types were fibroin derived from spiders) were extracted. Among all extracted fibroin, values of z/w were calculated, using the calculation method described above, from amino acid sequences of naturally derived fibroin had domain sequences represented by Formula 1: $[(A)_n$ motif-REP$]_m$ and in which the content proportions of the amino acid sequences consisting of GGX in the fibroins were 6% or lower. The results are shown in Fig. 2. In Fig. 2, the horizontal axis represents z/w (%), and the vertical axis represents a frequency. As is clear from Fig. 2, the values of z/w in naturally derived fibroins are all smaller than 50.9% (the largest value is 50.86%).

z/w in the second artificial fibroin is preferably 50.9% or higher, more preferably 56.1% or higher, even more preferably 58.7% or higher, still more preferably 70% or higher, and still even more preferably 80% or higher. The upper limit of z/w is not particularly limited but may be, for example, 95% or less.

The second artificial fibroin can be obtained by, for example, performing modification so that at least a part of base sequences encoding glycine residues in a cloned gene sequence for the naturally derived fibroin are substituted so as to encode another amino acid residue. In this case, one glycine residue in a GGX motif and GPGXX motif may be selected as a glycine residue to be modified or may be substituted so that z/w becomes 50.9% or more. It is also possible to obtain the second artificial fibroin by, for example, designing an amino acid sequence satisfying the above aspect from the amino acid sequence of the naturally derived fibroin and chemically synthesizing a nucleic acid encoding the designed amino acid sequence. In any case, in addition to the modification corresponding to a substitution of a glycine residue in REP in the amino acid sequence of the naturally derived fibroin with another amino acid residue, further amino acid sequence modification may be performed, which corresponds to a substitution, a deletion, an insertion, and/or an addition of one or a plurality of amino acid residues.

Another amino acid residue above is not particularly limited as long as it is an amino acid residue other than a glycine residue, and the amino acid residue is preferably a hydrophobic amino acid residue such as a valine (V) residue, a leucine (L) residue, an isoleucine (I) residue, a methionine (M) residue, a proline (P) residue, a phenylalanine (F) residue, and a tryptophan (W) residue, and a hydrophilic amino acid residue such as a glutamine (Q) residue, an asparagine (N) residue, a serine (S) residue, a lysine (K) residue, and a glutamic acid (E) residue, more preferably a valine (V) residue, a leucine (L) residue, an isoleucine (I) residue, a phenylalanine (F) residue, and a glutamine (Q) residue, and even more preferably a glutamine (Q) residue.

More specific examples of the second artificial fibroin can include artificial fibroin having (2-i) an amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799) and (2-ii) an amino acid sequence having a sequence identity of 90% or higher with the amino acid sequence set forth in SEQ ID NO: 6, SEQ ID NO: 7, SEQ ID NO: 8, or SEQ ID NO: 9.

The artificial fibroin of (2-i) will be described. The amino acid sequence set forth in SEQ ID NO: 6 is obtained by substituting all GGX's in REP in an amino acid sequence set forth in SEQ ID NO: 10 (Met-PRT313), which corresponds to the naturally derived fibroin, with GQX's. The amino acid sequence set forth in SEQ ID NO: 7 is obtained by deleting every other two $(A)_n$ motifs from the N-terminal side to the C-terminal side from the amino acid sequence set forth in SEQ ID NO: 6 and further inserting one $[(A)_n$ motif-REP$]$ before the C-terminal sequence. The amino acid sequence set forth in SEQ ID NO: 8 is obtained by inserting two alanine residues at the C-terminal side of each $(A)_n$ motif of the amino acid sequence set forth in SEQ ID NO: 7 and further substituting a part of glutamine (Q) residues with a serine (S) residue to delete a part of amino acids at the C-terminal side so as to be almost the same as a molecular weight of SEQ ID NO: 7. The amino acid sequence set forth in SEQ ID NO: 9 is obtained by adding a predetermined hinge sequence and His-tag sequence to the C-terminus of a sequence in which a region of 20 domain sequences (here, several amino acid residues on the C-terminal side of the region are substituted) existing in the amino acid sequence set forth in SEQ ID NO: 7 is repeated 4 times.

A value of z/w in the amino acid sequence set forth in SEQ ID NO: 10 (corresponding to naturally derived fibroin) is 46.8%. Values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, values of x/y in the amino acid sequences set forth in SEQ ID NO: 10, SEQ ID NO: 6, SEQ ID NO: 7, SEQ ID NO: 8, and SEQ ID NO: 9 at a Giza ratio (to be described later) of 1:1.8 to 11.3 are 15.0%, 15.0%, 93.4%, 92.7%, and 89.8%, respectively.

The artificial fibroin of (2-i) may consist of the amino acid sequence set forth in SEQ ID NO: 6, SEQ ID NO: 7, SEQ ID NO: 8, or SEQ ID NO: 9.

The artificial fibroin of (2-ii) has an amino acid sequence having a sequence identity of 90% or higher with the amino acid sequence set forth in SEQ ID NO: 6, SEQ ID NO: 7, SEQ ID NO: 8, or SEQ ID NO: 9. The artificial fibroin of (2-ii) is also a protein having a domain sequence represented by Formula 1: $[(A)_n$ motif-REP$]_m$. The sequence identity is preferably 95% or more.

The artificial fibroin of (2-ii) has a sequence identity of 90% or higher with the amino acid sequence set forth in SEQ ID NO: 6, SEQ ID NO: 7, SEQ ID NO: 8, or SEQ ID NO: 9, and in a case where a total number of amino acid residues in amino acid sequences consisting of XGX (where X represents an amino acid residue other than glycine) which are contained in REP is denoted by z, and a total number of amino acid residues in REP's in the domain sequence is denoted by w, z/w is preferably 50.9% or higher.

The second artificial fibroin may contain a tag sequence at one or both of the N-terminus and the C-terminus thereof. By containing a tag sequence, isolation, immobilization, detection, visualization, and the like of the artificial fibroin become possible.

Examples of the tag sequence can include an affinity tag using specific affinity (binding properties or affinity) to another molecule. A specific example of the affinity tag can include a histidine tag (His tag). His tags are short peptides having about 4 to 10 histidine residues and specifically binding to metal ions such as nickel. Accordingly, it is possible to use a His tag to isolate an artificial fibroin by chelating metal chromatography. Specific examples of the tag sequence include an amino acid sequence set forth in SEQ ID NO: 11 (an amino acid sequence having a His-tag sequence and a hinge sequence).

In addition, it is possible to employ a tag sequence such as glutathione-S-transferase (GST) that specifically binds to glutathione or a maltose binding protein (MBP) that specifically binds to maltose.

An "epitope tag" utilizing an antigen-antibody reaction is employable. Adding a peptide (epitope) exhibiting antigenicity as a tag sequence allows binding of an antibody to the epitope. Examples of the epitope tag can include HA tag (peptide sequence of influenza hemagglutinin), myc tag, and FLAG tag. The use of the epitope tag allows purification of the artificial fibroin to be easily performed with high specificity.

Further, it is possible to use a tag sequence which is cleaved with a specific protease. By treating a protein adsorbed via the tag sequence with a protease, the artificial fibroin from which the tag sequence is cleaved can be recovered.

More specific examples of the artificial fibroin having a tag sequence can include artificial fibroin having (2-iii) an amino acid sequence set forth in SEQ ID NO: 12 (PRT380), SEQ ID NO: 13 (PRT410), SEQ ID NO: 14 (PRT525), or SEQ ID NO: 15 (PRT799) and (2-iv) an amino acid sequence having a sequence identity of 90% or higher with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-termini of the amino acid sequences set forth in SEQ ID NO: 10, SEQ ID NO: 6, SEQ ID NO: 7, SEQ ID NO: 8, and SEQ ID NO: 9, respectively.

The artificial fibroin of (2-iii) may consist of the amino acid sequence set forth in SEQ ID NO: 12, SEQ ID NO: 13, SEQ ID NO: 14, or SEQ ID NO: 15.

The artificial fibroin of (2-iv) has an amino acid sequence having a sequence identity of 90% or higher with the amino acid sequence set forth in SEQ ID NO: 12, SEQ ID NO: 13, SEQ ID NO: 14, or SEQ ID NO: 15. The artificial fibroin of (2-iv) is also a protein having a domain sequence represented by Formula 1: $[(A)_n$ motif-REP$]_m$. The sequence identity is preferably 95% or more.

The artificial fibroin of (2-iv) has a sequence identity of 90% or higher with the amino acid sequence set forth in SEQ ID NO: 12, SEQ ID NO: 13, SEQ ID NO: 14, or SEQ ID NO: 15, and in a case where a total number of amino acid residues in amino acid sequences consisting of XGX (where X represents an amino acid residue other than glycine) which are contained in REP is denoted by z, and a total number of amino acid residues in REP's in the domain sequence is denoted by w, z/w is preferably 50.9% or higher.

The second artificial fibroin may contain a secretory signal for releasing a protein produced in an artificial protein production system to the outside of a host. A sequence of the secretory signal is designed appropriately depending on the type of the host.

The domain sequence of the third artificial fibroin has an amino acid sequence in which the content of the $(A)_n$ motif is reduced, as compared with naturally derived fibroin. It can be said that the domain sequence of the third artificial fibroin has an amino acid sequence corresponding to an amino acid sequence in which at least one or a plurality of $(A)_n$ motifs are deleted, as compared with naturally derived fibroin.

The third artificial fibroin may have an amino acid sequence corresponding to an amino acid sequence in which 10 to 40% of the $(A)_n$ motifs are deleted from naturally derived fibroin.

The domain sequence of the third artificial fibroin may have an amino acid sequence corresponding to an amino

acid sequence in which at least one $(A)_n$ motif of every one to three $(A)_n$ motifs is deleted from the N-terminal side to the C-terminal side, as compared with naturally derived fibroin.

The third artificial fibroin may have a domain sequence having an amino acid sequence corresponding to an amino acid sequence in which deletion of at least two consecutive $(A)_n$ motifs and deletion of one $(A)_n$ motif are repeated in this order from the N-terminal side to the C-terminal side, as compared with naturally derived fibroin.

The third artificial fibroin may have a domain sequence having an amino acid sequence corresponding to an amino acid sequence in which at least $(A)_n$ motif every other two positions is deleted from the N-terminal side to the C-terminal side.

The third artificial fibroin has a domain sequence represented by Formula 1: $[(A)_n$ motif-REP$]_m$, and may have an amino acid sequence in which x/y is 20% or more, 30% or more, 40% or more, or 50% or more in a case where the numbers of amino acid residues in REPs of two adjacent $[(A)_n$ motif-REP] units are sequentially compared from the N-terminal side to the C-terminal side, and the number of amino acid residues in one REP having a smaller number of amino acid residues is defined as 1, the maximum value of the total value of the number of amino acid residues in the two adjacent $[(A)_n$ motif-REP] units where the ratio of the number of amino acid residues in the other REP is 1.8 to 11.3 is defined as x, and the total number of amino acid residues in the domain sequence is defined as y. The number of alanine residues with respect to the total number of amino acid residues in the $(A)_n$ motif is 83% or more, preferably 86% or more, more preferably 90% or more, still more preferably 95% or more, and even still more preferably 100% (meaning that the $(A)_n$ motif consists of only alanine residues).

A method for calculating x/y will be described in more detail with reference to Fig. 1. Fig. 1 shows a domain sequence excluding the N-terminal sequence and the C-terminal sequence from the artificial fibroin. The domain sequence has a sequence of $(A)_n$ motif-first REP (50 amino acid residues)-$(A)_n$ motif-second REP (100 amino acid residues)-$(A)_n$ motif-third REP (10 amino acid residues)-$(A)_n$ motif-fourth REP (20 amino acid residues)-$(A)_n$ motif-fifth REP (30 amino acid residues)-$(A)_n$ motif from the N-terminal side (left side).

The two adjacent $[(A)_n$ motif-REP] units are sequentially selected from the N-terminal side to the C-terminal side so as not to overlap. At this time, an unselected $[(A)_n$ motif-REP] unit may exist. Fig. 1 shows Pattern 1 (a comparison between the first REP and the second REP, and a comparison between the third REP and the fourth REP), Pattern 2 (a comparison between the first REP and the second REP, and a comparison between the fourth REP and the fifth REP), Pattern 3 (a comparison between the second REP and the third REP, and a comparison between the fourth REP and the fifth REP), and Pattern 4 (a comparison between the first REP and the second REP). Note that $[(A)_n$ motif-REP] units may be selected in other ways.

Subsequently, the number of amino acid residues of each REP in the selected two adjacent $[(A)_n$ motif-REP] units is compared for each pattern. The comparison is performed by setting the smaller number of amino acid residues as 1 and determining the ratio of the number of amino acid residues in the other REP. For example, comparing the first REP (50 amino acid residues) and the second REP (100 amino acid residues), a ratio of amino acid residues in the second REP is 100/50 = 2, defining the first REP having fewer amino acid residues as 1. Similarly, comparing the fourth REP (20 amino acid residues) and the fifth REP (30 amino acid residues), a ratio of amino acid residues in the fifth REP is 30/20 = 1.5, defining the fourth REP having fewer amino acid residues as 1.

In Fig. 1, a set of $[(A)_n$ motif-REP] units in which the ratio of the number of amino acid residues of the other REP is 1.8 to 11.3 in a case where one REP having a smaller number of amino acid residues is defined as 1 is indicated by a solid line. In the present specification, this ratio will be referred to as a Giza ratio. A set of $[(A)_n$ motif-REP] units in which the ratio of the number of amino acid residues of the other REP is less than 1.8 or more than 11.3 in a case where one REP having a smaller number of amino acid residues is defined as 1 is indicated by a broken line.

In each pattern, the number of all amino acid residues in two adjacent $[(A)_n$ motif-REP] units indicated by solid lines (including not only the number of amino acid residues in REP but also the number of amino acid residues in $(A)_n$ motif) are summed up. The values of the sums are compared with each other, and the value of the sum of a pattern with the largest sum (maximum value of the sum) is denoted by x. In the example shown in Fig. 1, the total value of Pattern 1 is the maximum.

Then, "x" is divided by the total number of amino acid residues "y" of the domain sequence to calculate x/y (%).

x/y in the third artificial fibroin is preferably 50% or higher, more preferably 60% or higher, even more preferably 65% or higher, still more preferably 70% or higher, still even more preferably 75% or higher, and particularly preferably 80% or higher. The upper limit of x/y is not particularly limited but may be, for example, 100% or less. With a GISA ratio of 1:1.9 to 11.3, x/y is preferably 89.6% or more. With a GISA ratio of 1:1.8 to 3.4, x/y is preferably 77.1% or more. With a GISA ratio of 1:1.9 to 8.4, x/y is preferably 75.9% or more. With a GISA ratio of 1:1.9 to 4.1, x/y is preferably 64.2% or more.

In a case where the third artificial fibroin is artificial fibroin in which at least seven of a plurality of $(A)_n$ motifs in the domain sequence consist of only alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as the value is 100% or less.

Here, x/y in the naturally derived fibroin will be described. First, fibroin of which the amino acid sequence

information is registered in NCBI GenBank was verified using the method exemplified above, and as a result, 663 types of fibroin (among these, 415 types were fibroin derived from spiders) were extracted. The values of x/y were calculated by the calculation method described above, from amino acid sequences of naturally derived fibroins consisting of a domain sequence represented by Formula 1: $[(A)_n \text{ motif-REP}]_m$, among all the extracted fibroins. The results in a case where the Giza ratio is 1:1.9 to 4.1 are shown in Fig. 3.

The horizontal axis in Fig. 3 represents x/y (%), and the vertical axis represents a frequency. As is clear from Fig. 3, the values of x/y in naturally derived fibroins are all smaller than 64.2% (the largest value is 64.14%) .

The third artificial fibroin can be obtained from, for example, a cloned gene sequence of naturally derived fibroin, by deleting one or a plurality of sequences encoding an $(A)_n$ motif so that x/y is 64.2% or more. In addition, for example, the third artificial fibroin can also be obtained, from the amino acid sequence of naturally derived fibroin, by designing an amino acid sequence corresponding to an amino acid sequence in which one or a plurality of $(A)_n$ motifs are deleted so that x/y is 64.2% or more, and chemically synthesizing a nucleic acid encoding the designed amino acid sequence. In any case, in addition to the modification corresponding to deletion of the $(A)_n$ motif from the amino acid sequence of naturally derived fibroin, modification of the amino acid sequence corresponding to substitution, deletion, insertion, and/or addition of one or a plurality of amino acid residues may be performed.

More specific examples of the third artificial fibroin can include artificial fibroin having (3-i) an amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799) and (3-ii) an amino acid sequence having a sequence identity of 90% or higher with the amino acid sequence set forth in SEQ ID NO: 17, SEQ ID NO: 7, SEQ ID NO: 8, or SEQ ID NO: 9.

The artificial fibroin of (3-i) will be described. The amino acid sequence set forth in SEQ ID NO: 17 is obtained by deleting every other two $(A)_n$ motifs from the N-terminal side to the C-terminal side from the amino acid sequence set forth in SEQ ID NO: 10 (Met-PRT313) corresponding to naturally derived fibroin and further inserting one $[(A)_n$ motif-REP] before the C-terminal sequence. The amino acid sequence set forth in SEQ ID NO: 7, SEQ ID NO: 8, or SEQ ID NO: 9 is as described in the second artificial fibroin.

A value of x/y in the amino acid sequence set forth in SEQ ID NO: 10 (corresponding to the naturally derived fibroin) at a Giza ratio of 1:1.8 to 11.3 is 15.0%. Values of x/y in the amino acid sequence set forth in SEQ ID NO: 17 and the amino acid sequence set forth in SEQ ID NO: 7 are both 93.4%. A value of x/y in the amino acid sequence set forth in SEQ ID NO: 8 is 92.7%. A value of x/y in the amino acid sequence set forth in SEQ ID NO: 9 is 89.8%. Values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The artificial fibroin of (3-i) may consist of the amino acid sequence set forth in SEQ ID NO: 17, SEQ ID NO: 7, SEQ ID NO: 8, or SEQ ID NO: 9.

The artificial fibroin of (3-ii) has an amino acid sequence having a sequence identity of 90% or higher with the amino acid sequence set forth in SEQ ID NO: 17, SEQ ID NO: 7, SEQ ID NO: 8, or SEQ ID NO: 9. The artificial fibroin of (3-ii) is also a protein having a domain sequence represented by Formula 1: $[(A)_n \text{ motif-REP}]_m$. The sequence identity is preferably 95% or more.

It is preferable that the artificial fibroin of (3-ii) has 90% or more sequence identity with the amino acid sequence set forth in SEQ ID NO: 17, SEQ ID NO: 7, SEQ ID NO: 8, or SEQ ID NO: 9, and x/y is 64.2% or more in a case where the numbers of amino acid residues in REPs of two adjacent $[(A)_n$ motif-REP] units are sequentially compared from the N-terminal side to the C-terminal side, and the number of amino acid residues in one REP having a small number of amino acid residues is defined as 1, the maximum value of the total value of the number of amino acid residues in the two adjacent $[(A)_n$ motif-REP] units where the ratio of the number of amino acid residues in the other REP is 1.8 to 11.3 (the Giza ratio is 1:1.8 to 11.3) is defined as x, and the total number of amino acid residues in the domain sequence is defined as y.

The third artificial fibroin may have the tag sequence described above at one or both of the N-terminus and the C-terminus thereof.

More specific examples of the artificial fibroin having a tag sequence can include artificial fibroin having (3-iii) an amino acid sequence set forth in SEQ ID NO: 18 (PRT399), SEQ ID NO: 13 (PRT410), SEQ ID NO: 14 (PRT525), or SEQ ID NO: 15 (PRT799) and (3-iv) an amino acid sequence having a sequence identity of 90% or higher with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-termini of the amino acid sequences set forth in SEQ ID NO: 17, SEQ ID NO: 7, SEQ ID NO: 8, and SEQ ID NO: 9, respectively.

The artificial fibroin of (3-iii) may consist of the amino acid sequence set forth in SEQ ID NO: 18, SEQ ID NO: 13, SEQ ID NO: 14, or SEQ ID NO: 15.

The artificial fibroin of (3-iv) has an amino acid sequence having a sequence identity of 90% or higher with the amino acid sequence set forth in SEQ ID NO: 18, SEQ ID NO: 13, SEQ ID NO: 14, or SEQ ID NO: 15. The artificial

fibroin of (3-iv) is also a protein having a domain sequence represented by Formula 1: $[(A)_n \text{ motif-REP}]_m$. The sequence identity is preferably 95% or more.

It is preferable that the artificial fibroin of (3-iv) has 90% or more sequence identity with the amino acid sequence set forth in SEQ ID NO: 18, SEQ ID NO: 13, SEQ ID NO: 14, or SEQ ID NO: 15, and x/y is 64.2% or more in a case where the number of amino acid residues in REPs in two adjacent $[(A)_n \text{ motif-REP}]$ units are sequentially compared from the N-terminal side to the C-terminal side, and the number of amino acid residues in one REP having a small number of amino acid residues is defined as 1, the maximum value of the total value of the number of amino acid residues in the two adjacent $[(A)_n \text{ motif-REP}]$ units where the ratio of the number of amino acid residues in the other REP is 1.8 to 11.3 is defined as x, and the total number of amino acid residues in the domain sequence is defined as y.

The third artificial fibroin may contain a secretory signal for releasing a protein produced in an artificial protein production system to the outside of a host. A sequence of the secretory signal is designed appropriately depending on the type of the host.

The domain sequence of the fourth artificial fibroin has an amino acid sequence in which the content of an $(A)_n$ motif and the content of glycine residues are reduced, as compared with naturally derived fibroin. It can be said that the domain sequence of the fourth artificial fibroin has an amino acid sequence corresponding to an amino acid sequence in which at least one or a plurality of $(A)_n$ motifs are deleted and at least one or a plurality of glycine residues in REP are substituted with another amino acid residue, as compared with naturally derived fibroin. That is, the fourth artificial fibroin is artificial fibroin having characteristics of both the second artificial fibroin and the third artificial fibroin described above. Specific aspects thereof and the like are as in the descriptions for the second artificial fibroin and the third artificial fibroin.

More specific examples of the fourth artificial fibroin can include artificial fibroin having (4-i) an amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799) and (4-ii) an amino acid sequence having a sequence identity of 90% or higher with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the artificial fibroin having the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth artificial fibroin may have an amino acid sequence having a region in which a hydropathy index is locally high, which corresponds to an amino acid sequence in which one or a plurality of amino acid residues in REP are substituted by amino acid residues having a high hydropathy index, and/or an amino acid sequence in which one or a plurality of amino acid residues having a high hydropathy index are inserted into REP, compared to the naturally derived fibroin.

The region locally having a high hydropathy index preferably includes two to four consecutive amino acid residues.

The amino acid residue having a high hydropathy index is more preferably an amino acid residue selected from isoleucine (I), valine (V), leucine (L), phenylalanine (F), cysteine (C), methionine (M), and alanine (A).

In addition to the modification corresponding to a substitution of one or a plurality of amino acid residues in REP with amino acid residues having a high hydropathy index and/or an insertion of one or a plurality of amino acid residues having a high hydropathy index into REP, compared to the naturally derived fibroin, further amino acid sequence modification may be performed on the fifth artificial fibroin, which corresponds to a substitution, a deletion, an insertion, and/or an addition of one or a plurality of amino acid residues, compared to the naturally derived fibroin.

The fifth artificial fibroin can be obtained from, for example, a cloned gene sequence for the naturally derived fibroin by substituting one or a plurality of hydrophilic amino acid residues (for example, amino acid residues having a negative value of hydropathy index) in REP with hydrophobic amino acid residues (for example, amino acid residues having a positive value of hydropathy index) and/or by inserting one or a plurality of hydrophobic amino acid residues into REP. It is also possible to obtain the fifth artificial fibroin by, for example, designing an amino acid sequence corresponding to an amino acid sequence in which one or a plurality of hydrophilic amino acid residues in REP in the amino acid sequence of the naturally derived fibroin are substituted by hydrophobic amino acid residues and/or an amino acid sequence in which one or a plurality of hydrophobic amino acid residues are inserted into REP in the amino acid sequence of the naturally derived fibroin and chemically synthesizing a nucleic acid encoding the designed amino acid sequence. In any case, in addition to the modification corresponding to a substitution of one or a plurality of hydrophilic amino acid residues in REP in the amino acid sequence of the naturally derived fibroin with hydrophobic amino acid residues and/or an insertion of one or a plurality of hydrophobic amino acid residues into REP in the amino acid sequence of the naturally derived fibroin, further amino acid sequence modification may be performed, which corresponds to a substitution, a deletion, an insertion, and/or an addition of one or a plurality of amino acid residues.

The fifth artificial fibroin has a domain sequence represented by Formula 1: $[(A)_n \text{ motif-REP}]_m$, and may have an amino acid sequence in which p/q is 6.2% or more in a case where in all REPs included in a sequence excluding the sequence from the $(A)_n$ motif located at the most C-terminal side to the C-terminus of the domain sequence from the

domain sequence, the total number of amino acid residues included in a region where the average value of hydropathy indices of four consecutive amino acid residues is 2.6 or more is defined as p, and the total number of amino acid residues included in the sequence excluding the sequence from the $(A)_n$ motif located at the most C-terminal side to the C-terminus of the domain sequence from the domain sequence is defined as q.

As the hydropathy index of an amino acid residue, a known index (Hydropathy index: Kyte J, & Doolittle R (1982) "A simple method for displaying the hydropathic character of a protein", J. Mol. Biol., 157, pp. 105-132) is used. Specifically, a hydropathy index (hereinafter, also referred to as "HI") of each amino acid is indicated in the following Table 1.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. In the calculation, a sequence excluding the sequence from the $(A)_n$ motif located at the most C-terminal side to the C-terminus of the domain sequence from the domain sequence represented by Formula 1 $[(A)_n$ motif-REP$]_m$ (hereinafter also referred to as "sequence A") is used. First, in all REPs included in the sequence A, an average hydropathy index of four consecutive amino acid residues is calculated. The average value of hydropathy indices is calculated by dividing the sum of HI's of all amino acid residues included in the four consecutive amino acid residues by 4 (the number of amino acid residues). The average value of hydropathy indices is calculated for every four consecutive amino acid residues (each amino acid residue is used in the calculation of an average value one to four times). Next, regions in which the average value of hydropathy indices of four consecutive amino acid residues is 2.6 or higher are specified. Even when a certain amino acid residue corresponds to a plurality of "four consecutive amino acid residues having an average hydropathy index of 2.6 or more", the region is regarded as including one amino acid residue. The total number of amino acid residues included in the region is "p". The total number of amino acid residues included in the sequence A is "q".

For example, in a case where "four consecutive amino acid residues having an average hydropathy index of 2.6 or more" are extracted from 20 places (no overlap), there are 20 sets of four consecutive amino acid residues (no overlap) in a region where four consecutive amino acid residues have an average hydropathy index of 2.6 or more, and thus, "p" is $20 \times 4 = 80$. Further, in a case where, for example, only one amino acid residue overlaps within two sets of "four consecutive amino acid residues of which the average value of hydropathy indices is 2.6 or higher", the region in which the average value of hydropathy indices of four consecutive amino acid residues is 2.6 or higher includes seven amino acid residues ($p = 2 \times 4 - 1 = 7$. "-1" is a deduction of the overlapping amino acid residue). For example, in a case of the domain sequence shown in Fig. 4, seven sets of "four consecutive amino acid residues of which the average value of a hydropathy index is 2.6 or higher" are present without overlaps, and thus, p is $7 \times 4 = 28$. In addition, for example, in the case of the domain sequence shown in Fig. 4, q is 4 + 50 + 4 + 40 + 4 + 10 + 4 + 20 + 4 + 30 = 170 (the $(A)_n$ motif located at the end of the C-terminal side is excluded). Next, p/q (%) can be calculated by dividing "p" by "q". In the case of Fig. 4, p/q is 28/170 = 16.47%.

p/q in the fifth artificial fibroin is preferably 6.2% or higher, more preferably 7% or higher, even more preferably 10% or higher, still more preferably 20% or higher, and still even more preferably 30% or higher. The upper limit of p/q is not particularly limited but may be, for example, 45% or less.

The fifth artificial fibroin can be obtained by, for example, modifying a cloned amino acid sequence of the naturally derived fibroin into an amino acid sequence having a region in which a hydropathy index is locally high by substituting one or a plurality of hydrophilic amino acid residues (for example, amino acid residues having a negative

value of hydropathy index) in REP with hydrophobic amino acid residues (for example, amino acid residues having a positive value of hydropathy index) and/or by inserting one or a plurality of hydrophobic amino acid residues into REP, so that the condition of p/q is satisfied. It is also possible to obtain the fifth artificial fibroin by, for example, designing an amino acid sequence satisfying the condition of p/q from the amino acid sequence of the naturally derived fibroin and chemically synthesizing a nucleic acid encoding the designed amino acid sequence. In any case, in addition to the modification corresponding to a substitution of one or a plurality of amino acid residues in REP with amino acid residues having a high hydropathy index and/or an insertion of one or a plurality of amino acid residues having a high hydropathy index into REP, compared to the naturally derived fibroin, further modification may be performed, which corresponds to a substitution, a deletion, an insertion, and/or an addition of one or a plurality of amino acid residues.

The amino acid residue having a high hydropathy index is not particularly limited but is preferably isoleucine (I), valine (V), leucine (L), phenylalanine (F), cysteine (C), methionine (M), and alanine (A). Among these examples, valine (V), leucine (L), and isoleucine (I) are more preferable.

More specific examples of the fifth artificial fibroin can include artificial fibroin having (5-i) an amino acid sequence set forth in SEQ ID NO: 19 (Met-PRT720), SEQ ID NO: 20 (Met-PRT665), or SEQ ID NO: 21 (Met-PRT666) and (5-ii) an amino acid sequence having a sequence identity of 90% or higher with the amino acid sequence set forth in SEQ ID NO: 19, SEQ ID NO: 20, or SEQ ID NO: 21.

The artificial fibroin of (5-i) will be described. The amino acid sequence set forth in SEQ ID NO: 19 is obtained by inserting amino acid sequences consisting of three amino acid residues (VLI) at two sites for each REP in the amino acid sequence set forth in SEQ ID NO: 7 (Met-PRT410) excluding the terminal domain sequence on the C-terminal side, substituting a part of glutamine (Q) residues with serine (S) residues, and deleting a part of amino acids on the C-terminal side. The amino acid sequence set forth in SEQ ID NO: 20 is obtained by inserting an amino acid sequence consisting of three amino acid residues (VLI) at one site for each REP in the amino acid sequence set forth in SEQ ID NO: 8 (Met-PRT525). The amino acid sequence set forth in SEQ ID NO: 21 is obtained by inserting amino acid sequences consisting of three amino acid residues (VLI) at two sites for each REP in the amino acid sequence set forth in SEQ ID NO: 8.

The artificial fibroin of (5-i) may consist of the amino acid sequence set forth in SEQ ID NO: 19, SEQ ID NO: 20, or SEQ ID NO: 21.

The artificial fibroin of (5-ii) has an amino acid sequence having a sequence identity of 90% or higher with the amino acid sequence set forth in SEQ ID NO: 19, SEQ ID NO: 20, or SEQ ID NO: 21. The artificial fibroin of (5-ii) is also a protein having a domain sequence represented by Formula 1: $[(A)_n \text{ motif-REP}]_m$. The sequence identity is preferably 95% or more.

It is preferable that the artificial fibroin of (5-ii) has 90% or more sequence identity with the amino acid sequence set forth in SEQ ID NO: 19, SEQ ID NO: 20, or SEQ ID NO: 21, and p/q is 6.2% or more in a case where in all REPs included in a sequence excluding the sequence from the $(A)_n$ motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in the sequence excluding the sequence from the $(A)_n$ motif located at the most the C-terminal side to the C-terminus of the domain sequence from the domain sequence is defined as q.

The fifth artificial fibroin may contain a tag sequence at one or both of the N-terminus and the C-terminus thereof.

More specific examples of the artificial fibroin having a tag sequence can include artificial fibroin having (5-iii) an amino acid sequence set forth in SEQ ID NO: 22 (PRT720), SEQ ID NO: 23 (PRT665), or SEQ ID NO: 24 (PRT666) and (5-iv) an amino acid sequence having a sequence identity of 90% or higher with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are obtained by adding the amino acid sequence set forth in SEQ ID NO: 11 (which has a His-tag sequence and a hinge sequence) to the N-termini of the amino acid sequences set forth in SEQ ID NO: 19, SEQ ID NO: 20, and SEQ ID NO: 21, respectively.

The artificial fibroin of (5-iii) may consist of the amino acid sequence set forth in SEQ ID NO: 22, SEQ ID NO: 23, or SEQ ID NO: 24.

The artificial fibroin of (5-iv) has an amino acid sequence having a sequence identity of 90% or higher with the amino acid sequence set forth in SEQ ID NO: 22, SEQ ID NO: 23, or SEQ ID NO: 24. The artificial fibroin of (5-iv) is also a protein having a domain sequence represented by Formula 1: $[(A)_n \text{ motif-REP}]_m$. The sequence identity is preferably 95% or more.

It is preferable that the artificial fibroin of (5-iv) has 90% or more sequence identity with the amino acid sequence set forth in SEQ ID NO: 22, SEQ ID NO: 23, or SEQ ID NO: 24, and p/q is 6.2% or more in a case where in all REPs included in a sequence excluding the sequence from the $(A)_n$ motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in the sequence excluding the sequence from the $(A)_n$ motif located at the most C-terminal side to the C-terminus of the domain sequence from the domain sequence is defined as q.

The fifth artificial fibroin may contain a secretory signal for releasing a protein produced in an artificial protein production system to the outside of a host. A sequence of the secretory signal is designed appropriately depending on the type of the host.

The sixth artificial fibroin has an amino acid sequence in which a content of glutamine residues is reduced, compared to the naturally derived fibroin.

It is preferable that the sixth artificial fibroin contains at least one motif selected from a GGX motif and a GPGXX motif in the amino acid sequence of REP.

In a case where the sixth artificial fibroin contains the GPGXX motif in REP, a content rate of the GPGXX motifs is generally 1% or higher. The content rate of the GPGXX motifs may be 5% or higher and is preferably 10% or higher. The upper limit of the content rate of the GPGXX motifs is not particularly limited and may be 50% or less or 30% or less.

In the present specification, the "content rate of the GPGXX" is a value calculated by the following method.

The content rate of the GPGXX motifs in fibroin (artificial fibroin or naturally derived fibroin) having a domain sequence represented by Formula 1: $[(A)_n$ motif-REP$]_m$ or Formula 2: $[(A)_n$ motif-REP$]_m$-$(A)_n$ motif is calculated as s/t, in a case where the number obtained by tripling the total number of GPGXX motifs in regions of all REPs included in a sequence excluding the sequence from the $(A)_n$ motif located at the most C-terminal side to the C-terminus of the domain sequence from the domain sequence (that is, corresponding to the total number of G and P in the GPGXX motifs) is defined as s, and the total number of amino acid residues in all REPs excluding a sequence from the $(A)_n$ motif located at the most C-terminal side to the C-terminus of the domain sequence from the domain sequence and further excluding the $(A)_n$ motifs is defined as t.

In the calculation of the content rate of the GPGXX motifs, the "sequence excluding the sequence from the $(A)_n$ motif located at the most C-terminal side to the C-terminus of the domain sequence from the domain sequence" is used to exclude the effect occurring due to the fact that the "sequence from the $(A)_n$ motif located at the most C-terminal side to the C-terminus of the domain sequence" (a sequence corresponding to REP) may include a sequence having a low correlation with the sequence characteristic of fibroin, which influences the calculation result of the content rate of the GPGXX motifs in a case where m is small (that is, in a case where the domain sequence is short). In a case where the "GPGXX motif" is located at the C-terminus of an REP, even when "XX" is "AA", for example, it is regarded as the "GPGXX motif".

Fig. 1 is a schematic view illustrating a domain sequence of artificial fibroin. The calculation of the content rate of the GPGXX motifs will be specifically described with reference to Fig. 1. First, in the domain sequence of the artificial fibroin illustrated in Fig. 1 (which is the "$[(A)_n$ motif-REP$]_m$-$(A)_n$ motif" type), all REPs are included in the "sequence excluding the sequence from the $(A)_n$ motif located at the most C-terminal side to the C-terminus of the domain sequence from the domain sequence" (in Fig. 1, the sequence indicated as a "region A"), and therefore, the number of the GPGXX motifs for calculating s is 7, and s is $7 \times 3 = 21$. Similarly, since all REPs are included in the "sequence excluding the sequence from the $(A)_n$ motif located at the most C-terminal side to the C-terminus of the domain sequence from the domain sequence" (in Fig. 1, the sequence indicated as the "region A"), the total number t of the amino acid residues in all REPs when the $(A)_n$ motifs are further excluded from the sequence is 50 + 40 + 10 + 20 + 30 = 150. Next, "s" is divided by "t" to calculate s/t (%). In the artificial fibroin shown in Fig. 1, s/t is 21/150 = 14.0%.

The content rate of glutamine residues in the sixth artificial fibroin is preferably 9% or lower, more preferably 7% or lower, even more preferably 4% or lower, and particularly preferably 0%.

In the present specification, the "content of the glutamine residues" is a value calculated by the following method.

The content rate of the glutamine residues in fibroin (artificial fibroin or naturally derived fibroin) having a domain sequence represented by Formula 1: $[(A)_n$ motif-REP$]_m$ or Formula 2: $[(A)_n$ motif-REP$]_m$-$(A)_n$ motif is calculated as u/t, in a case where the total number of glutamine residues included in regions of all REPs included in a sequence excluding the sequence from the $(A)_n$ motif located at the most C-terminal side to the C-terminus of the domain sequence from the domain sequence (a sequence corresponding to the "region A" in Fig. 1) is defined as u, and the total number of amino acid residues in all REPs in the sequence excluding the sequence from the $(A)_n$ motif located at the most C-terminal side to the C-terminus of the domain sequence from the domain sequence and further excluding the $(A)_n$ motifs is defined as t. In the calculation of the content rate of the glutamine residues, the reason for targeting the "sequence excluding the sequence from the $(A)_n$ motif located at the most C-terminal side to the C-terminus of the domain sequence from the domain sequence" is the same as the reason described above.

The domain sequence of the sixth artificial fibroin may have an amino acid sequence corresponding to an amino acid sequence in which one or a plurality of glutamine residues in REP are deleted or substituted by other amino acid residues, compared to the naturally derived fibroin.

The "another amino acid residue" may be an amino acid residue other than the glutamine residue but is preferably an amino acid residue having a higher hydropathy index than that of the glutamine residue. The hydropathy index of each amino acid residue is shown in Table 1.

As shown in Table 1, an example of the amino acid residue having a higher hydropathy index than that of the glutamine residue can include one selected from isoleucine (I), valine (V), leucine (L), phenylalanine (F), cysteine (C),

methionine (M), alanine (A), glycine (G), threonine (T), serine (S), tryptophan (W), tyrosine (Y), proline (P), and histidine (H). Among these examples, the amino acid residue is more preferably one selected from isoleucine (I), valine (V), leucine (L), phenylalanine (F), cysteine (C), methionine (M), and alanine (A), and still more preferably one selected from isoleucine (I), valine (V), leucine (L), and phenylalanine (F).

A degree of hydrophobicity (hydropathy index) of REP of the sixth artificial fibroin may be, for example, -0.80 or more, -0.70, -0.60 or more, -0.50 or more, -0.40 or more, -0.30 or more, -0.20 or more, -0.10 or more, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. The upper limit of the degree of hydrophobicity of each REP is not particularly limited but may be 1.0 or less or 0.7 or less.

In the present specification, the "degree of hydrophobicity of each REP" is a value calculated by the following method.

The degree of hydrophobicity of REP in fibroin having a domain sequence represented by Formula 1: $[(A)_n$ motif-REP$]_m$ or Formula 2: $[(A)_n$ motif-REP$]_m$-$(A)_n$ motif (artificial fibroin or naturally derived fibroin) is calculated as v/t, in a case where the sum of hydropathy indices of amino acid residues in regions of all REPs included in a sequence excluding the sequence from the $(A)_n$ motif located at the most C-terminal side to the C-terminus of the domain sequence from the domain sequence (a sequence corresponding to the "region A" in Fig. 1) is defined as v, and the total number of amino acid residues in all REPs in the sequence excluding the sequence from the $(A)_n$ motif located at the most C-terminal side to the C-terminus of the domain sequence from the domain sequence and further excluding the $(A)_n$ motifs is defined as t. In the calculation of the degree of hydrophobicity of REP, the reason for targeting the "sequence excluding the sequence from the $(A)_n$ motif located at the most C-terminal side to the C-terminus of the domain sequence from the domain sequence" is the same as the reason described above.

In addition to the modification corresponding to a deletion of one or a plurality of glutamine residues in REP and/or a substitution of one or a plurality of glutamine residues in REP with other amino acid residues, compared to the naturally derived fibroin, further amino acid sequence modification may be performed on the domain sequence of the sixth artificial fibroin, which corresponds to a substitution, a deletion, an insertion, and/or an addition of one or a plurality of amino acid residues.

The sixth artificial fibroin can be obtained from, for example, a cloned gene sequence for the naturally derived fibroin by deleting one or a plurality of glutamine residues in REP and/or substituting one or a plurality of glutamine residues in REP with other amino acid residues. It is also possible to obtain the sixth artificial fibroin by, for example, designing an amino acid sequence corresponding to an amino acid sequence in which one or a plurality of glutamine residues in REP in the amino acid sequence of the naturally derived fibroin are deleted and/or one or a plurality of glutamine residues in REP in the amino acid sequence of the naturally derived fibroin are substituted by other amino acid residues and chemically synthesizing a nucleic acid encoding the designed amino acid sequence.

More specific examples of the sixth artificial fibroin can include artificial fibroin having (6-i) an amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028) and artificial fibroin having (6-ii) an amino acid sequence having a sequence identity of 90% or higher with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The artificial fibroin of (6-i) will be described. The amino acid sequence set forth in SEQ ID NO: 25 is obtained by substituting all QQ's in the amino acid sequence set forth in SEQ ID NO: 7 (Met-PRT410) with VL's. The amino acid sequence set forth in SEQ ID NO: 26 is obtained by substituting all QQ's in the amino acid sequence set forth in SEQ ID NO: 7 with TS's and substituting the remaining Q's with A's. The amino acid sequence set forth in SEQ ID NO: 27 is obtained by substituting all QQ's in the amino acid sequence set forth in SEQ ID NO: 7 with VL's and substituting the remaining Q's with I's. The amino acid sequence set forth in SEQ ID NO: 28 is obtained by substituting all QQ's in the amino acid sequence set forth in SEQ ID NO: 7 with VI's and substituting the remaining Q's with L's. The amino acid sequence set forth in SEQ ID NO: 29 is obtained by substituting all QQ's in the amino acid sequence set forth in SEQ ID NO: 7 with VF's and substituting the remaining Q's with I's.

The amino acid sequence set forth in SEQ ID NO: 30 is obtained by substituting all QQ's in the amino acid sequence set forth in SEQ ID NO: 8 (Met-PRT525) with VL's. The amino acid sequence set forth in SEQ ID NO: 31 is obtained by substituting all QQ's in the amino acid sequence set forth in SEQ ID NO: 8 with VL's and substituting the remaining Q's with I's.

The amino acid sequence set forth in SEQ ID NO: 32 is obtained by substituting all QQ's with VF's in a sequence in which a region of 20 domain sequences existing in the amino acid sequence set forth in SEQ ID NO: 7 (Met-PRT410) is repeated twice and substituting the remaining Q's with I's.

The amino acid sequence set forth in SEQ ID NO: 41 (Met-PRT917) is obtained by substituting all QQ's in the amino acid sequence set forth in SEQ ID NO: 7 with LI's and substituting the remaining Q's with V's. The amino acid

sequence set forth in SEQ ID NO: 42 (Met-PRT1028) is obtained by substituting all QQ's in the amino acid sequence set forth in SEQ ID NO: 7 with IF's and substituting the remaining Q's with T's.

The content rate of glutamine residues in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or lower (Table 2).

[Table 2]

| Modified fibroin | Content rate of glutamine residues | Content rate of GPGXX motifs | Degree of hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.36 |
| Met-PRT917 (SEQ ID NO: 41) | 0.0% | 27.9% | 0.46 |
| Met-PRT1028 (SEQ ID NO: 42) | 0.0% | 28.1% | 0.05 |

The artificial fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The artificial fibroin of (6-ii) has an amino acid sequence having a sequence identity of 90% or higher with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The artificial fibroin of (6-ii) is also a protein having a domain sequence represented by Formula 1: $[(A)_n \text{ motif-REP}]_m$ or Formula 2: $[(A)_n \text{ motif-REP}]_m\text{-}(A)_n$ motif. The sequence identity is preferably 95% or more.

The content rate of glutamine residues in the artificial fibroin of (6-ii) is preferably 9% or lower. In addition, the content rate of the GPGXX motifs in the artificial fibroin of (6-ii) is preferably 10% or higher.

The sixth artificial fibroin may have a tag sequence at one or both of the N-terminus and the C-terminus thereof. By containing a tag sequence, isolation, immobilization, detection, visualization, and the like of the artificial fibroin become possible.

More specific examples of the artificial fibroin having a tag sequence can include artificial fibroin having (6-iii) an amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028) and artificial fibroin having (6-iv) an amino acid sequence having a sequence identity of 90% or higher with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 are obtained by adding the amino acid sequence set forth in SEQ ID NO: 11 (which has a His-tag sequence and a hinge sequence) to the N-termini of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42, respectively. Since only the tag sequence is added to the N-termini, the content rates of glutamine residues do not change, and the content rate of glutamine residues in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is 9% or lower (Table 3).

[Table 3]

| Modified fibroin | Content rate of glutamine residues | Content rate of GPGXX motifs | Degree of hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The artificial fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The artificial fibroin of (6-iv) has an amino acid sequence having a sequence identity of 90% or higher with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The artificial fibroin of (6-iv) is also a protein having a domain sequence represented by Formula 1: $[(A)_n$ motif-REP$]_m$ or Formula 2: $[(A)_n$ motif-REP$]_m$-$(A)_n$ motif. The sequence identity is preferably 95% or more.

The content rate of glutamine residues in the artificial fibroin of (6-iv) is preferably 9% or lower. In addition, the content rate of the GPGXX motifs in the artificial fibroin of (6-iv) is preferably 10% or higher.

The sixth artificial fibroin may contain a secretory signal for releasing a protein produced in an artificial protein production system to the outside of a host. A sequence of the secretory signal is designed appropriately depending on the type of the host.

The artificial fibroin may have at least two or more of the characteristics among the characteristics of the first artificial fibroin, the second artificial fibroin, the third artificial fibroin, the fourth artificial fibroin, the fifth artificial fibroin, and the sixth artificial fibroin.

The artificial fibroin according to the present embodiment may be a protein having a domain sequence represented by Formula 1: [(A)n motif-REP]m or Formula 2: [(A)n motif-REP]m-(A)n motif. An amino acid sequence (N-terminal

sequence and C-terminal sequence) may be added to either or both of the N-terminus and the C-terminus of the domain sequence of the artificial fibroin. The N-terminal sequence and the C-terminal sequence are typically, but are not limited to, regions not having repeats of fibroin-specific amino acid motifs and having about 100 amino acid residues.

In the present specification, the term "domain sequence" is an amino acid sequence that generates a crystalline region (typically corresponding to $(A)_n$ motif of an amino acid sequence) and an amorphous region (typically corresponding to REP of an amino acid sequence) specific to fibroin and is represented by Formula 1: $[(A)_n$ motif-REP$]_m$ or Formula 2: $[(A)_n$ motif-REP$]_m$-$(A)_n$ motif. Here, the $(A)_n$ motif represents an amino acid sequence consisting of 4 to 27 amino acid residues, and the number of alanine residues with respect to the total number of amino acid residues in the $(A)_n$ motif is 80% or more. REP represents an amino acid sequence consisting of 10 to 200 amino acid residues. m represents an integer of 10 to 300. m is preferably an integer of 20 to 300, and more preferably an integer of 30 to 300. The plurality of $(A)_n$ motifs may be the same amino acid sequence or different amino acid sequences. A plurality of REPs may be identical or different amino acid sequences.

It is preferable that at least seven of the plurality of $(A)_n$ motifs in the domain sequence consist of only alanine residues. The phrase "consist of only alanine residues" means that the $(A)_n$ motif has an amino acid sequence represented by $(Ala)_k$ (where Ala represents an alanine residue). k preferably represents an integer of 4 to 27, more preferably an integer of 4 to 20, and still more preferably an integer of 4 to 16.

The REP consists of 10 to 200 amino acid residues. One or more of the amino acid residues constituting the REP may be an amino acid residue selected from the group consisting of a glycine residue, a serine residue, and an alanine residue. That is, the REP may have an amino acid residue selected from the group consisting of a glycine residue, a serine residue, and an alanine residue.

One or more of the amino acid residues constituting the REP may be a hydrophobic amino acid residue. That is, the REP preferably has a hydrophobic amino acid residue. The hydrophobic amino acid residue means an amino acid residue having a positive hydropathy index. As the hydropathy index (hereinafter, also referred to as "HI") of the amino acid residue, a known index known index is used (Hydropathy index: Kyte J, & Doolittle R (1982)" A simple method for displaying the hydropathic character of a protein", J. Mol. Biol., 157, pp. 105-132). Examples of the hydrophobic amino acid residue include isoleucine (HI: 4.5), valine (HI: 4.2), leucine (HI: 3.8), phenylalanine (HI: 2.8), methionine (HI: 1.9), and alanine (HI: 1.8).

In the artificial fibroin according to the present embodiment, it is preferable that the domain sequence has an amino acid sequence corresponding to an amino acid sequence in which a cysteine residue is inserted into an REP in comparison to naturally derived fibroin.

The domain sequence preferably has an amino acid sequence corresponding to an amino acid sequence in which a cysteine residue is inserted at a position adjacent to a glycine residue, a serine residue, or an alanine residue in an REP, and more preferably has an amino acid sequence corresponding to an amino acid sequence in which a cysteine residue is inserted at a position adjacent to a glycine residue in an REP. The cysteine residue in the REP may be located between the glycine residue, the serine residue, or the alanine residue and the glycine residue, the serine residue, or the alanine residue, or may be located between the serine residue and the glycine residue.

The domain sequence preferably has an amino acid sequence corresponding to an amino acid sequence in which a cysteine residue is inserted at a position adjacent to a hydrophobic amino acid residue in an REP. In this case, the hydrophobic amino acid residues are immobilized by a hydrophobic interaction between molecules. The cysteine residue in the REP may be located next to the hydrophobic amino acid residue, may be located between the hydrophobic amino acid residue and an amino acid residue other than the hydrophobic amino acid residue, may be located between the hydrophobic amino acid residue and the glycine residue, the serine residue, or the alanine residue, or may be located between the hydrophobic amino acid residue and the glycine residue. The hydrophobic amino acid residue may be one selected from the group consisting of an isoleucine residue, a valine residue, a leucine residue, a phenylalanine residue, a methionine residue, and an alanine residue.

The domain sequence may have an amino acid sequence corresponding to an amino acid sequence in which a cysteine residue is inserted into an REP located in the vicinity of an N-terminus and/or a C-terminus of the domain sequence, in comparison to naturally derived fibroin. In this case, the molecular chain can be lengthened. In the present specification, the REP located in the vicinity of the N-terminus of the domain sequence means an REP located at positions 1 to 3 from the N-terminus of the domain sequence. For example, the cysteine residue may be located in the REP located at positions 1 and 2 from the N-terminus of the domain sequence. In the present specification, the REP located in the vicinity of the C-terminus of the domain sequence means an REP located at positions 1 to 3 from the C-terminus of the domain sequence. For example, the cysteine residue may be located in the REP located at positions 1 and 2 from the C-terminus of the domain sequence. The cysteine residue is preferably located in the REP located closest to the N-terminal side and/or closest to the C-terminal side of the domain sequence.

The domain sequence may have an amino acid sequence corresponding to an amino acid sequence in which a cysteine residue is inserted into the center or the vicinity of the center thereof in an REP, in comparison to naturally derived fibroin. In the present specification, the vicinity of the center of the amino acid sequence in the REP indicates

positions 1 to 5 from the amino acid residue located in the center of the REP (the amino acid residue on the N-terminal side when two amino acid residues located in the center are present) toward the N-terminal side, or positions 1 to 5 from the amino acid residue located in the center of the REP (the amino acid residue on the C-terminal side when two amino acid residues located in the center are present). For example, the cysteine residue may be located in the middle of the REP, or may be located at positions 1 to 3 or 1 and 2 from the amino acid residue located in the middle of the REP toward the N-terminal side or the C-terminal side. Here, for example, in a case where a first segment containing a polypeptide skeleton in which cysteine residues are inserted so as to be located one by one on the N-terminal side and the C-terminal side of the domain sequence is used, a synthetic polymer in which a first segment and a second segment are alternately linked so as to be located one by one can be obtained. In a molded body (for example, a fiber, a film, a gel, or the like) obtained using the synthetic polymer, improvement in elongation can be expected. In addition, in a case where a first segment containing a polypeptide skeleton in which a cysteine residue is inserted so as to be located more centrally than the N-terminal side and the C-terminal side of the domain sequence is used, a synthetic polymer in which the second segment is linked to the first segment can be expected to have improved solubility in a solvent.

The artificial fibroin preferably contains a GPGXX motif (G represents a glycine residue, P represents a proline residue, and X represents an amino acid residue other than the glycine residue) in the amino acid sequence of the REP. The motif is contained in the REP, such that elongation of the artificial fibroin can be improved.

In a case where the artificial fibroin contains the GPGXX motif in REP, a content rate of the GPGXX motifs is generally 1% or higher. The content rate of the GPGXX motifs may be 5% or higher and is preferably 10% or higher. In this case, the stress of the artificial fibroin fiber is further increased. The upper limit of the content rate of the GPGXX motifs is not particularly limited and may be 50% or less or 30% or less.

In the present specification, the "content rate of the GPGXX motif" is a value calculated by the following method. The content rate of the GPGXX motifs is calculated as c/d in a case where in all REPs included in a sequence excluding the sequence from the $(A)_n$ motif located at the most C-terminal side to the C-terminal of the domain sequence in fibroin including a domain sequence represented by Formula 1: $[(A)_n$ motif-REP$]_m$ or Formula 2: $[(A)_n$ motif - REP$]_m$-$(A)_n$ motif, a number three times the total number of GPGXX motifs included in this region is defined as c (that is, a number corresponding to the total number of G and P in the GPGXX motif), and the total number of amino acid residues in all REPs excluding the sequence excluding the sequence from the $(A)_n$ motif located at the most C-terminal side to the C-terminal of the domain sequence from the domain sequence and further excluding the $(A)_n$ motifs is defined as d.

In the calculation of the content rate of the GPGXX motifs, the "sequence excluding the sequence from the $(A)_n$ motif located at the most C-terminal side to the C-terminus of the domain sequence from the domain sequence" is used to exclude the effect occurring due to the fact that the "sequence from the $(A)_n$ motif located at the most C-terminal side to the C-terminus of the domain sequence" (a sequence corresponding to REP) may include a sequence having a low correlation with the sequence characteristic of fibroin, which influences the calculation result of the content rate of the GPGXX motifs in a case where m is small (that is, in a case where the domain sequence is short). In a case where the "GPGXX motif" is located at the C-terminus of an REP, even when "XX" is "AA", for example, it is regarded as the "GPGXX motif".

Fig. 5 is a schematic view illustrating a domain sequence of fibroin. The method for calculating the content rate of the GPGXX motifs will be specifically described with reference to Fig. 5. First, in the domain sequence of the fibroin illustrated in Fig. 5 (which is the "$[(A)_n$ motif-REP$]_m$-$(A)_n$ motif" type), all REPs are included in the "sequence excluding the sequence from the $(A)_n$ motif located at the most C-terminal side to the C-terminus of the domain sequence from the domain sequence" (in Fig. 5, the sequence indicated as a "region A"), and therefore, the number of the GPGXX motifs for calculating c is 7, and c is $7 \times 3 = 21$. Similarly, since all REPs are included in the "sequence excluding the sequence from the $(A)_n$ motif located at the most C-terminal side to the C-terminus of the domain sequence from the domain sequence" (in Fig. 5, the sequence indicated as the "region A"), the total number d of the amino acid residues in all REPs when the $(A)_n$ motifs are further excluded from the sequence is 50 + 40 + 10 + 20 + 30 = 150. Next, "c" is divided by "d" to calculate c/d (%). In the fibroin shown in Fig. 5, c/d is 21/150 = 14.0%.

A degree of hydrophobicity of the REP in the artificial fibroin may be, for example, -0.80, -0.70, -0.06 or more, -0.50 or more, -0.40 or more, -0.30 or more, -0.20 or more, -0.10 or more, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. The upper limit of the degree of hydrophobicity of each REP is not particularly limited but may be 1.0 or less or 0.7 or less.

In the present specification, the "degree of hydrophobicity of each REP" is a value calculated by the following method. The degree of hydrophobicity of REP is calculated as e/f in a case where in all REPs included in a sequence excluding the sequence from the $(A)_n$ motif located at the most C-terminal side to the C-terminal of the domain sequence from the domain sequence (the sequence corresponding to the "region A" in Fig. 5) in fibroin including the domain sequence represented by Formula 1: $[(A)_n$ motif-REP$]_m$ or Formula 2: $[(A)_n$ motif -REP$]_m$-$(A)_n$ motif, the total sum of the hydropathy indices of each of amino acid residues in this region is defined as e, and the total number of amino acid residues in all REPs excluding the sequence from the $(A)_n$ motif located at the most C-terminal side to the C-terminal

of the domain sequence from the domain sequence and further excluding the $(A)_n$ motifs is defined as f. In the calculation of the degree of hydrophobicity of REP, the reason for targeting the "sequence excluding the sequence from the $(A)_n$ motif located at the most C-terminal side to the C-terminus of the domain sequence from the domain sequence" is the same as the reason described above.

The domain sequence may have an amino acid sequence corresponding to an amino acid sequence in which 1 or more and less than 16 cysteine residues are inserted into an REP, in comparison to naturally derived fibroin. That is, the total number of cysteine residues corresponding to the cysteine residues inserted into the REP may be 1 or more and less than 16. The total number of cysteine residues corresponding to the cysteine residues inserted into the REP may be 1 or more and 12 or less, 1 or more and 10 or less, 1 or more and 8 or less, 1 or more and 6 or less, or 2 or more and 4 or less. The number of cysteine residues per REP in the domain sequence may be, for example, 1 to 3, 1 or 2, or 1.

The total number of cysteine residues in the artificial fibroin according to the present embodiment may be 1 or more and less than 16, 1 or more and 12 or less, 1 or more and 10 or less, 1 or more and 8 or less, 1 or more and 6 or less, or 2 or more and 4 or less.

The artificial fibroin according to the present embodiment may be obtained by, for example, modification of an amino acid sequence corresponding to substitution, deletion, insertion, and/or addition of one or a plurality of amino acid residues in addition to modification related to the cysteine residue in the REP described above, in comparison to naturally derived fibroin.

The molecular weight of the artificial fibroin according to the present embodiment is not particularly limited, and may be, for example, 10 kDa or more and 700 kDa or less. The molecular weight of the artificial fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa, or may be 600 kDa or less, 500 kDa or less, 400 kDa or less, less than 360 kDa, 300 kDa or less, or 200 kDa or less.

The artificial fibroin according to the present embodiment preferably has an amino acid sequence corresponding to an amino acid sequence in which a cysteine residue is inserted, and more specific examples of the artificial fibroin can include (i) artificial fibroin having an amino acid sequence set forth in SEQ ID NO: 46, SEQ ID NO: 47, SEQ ID NO: 48, SEQ ID NO: 49, SEQ ID NO: 50, or SEQ ID NO: 51, and (ii) artificial fibroin having an amino acid sequence set forth in SEQ ID NO: 52, SEQ ID NO: 53, SEQ ID NO: 54, or SEQ ID NO: 55.

The amino acid sequence (PRT1) set forth in SEQ ID NO: 46 is obtained by inserting one cysteine residue into an REP located on the most N-terminal side of a domain sequence in the amino acid sequence (PRT11) set forth in SEQ ID NO: 56. That is, the total number of cysteine residues in the amino acid sequence (PRT1) set forth in SEQ ID NO: 46 is 1.

The amino acid sequence (PRT2) set forth in SEQ ID NO: 47 is obtained by inserting one cysteine residue into an REP located on the most N-terminal side of a domain sequence in the amino acid sequence (PRT12) set forth in SEQ ID NO: 57. That is, the total number of cysteine residues in the amino acid sequence (PRT2) set forth in SEQ ID NO: 47 is 1.

The amino acid sequence (PRT3) set forth in SEQ ID NO: 48 is obtained by inserting one cysteine residue into an REP located on each of the most N-terminal side and an REP located on the most C-terminal side of a domain sequence in the amino acid sequence (PRT12) set forth in SEQ ID NO: 57. That is, the total number of cysteine residues in the amino acid sequence (PRT3) set forth in SEQ ID NO: 48 is 2.

The amino acid sequence (PRT4) set forth in SEQ ID NO: 49 is obtained by inserting one cysteine residue into an REP located at positions 1 and 2 on each of the N-terminal side and the C-terminal side of a domain sequence in the amino acid sequence (PRT12) set forth in SEQ ID NO: 57. That is, the total number of cysteine residues in the amino acid sequence (PRT4) set forth in SEQ ID NO: 49 is 4.

The amino acid sequence (PRT5) set forth in SEQ ID NO: 50 is obtained by inserting one cysteine residue into an REP located at positions 1 to 4 on each of the N-terminal side and the C-terminal side of a domain sequence in the amino acid sequence (PRT12) set forth in SEQ ID NO: 57. That is, the total number of cysteine residues in the amino acid sequence (PRT5) set forth in SEQ ID NO: 50 is 8.

The amino acid sequence (PRT6) set forth in SEQ ID NO: 51 is obtained by inserting one cysteine residue into an REP located at positions 1 to 8 on each of the N-terminal side and the C-terminal side of a domain sequence in the amino acid sequence (PRT12) set forth in SEQ ID NO: 57. That is, the total number of cysteine residues in the amino acid sequence (PRT6) set forth in SEQ ID NO: 51 is 16.

The amino acid sequence (PRT7) set forth in SEQ ID NO: 52 is obtained by inserting one cysteine residue into an REP located on each of the most N-terminal side and the most C-terminal side of a domain sequence in the amino acid sequence (PRT13) set forth in SEQ ID NO: 58. That is, the total number of cysteine residues in the amino acid sequence (PRT7) set forth in SEQ ID NO: 52 is 2.

The amino acid sequence (PRT8) set forth in SEQ ID NO: 53 is obtained by inserting one cysteine residue into

an REP located on each of the most N-terminal side and the most C-terminal side of a domain sequence in the amino acid sequence (PRT14) set forth in SEQ ID NO: 59. That is, the total number of cysteine residues in the amino acid sequence (PRT8) set forth in SEQ ID NO: 53 is 2.

The amino acid sequence (PRT9) set forth in SEQ ID NO: 54 is obtained by inserting one cysteine residue into an REP located at positions 1 to 4 on each of the N-terminal side and the C-terminal side of a domain sequence in the amino acid sequence (PRT14) set forth in SEQ ID NO: 59. That is, the total number of cysteine residues in the amino acid sequence (PRT9) set forth in SEQ ID NO: 54 is 8.

The amino acid sequence (PRT10) set forth in SEQ ID NO: 55 is obtained by inserting one cysteine residue into an REP located at positions 1 to 8 on each of the N-terminal side and the C-terminal side of a domain sequence in the amino acid sequence (PRT14) set forth in SEQ ID NO: 59. That is, the total number of cysteine residues in the amino acid sequence (PRT10) set forth in SEQ ID NO: 55 is 16.

The artificial fibroin of (i) may have only an amino acid sequence set forth in SEQ ID NO: 46, SEQ ID NO: 47, SEQ ID NO: 48, SEQ ID NO: 49, SEQ ID NO: 50, or SEQ ID NO: 51. The artificial fibroin of (ii) may have only an amino acid sequence set forth in SEQ ID NO: 52, SEQ ID NO: 53, SEQ ID NO: 54, or SEQ ID NO: 55.

The artificial fibroin described above may have a tag sequence at either or both of the N-terminus and C-terminus. Therefore, isolation, immobilization, detection, visualization, and the like of the artificial fibroin are possible.

Examples of the tag sequence can include an affinity tag using specific affinity (binding properties or affinity) to another molecule. A specific example of the affinity tag can include a histidine tag (His tag). His tags are short peptides having about 4 to 10 histidine residues and specifically binding to metal ions such as nickel. Accordingly, it is possible to use a His tag to isolate an artificial fibroin by chelating metal chromatography. A specific example of the tag sequence includes an amino acid sequence set forth in SEQ ID NO: 70 or SEQ ID NO: 71 (amino acid sequence including a His tag).

In addition, it is possible to employ a tag sequence such as glutathione-S-transferase (GST) that specifically binds to glutathione or a maltose binding protein (MBP) that specifically binds to maltose.

An "epitope tag" utilizing an antigen-antibody reaction is employable. Adding a peptide (epitope) exhibiting antigenicity as a tag sequence allows binding of an antibody to the epitope. Examples of the epitope tag can include HA tag (peptide sequence of influenza hemagglutinin), myc tag, and FLAG tag. The use of the epitope tag allows purification of the artificial fibroin to be easily performed with high specificity.

Further, it is possible to use a tag sequence which is cleaved with a specific protease. By treating a protein adsorbed via the tag sequence with a protease, the artificial fibroin from which the tag sequence is cleaved can be recovered.

More specific examples of the artificial fibroin having a tag sequence can include (iii) artificial fibroin having an amino acid sequence set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), or SEQ ID NO: 65 (PRT20), and (iv) artificial fibroin having an amino acid sequence set forth in SEQ ID NO: 66 (PRT21), SEQ ID NO: 67 (PRT22), SEQ ID NO: 68 (PRT23), or SEQ ID NO: 69 (PRT24).

Note that the amino acid sequences set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), and SEQ ID NO: 65 (PRT20) are obtained by introducing tag sequences having an amino acid sequence set forth in SEQ ID NO: 70 into N-termini of the amino acid sequences set forth in SEQ ID NO: 46 (PRT1), SEQ ID NO: 47 (PRT2), SEQ ID NO: 48 (PRT3), SEQ ID NO: 49 (PRT4), SEQ ID NO: 50 (PRT5), and SEQ ID NO: 51 (PRT6), respectively. In addition, the amino acid sequences set forth in SEQ ID NO: 66 (PRT21), SEQ ID NO: 67 (PRT22), SEQ ID NO: 68 (PRT23), and SEQ ID NO: 69 (PRT24) are obtained by introducing tag sequences having an amino acid sequence set forth in SEQ ID NO: 70 into N-termini of the amino acid sequences set forth in SEQ ID NO: 52 (PRT7), SEQ ID NO: 53 (PRT8), SEQ ID NO: 54 (PRT9), and SEQ ID NO: 55 (PRT10), respectively.

The artificial fibroin of (iii) may have only an amino acid sequence set forth in SEQ ID NO: 60, SEQ ID NO: 61, SEQ ID NO: 62, SEQ ID NO: 63, SEQ ID NO: 64, or SEQ ID NO: 65.

Content rates of GPGXX motifs of the artificial fibroin having the amino acid sequences set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), and SEQ ID NO: 65 (PRT20) are 40.2%, 39.9%, 39.9%, 39.7%, 39.3%, and 38.6%, respectively, or are all 10% or more.

The artificial fibroin of (iv) may have only an amino acid sequence set forth in SEQ ID NO: 66, SEQ ID NO: 67, SEQ ID NO: 68, or SEQ ID NO: 69.

Content rates of GPGXX motifs of the artificial fibroin having the amino acid sequences set forth in SEQ ID NO: 66 (PRT21), SEQ ID NO: 67 (PRT22), SEQ ID NO: 68 (PRT23), and SEQ ID NO: 69 (PRT24) are 39.9%, 39.9%, 39.3%, and 38.6%, respectively, or are all 10% or more.

The above-mentioned artificial fibroin may include a secretory signal for releasing the protein produced in the recombinant protein production system to the outside of a host. A sequence of the secretory signal is designed appropriately depending on the type of the host.

[Nucleic acid]

The nucleic acid according to an embodiment encodes the artificial fibroin. Specific examples of the nucleic acid include nucleic acids encoding artificial fibroin having an amino acid sequence set forth in SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, or SEQ ID NO: 55, and artificial fibroin having an amino acid sequence (tag sequence) set forth in SEQ ID NO: 70 or SEQ ID NO: 71 attached to either or both of the N-terminus and C-terminus of each of these amino acid sequences.

The nucleic acid according to an embodiment is a nucleic acid hybridizing with a complementary strand of the nucleic acid encoding the artificial fibroin under stringent conditions and encoding artificial fibroin including a domain sequence represented by Formula 1: $[(A)_n \text{ motif-REP}]_m$, or Formula 2: $[(A)_n \text{ motif-REP}]_m\text{-}(A)_n$ motif. The domain sequence of the artificial fibroin encoded by the nucleic acid has an amino acid sequence corresponding to an amino acid sequence in which a cysteine residue is inserted into an REP in comparison to naturally derived fibroin.

The term "stringent conditions" refers to conditions under which a so-called specific hybrid is formed and a non-specific hybrid is not formed. The "stringent conditions" may be any of low stringent conditions, moderately stringent conditions and highly stringent conditions. The low stringent conditions mean that hybridization occurs only in the case where there is at least 85% or more identity between the sequences, and include, for example, conditions of hybridization at 42°C using 5×SSC containing 0.5% SDS. The moderately stringent conditions mean that hybridization occurs only in the case where there is at least 90% or more identity between the sequences, and include, for example, conditions of hybridization at 50°C using 5×SSC containing 0.5% SDS. The highly stringent conditions mean that hybridization occurs only in the case where there is at least 95% or more identity between the sequences, and include, for example, conditions of hybridization at 60°C using 5×SSC containing 0.5% SDS.

[Host and expression vector]

An expression vector according to an embodiment has the nucleic acid sequence and one or a plurality of regulatory sequences operably linked to the nucleic acid sequence. The regulatory sequence controls the expression of a recombinant protein in a host (for example, a promoter, an enhancer, a ribosome binding sequence, and a transcription termination sequence) and is appropriately selected depending on the type of the host. The type of the expression vector is appropriately selected depending on the type of the host. Examples of the expression vector include plasmid vector, viral vector, cosmid vector, fosmid vector, and artificial chromosome vector.

The host according to an embodiment is a host which has been transformed with the expression vector. Both prokaryotes and eukaryotes such as yeasts, filamentous fungi, insect cells, animal cells, and plant cells are suitably used as a host.

As the expression vector, an expression vector which can autonomously replicate in a host cell or can be incorporated into a chromosome of a host and which contains a promoter at a position capable of transcribing the nucleic acid according to an embodiment is suitably used.

In a case where a prokaryote such as a bacterium is used as a host, the expression vector according to an embodiment is preferably a vector which is capable of autonomous replication in the prokaryote and at the same time includes a promoter, a ribosome binding sequence, a nucleic acid according to an embodiment and a transcription termination sequence. The expression vector may contain a gene that controls the promoter.

Examples of the prokaryote can include microorganisms belonging to the genera *Escherichia, Brevibacillus, Serratia, Bacillus, Microbacterium, Brevibacterium, Corynebacterium,* and *Pseudomonas.*

Examples of the microorganisms belonging to the genus *Escherichia* can include *Escherichia coli* BL21 (Novagen, Inc.), *Escherichia coli* BL21(DE3) (Life Technologies Corporation), *Escherichia coli* BLR(DE3) (Merck Millipore), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No.49, *Escherichia coli* Rosetta(DE3) (Novagen, Inc.), *Escherichia coli* TB1, *Escherichia coli* Tuner (Novagen, Inc.), *Escherichia coli* Tuner(DE3) (Novagen, Inc.), *Escherichia coli* W1485, *Escherichia coli* W3110 (ATCC 27325), *Escherichia coli* XL1-Blue, and *Escherichia coli* XL2-Blue.

Examples of microorganisms belonging to the genus Brevibacillus can include Brevibacillus agri, Brevibacillus borstelensis, Brevibacillus centrosporus, Brevibacillus formosus, Brevibacillus invocatus, Brevibacillus laterosporus, Brevibacillus limnophilus, Brevibacillus parabrevis, Brevibacillus reuszeri, Brevibacillus thermoruber, Brevibacillus brevis 47 (FERM BP-1223), Brevibacillus brevis 47K (FERM BP-2308), Brevibacillus brevis 47-5 (FERM BP-1664), Brevibacillus brevis 47-5Q (JCM8975), Brevibacillus choshinensis HPD31 (FERM BP-1087), Brevibacillus choshinensis HPD31-S (FERM BP-6623), Brevibacillus choshinensis HPD31-OK (FERM BP-4573), and Brevibacillus choshinensis SP3 strain (manufactured by Takara Bio, Inc.).

Examples of microorganisms belonging to the genus Serratia can include Serratia liquefacience ATCC 14460, Serratia entomophila, Serratia ficaria, Serratia fonticola, Serratia grimesii, Serratia proteamaculans, Serratia odorifera,

Serratia plymuthica, and Serratia rubidaea.

Examples of microorganisms belonging to the genus Bacillus can include Bacillus subtilis and Bacillus amyloliquefaciens.

Examples of microorganisms belonging to the genus Microbacterium can include Microbacterium ammoniaphilum ATCC 15354.

Examples of microorganisms belonging to the genus Brevibacterium can include Brevibacterium divaricatum (Corynebacterium glutamicum) ATCC 14020, Brevibacterium flavum (Corynebacterium glutamicum ATCC 14067) ATCC 13826, ATCC 14067, Brevibacterium immariophilum ATCC 14068, Brevibacterium lactofermentum (Corynebacterium glutamicum ATCC 13869) ATCC 13665, ATCC 13869, Brevibacterium roseum ATCC 13825, Brevibacterium saccharolyticum ATCC 14066, Brevibacterium tiogenitalis ATCC 19240, Brevibacterium album ATCC 15111, and Brevibacterium cerinum ATCC 15112.

Examples of microorganisms belonging to the genus Corynebacterium can include Corynebacterium ammoniagenes ATCC 6871, ATCC 6872, Corynebacterium glutamicum ATCC 13032, Corynebacterium glutamicum ATCC 14067, Corynebacterium acetoacidophilum ATCC 13870, Corynebacterium·acetoglutamicum ATCC 15806, Corynebacterium alkanolyticum ATCC 21511, Corynebacterium callunae ATCC 15991, Corynebacterium glutamicum ATCC 13020, ATCC 13032, ATCC 13060, Corynebacterium lilium ATCC 15990, Corynebacterium melassecola ATCC 17965, Corynebacterium thermoaminogenes AJ12340 (FERMBP-1539), and Corynebacterium herculis ATCC 13868.

Examples of microorganisms belonging to the genus Pseudomonas can include Pseudomonas putida, Pseudomonas fluorescens, Pseudomonas brassicacearum, Pseudomonas fulva, and Pseudomonas sp. D-0110.

A method for introducing an expression vector into the host cell may employ any method as long as the method introduces DNA into the host cell. Examples thereof can include a method using calcium ions [Proc. Natl. Acad. Sci. USA, 69, 2110 (1972)], a protoplast method (Japanese Unexamined Patent Publication No. S63-248394), or a method described in Gene, 17, 107 (1982) or Molecular & General Genetics, 168, 111 (1979).

Transformation of microorganisms belonging to the genus *Brevibacillus* can be carried out by, for example, a method of Takahashi et al. (J. Bacteriol., 1983, 156: 1130-1134), a method of Takagi et al. (Agric. Biol. Chem., 1989, 53: 3099-3100), or a method of Okamoto et al. (Biosci. Biotechnol. Biochem., 1997, 61: 202-203).

Examples of the vector into which the nucleic acid according to an embodiment is introduced (hereinafter, simply referred to as "vector") can include pBTrp2, pBTac1, and pBTac2 (all commercially available from Boehringer Mannheim GmbH), pKK233-2 (manufactured by Pharmacia Corporation), pSE280 (manufactured by Invitrogen Corporation), pGEMEX-1 (manufactured by Promega Corporation), pQE-8 (manufactured by QIAGEN Corporation), pKYP10 (Japanese Unexamined Patent Publication No. S58-110600), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene Corporation), pTrs30 [constructed from *Escherichia coli* JM109/pTrS30 (FERM BP-5407)], pTrs32 [constructed from *Escherichia coli* JM109/pTrS32 (FERM BP-5408)], pGHA2 [constructed from *Escherichia coli* IGHA2 (FERM B-400), Japanese Unexamined Patent Publication No. S60-221091], pGKA2 [constructed from *Escherichia coli* IGKA2 (FERM BP-6798), Japanese Unexamined Patent Publication No. 60-221091], pTerm2 (US 4686191, US 4939094, US 5160735), pSupex, pUB110, pTP5, pC194, pEG400 [J. Bacteriol., 172, 2392 (1990)], pGEX (manufactured by Pharmacia Corporation), and pET systems (manufactured by Novagen, Inc.).

In the case where *Escherichia coli* is used as a host, for example, pUC18, pBluescriptII, pSupex, pET22b, pCold, and the like can be mentioned as a suitable vector.

Specific examples of a suitable vector for a microorganism belonging to the genus *Brevibacillus* can include pUB110 or pHY500 known as a *Bacillus subtilis* vector (JP H2-31682 A), pNY700 (JP H4-278091 A), pHY4831 (J. Bacteriol., 1987, 1239-1245), pNU200 (Shigezo Udaka, Journal of the Agricultural Chemical Society of Japan, 1987, 61: 669-676), pNU100 (Appl. Microbiol. Biotechnol., 1989, 30: 75-80), pNU211 (J. Biochem., 1992, 112: 488-491), pNU211R2L5 (JP H7-170984 A), pNH301 (Appl. Environ. Microbiol., 1992, 58: 525-531), pNH326, pNH400 (J. Bacteriol., 1995, 177: 745-749), pHT210 (JP H6-133782 A), pHT110R2L5 (Appl. Microbiol. Biotechnol., 1994, 42: 358-363), pNCO2 which is a shuttle vector between *Escherichia coli* and the microorganism belonging to the genus *Brevibacillus* (JP 2002-238569 A), and the like.

The promoter is not limited so long as it functions in the host cells. Examples of the promoter can include a promoter derived from *Escherichia coli,* a phage, or the like such as a trp promoter (Ptrp), a lac promoter, a PL promoter, a PR promoter, and a T7 promoter. It is also possible to use an artificially designed and modified promoter such as a promoter in which two Ptrp's are arranged in tandem (Ptrp×2), a tac promoter, a lacT7 promoter, and a let I promoter.

It is preferable to use a plasmid in which the distance between the Shine-Dalgarno sequence, which is a ribosome binding site, and the start codon is adjusted to an appropriate distance (for example, 6 to 18 bases). In the expression vector, a transcription termination sequence is not necessarily required for the expression of the nucleic acid, but it is preferable to arrange a transcription termination sequence immediately below a structural gene.

Examples of eukaryotic hosts include yeast, filamentous fungi (mold and the like), and insect cells.

Examples of the yeast can include yeasts belonging to the genera *Saccharomyces, Schizosaccharomyces,*

*Kluyveromyces, Trichosporon, Schwanniomyces, Pichia, Candida, Yarrowia,* and *Hansenula*. More specific examples of the yeast can include Saccharomyces cerevisiae, Schizosaccharomyces pombe, Kluyveromyces lactis, Kluyveromyces marxianus, Trichosporon pullulans, Schwanniomyces alluvius, Schwanniomyces occidentalis, Candida utilis, Pichia pastoris, Pichia angusta, Pichia methanolica, Pichia polymorpha, Pichia stipitis, Yarrowia lipolytica, and Hansenula polymorpha.

It is preferable that the expression vector in the case where yeast is used as a host cell usually include an origin of replication (in the case where amplification in a host is required), a selection marker for propagation of the vector in *Escherichia coli,* a promoter and a terminator for recombinant protein expression in yeast, and a selection marker for yeast.

In the case where the expression vector is a non-integrating vector, it is preferable to further include an autonomously replicating sequence (ARS). This makes it possible to improve the stability of the expression vectors in cells (Myers, A. M., et al. (1986) Gene 45: 299-310).

Examples of the vector in the case where yeast is used as a host can include YEP13(ATCC 37115), YEp24(ATCC 37051), YCp50(ATCC 37419), YIp, pHS19, pHS15, pA0804, pHIL3Ol, pHIL-S1, pPIC9K, pPICZ$\alpha$, pGAPZ$\alpha$, and pPICZ B.

The promoter is not limited as long as it can be expressed in yeast. Examples of the promoter can include a promoter of glycolytic genes such as hexose kinase, a PHOS promoter, a PGK promoter, a GAP promoter, an ADH promoter, a gal 1 promoter, a gal 10 promoter, a heat shock polypeptide promoter, an MF$\alpha$1 promoter, a CUP 1 promoter, a pGAP promoter, a pGCW14 promoter, an AOX1 promoter, and an MOX promoter.

As a method for introducing an expression vector into yeast, any method can be used as long as it is a method for introducing a DNA into yeast. Examples of a method for introducing an expression vector into yeast can include an electroporation method (Methods Enzymol., 194, 182 (1990)), a spheroplast method (Proc. Natl. Acad. Sci., USA, 81, 4889 (1984)), a lithium acetate method (J. Bacteriol., 153, 163 (1983)), and a method described in Proc. Natl. Acad. Sci. USA, 75, 1929 (1978).

Examples of the filamentous fungus can include fungi belonging to the genera Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus, and Rhizomucor.

Specific examples of filamentous fungi can include Acremonium alabamense, Acremonium cellulolyticus, Aspergillus aculeatus, Aspergillus awamori, Aspergillus oryzae, Aspergillus sake, Aspergillus sojae, Aspergillus tubigensis, Aspergillus niger, Aspergillus nidulans, Aspergillus parasiticus, Aspergillus ficuum, Aspergillus phoeicus, Aspergillus foetidus, Aspergillus flavus, Aspergillus fumigatus, Aspergillus japonicus, Trichoderma viride, Trichoderma harzianum, Trichoderma reseei, Chrysosporium lucknowense, Thermoascus, Sporotrichum, Sporotrichum cellulophilum, Talaromyces, Thielavia terrestris, Thielavia, Neurospora crassa, Fusarium oxysporus, Fusarium graminearum, Fusarium venenatum, Humicola insolens, Penicillium chrysogenum, Penicillium camemberti, Penicillium canescens, Penicillium emersonii, Penicillium funiculosum, Penicillium griseoroseum, Penicillium purpurogenum, Penicillium roqueforti, Myceliophtaora thermophilum, Mucor ambiguus, Mucor circinelloides, Mucor fragilis, Mucor hiemalis, Mucor inaequisporus, Mucor oblongiellipticus, Mucor racemosus, Mucor recurvus, Mocor saturninus, Mocor subtilissmus, Ogataea polymorpha, Phanerochaete chrysosporium, Rhizomucor miehei, Rhizomucor pusillus, and Rhizopus arrhizus.

The promoter in a case where the host is a filamentous fungus may be any of a gene related to glycolysis, a gene related to constitutive expression, an enzyme gene related to hydrolysis, and the like. Specific examples of the promoter in the case where the host is a filamentous fungus can include amyB, glaA, agdA, glaB, TEF1, xynF1 tannase gene, No. 8AN, gpdA, pgkA, enoA, melO, sodM, catA, and catB.

Introduction of the expression vector into a filamentous fungi can be carried out by a conventionally known method. Examples of the method for introducing the expression vector into a filamentous fungi can include a method of Cohen et al. (calcium chloride method) [Proc. Natl. Acad. Sci. USA, 69: 2110 (1972)], a protoplast method [Mol. Gen. Genet., 168: 111 (1979)], a competent method [J. Mol. Biol., 56: 209 (1971)], an electroporation method, and the like.

Examples of the insect cells include insect cells of Lepidoptera. More specific examples of the insect cells include insect cells derived from Spodoptera frugiperda such as Sf9 and Sf21, and insect cells derived from Trichoplusia ni such as High 5.

Examples of the vector in the case where an insect cell is used as a host can include baculoviruses such as Autographa californica nuclear polyhedrosis virus which is a virus that infects insects belonging to the family Noctuidae (Baculovirus Expression Vectors, A Laboratory Manual, W. H. Freeman and Company, New York (1992)).

In the case where an insect cell is used as a host, a polypeptide can be expressed by the method described in, for example, Current Protocols in Molecular Biology, Baculovirus Expression Vectors, A Laboratory Manual, W. H. Freeman and Company, New York (1992), or Bio/Technology, 6, 47 (1988). That is, a recombinant gene transfer vector and a baculovirus are co-introduced into an insect cell to obtain a recombinant virus (expression vector) in an insect cell culture supernatant, and then the recombinant virus is further infected into an insect cell, whereby the polypeptide can be expressed. Examples of the gene transfer vector used in the above method can include pVL1392, pVL1393, and pBlueBacIII (all manufactured by Invitorogen Corporation).

As a method for co-introducing a recombinant gene transfer vector and a baculovirus into an insect cell for

constructing the recombinant virus, for example, a calcium phosphate method (Japanese Unexamined Patent Publication No. H2-227075), a lipofection method (Proc. Natl. Acad. Sci. USA, 84, 7413 (1987)), or the like can be mentioned.

The recombinant vector according to an embodiment preferably further contains a selection marker gene for selecting a transformant. For example, in *Escherichia coli,* resistance genes for various drugs such as tetracycline, ampicillin, and kanamycin can be used as selection marker genes. A recessive selection marker capable of complementing a genetic mutation involved in auxotrophy can also be used. In yeast, a resistance gene for geneticin can be used as a selection marker gene, and a gene complementing a genetic mutation involved in auxotrophy, or a selection marker such as LEU2, URA3, TRP1, or HIS3 can also be used. Examples of the selection marker gene for filamentous fungi include a marker gene selected from the group consisting of niaD (Biosci. Biotechnol. Biochem., 59, 1795-1797 (1995)), argB (Enzyme Microbiol Technol, 6, 386-389, (1984)), sC (Gene, 84, 329-334, (1989)), ptrA (BiosciBiotechnol Biochem, 64, 1416-1421, (2000)), pyrG (BiochemBiophys Res Commun, 112, 284-289, (1983)), amdS (Gene, 26, 205-221, (1983)), aureobasidin resistance gene (Mol Gen Genet, 261, 290-296, (1999)), benomyl resistance gene (Proc Natl Acad Sci USA, 83, 4869-4873, (1986)) and hygromycin resistance gene (Gene, 57, 21-26, (1987)), and a leucine auxotrophy-complementing gene. Further, in the case where the host is an auxotrophic mutant strain, a wild-type gene complementing the auxotrophy can also be used as a selection marker gene.

The selection of the host transformed with the expression vector according to an embodiment can be carried out by plaque hybridization and colony hybridization using a probe that selectively binds to the nucleic acid. As the probe, it is possible to use a probe obtained by modifying a partial DNA fragment amplified by a PCR method based on sequence information of the nucleic acid with a radioisotope or digoxigenin.

[Production method of artificial fibroin]

The artificial fibroin according to the present embodiment can be produced by a method including a step of expressing the nucleic acid in a host transformed with the expression vector. As for the expression method, secretory production, fusion protein expression, or the like, in addition to direct expression, can be carried out according to the method described in Molecular Cloning, 2nd edition. In the case where it is expressed by yeast, an animal cell, or an insect cell, artificial fibroin can be obtained as a polypeptide to which a sugar or sugar chain is added.

The artificial fibroin according to the present embodiment can be produced, for example, by culturing a host transformed with the expression vector in a culture medium, producing and accumulating the artificial fibroin according to the present embodiment in the culture medium, and then collecting the artificial fibroin from the culture medium. A method for culturing the host in the culture medium can be performed according to a method generally used for culturing a host.

When the host is a prokaryote such as *Escherichia coli* or a eukaryote such as yeast, as the culture medium of the host, either a natural medium or a synthetic medium may be used as long as the medium contains a carbon source, a nitrogen source, an inorganic salt, and the like that can be assimilated by the host, and can efficiently culture the host.

The carbon source may be any carbon source that can be assimilated by the host. As the carbon source, for example, carbohydrates such as glucose, fructose, sucrose, molasses containing these examples, starch, and starch hydrolysate; organic acids such as acetic acid and propionic acid; and alcohols such as ethanol and propanol can be used.

Examples of the nitrogen source include ammonium salts of inorganic or organic acids such as ammonia, ammonium chloride, ammonium sulfate, ammonium acetate, and ammonium phosphate and also include other nitrogen-containing compounds, peptone, meat extract, yeast extract, corn steep liquor, casein hydrolysate, soybean cake, soybean cake hydrolysate, various fermenters, and digests thereof.

As the inorganic salt, for example, monopotassium phosphate, dipotassium phosphate, magnesium phosphate, magnesium sulfate, sodium chloride, iron(II) sulfate, manganese sulfate, copper sulfate, and calcium carbonate can be used.

Prokaryotes such as *Escherichia coli* or eukaryotes such as yeast are cultured under aerobic conditions by, for example, shaking or aeration and agitation in submerged culture. The culture temperature is, for example, 15 to 40°C. The culture time is typically 16 hours to 7 days. The pH of the culture medium during the culturing is preferably maintained at 3.0 to 9.0. The pH of the culture medium is adjusted using, for example, an inorganic acid, an organic acid, an alkali solution, urea, calcium carbonate, and ammonia.

In addition, antibiotics such as ampicillin and tetracycline may be optionally added to the culture medium during the culturing. In culturing of a microorganism transformed with an expression vector using an inducible promoter as a promoter, an inducer may be optionally added to the medium. For example, in culturing of a microorganism transformed with an expression vector using a lac promoter, isopropyl-β-D-thiogalactopyranoside may be added to the medium, and in culturing of a microorganism transformed with an expression vector using a trp promoter, indole acrylic acid may be added to the medium.

As a culture medium for insect cells, for example, commonly used TNM-FH medium (manufactured by Pharmingen Inc.), Sf-900 II SFM medium (manufactured by Life Technologies Corporation), ExCell 400 and ExCell 405 (both manufactured by JRH Biosciences Inc.), Grace's Insect Medium (Nature, 195, 788 (1962)), and the like can be used.

Culture of insect cells can be carried out, for example, for a culture time of 1 to 5 days under conditions such as pH 6 to 7 of culture medium and culture temperature 25 to 30°C. In addition, an antibiotic such as gentamicin may be added to the culture medium as necessary during the culture.

In the case where the host is a plant cell, the transformed plant cell may be directly cultured, or it may be differentiated into a plant organ and then cultured. As the culture medium for culturing a plant cell, for example, commonly used Murashige and Skoog (MS) medium, White medium, or a medium in which a plant hormone such as auxin or cytokinin is added to these media can be used.

Culture of animal cells can be carried out, for example, for a culture time of 3 to 60 days under conditions such as pH 5 to 9 of the culture medium and culture temperature 20 to 40°C. In addition, an antibiotic such as kanamycin or hygromycin may be added to the medium as necessary during the culture.

As a production method of artificial fibroin using a host transformed with the expression vector, there are a production method of the artificial fibroin in a host cell, a method for secreting the artificial fibroin outside the host cell, and a production method of the artificial fibroin on the outer membrane of the host cell. Each of these methods can be selected depending on the host cell to be used and the structure of the artificial fibroin to be produced.

For example, in the case where artificial fibroin is produced in the host cell or on the outer membrane of the host cell, the production method can be altered to actively secrete the artificial fibroin outside the host cell according to the method of Paulson et al. (J. Biol. Chem., 264, 17619 (1989)), the method of Lowe et al. (Proc. Natl. Acad. Sci. USA, 86, 8227 (1989), Genes Develop., 4, 1288 (1990)), or the methods described in JP 5-336963 A, WO 94/23021 A, and the like. That is, the artificial fibroin can be actively secreted outside the host cell by expressing the artificial fibroin in a form in which a signal peptide is added to a polypeptide containing an active site of artificial fibroin using a gene recombination technique.

The artificial fibroin produced by the host transformed with the expression vector can be isolated and purified by a method commonly used for protein isolation and purification. For example, in a case where the artificial fibroin is expressed being dissolved in a cell, after completion of the culturing, the host cell is collected by centrifugation and suspended in an aqueous buffer, and then, disrupted with an ultrasonicator, a French press, a Manton-Gaulin homogenizer, a Dyno-Mill, or the like, thereby obtaining a cell-free extract. A purified preparation is obtained from supernatant obtained by centrifuging the cell-free extract according to the following methods commonly used for protein isolation and purification or according to a combination of the following methods, that is, solvent extraction, salting-out using ammonium sulfate or the like, desalting, precipitation using an organic solvent, anion exchange chromatography using a resin such as diethylaminoethyl (DEAE)-sepharose or DIAION HPA-75 (available from Mitsubishi Kasei Kogyo Kabushiki Kaisha), cation exchange chromatography using a resin such as S-sepharose FF (available from Pharmacia Corporation), hydrophobic chromatography using a resin such as butyl sepharose and phenyl sepharose, gel filtration using a molecular sieve, affinity chromatography, chromatofocusing, and electrophoresis such as isoelectric focusing phoresis.

The aforementioned chromatographies may preferably employ phenyl-TOYOPEARL (Tosoh Corporation), DEAE-TOYOPEARL (Tosoh Corporation), and Sephadex G-150 (Pharmacia Biotech Inc.) .

In the case where the artificial fibroin is expressed by the formation of an insoluble matter in the cell, similarly, the host cells are recovered, disrupted and centrifuged to recover the insoluble matter of the artificial fibroin as a precipitated fraction. The recovered insoluble matter of the artificial fibroin can be solubilized with a protein denaturing agent. After this operation, a purified preparation of artificial fibroin can be obtained by the same isolation and purification method as described above.

In the case where artificial fibroin or a derivative in which a sugar chain has been added to the artificial fibroin is secreted extracellularly, the artificial fibroin or the derivative thereof can be recovered from the culture supernatant. In other words, culture supernatant is obtained by centrifugation of a culture or by treating the culture with other techniques, and from the culture supernatant, a purified preparation is yielded by isolation and purification in a similar manner.

(Second segment)

The second segment contains a molecular group having a plasticizing function for the polypeptide skeleton, the molecular group having the plasticizing function for the polypeptide skeleton refers to a molecular group in which an intermolecular force between the molecular groups is smaller than an intermolecular force between the polypeptide skeletons, and in a case where the molecular groups are mixed with each other, the flexibility of the material can be improved as compared with the polypeptide skeleton alone. In addition, the molecular group having the plasticizing function for the polypeptide skeleton can also be said to be a molecular group having a melting point or a glass transition temperature lower than that of the polypeptide skeleton. Examples of such a molecular group include polyethers described below. Note that the plasticizing function can also be said to be a function of improving flexibility or a function of increasing

an elongation at break in bending or pulling. In addition, as the molecular group having the plasticizing function for the polypeptide skeleton, a molecular group having biodegradability or a molecular group derived from biomass is suitably used. Therefore, it is sufficiently expected that the synthetic polymer (molding material) as a whole has biodegradability, and furthermore, the production energy of the synthetic polymer (molding material) can be reduced.

The second segment may contain a plurality of molecular groups. In addition, the second segment may contain a plurality of molecular groups, and the plurality of molecular groups may be linked to each other. In addition, the connection between the molecular groups may partially have a branch. Since the connection partially has a branch, the second segment can have a branched structure by the plurality of molecular groups, and a plurality of bonds with the first segment can be formed for each branch. That is, a network structure of the first segment and the second segment can also be formed by introducing a branch into a part of the connection. As described above, the second segment contains the plurality of molecular groups, and these molecular groups are connected to each other, such that the designed width of the second segment can be further expanded, and for example, the flexibility of the synthetic polymer can be more easily adjusted. Note that in a case where the second segment contains only one molecular group, a plurality of second segments may be connected to each other, and at least one of the plurality of second segments may be bonded to the first segment. Alternatively, a plurality of second segments may be bonded to one first segment. Note that in a case where the plurality of second segments are connected to each other, a branch may be introduced into a part of the connection between the plurality of second segments to form a network of the second segments.

The second segment may contain, for example, a skeleton derived from at least one selected from the group consisting of polyether, polyester, polycarbonate, polyamide, polyol (polyvinyl alcohol or the like), polyolefin, polyacetal, polyketal, poly(meth)acrylate, silicone, polyurethane, polyalkyleneimine, a phenolic resin, a urea resin, a melamine resin, and polysaccharides, and preferably, may include at least one functional group selected from the group consisting of a polyether group, a polyester group, a polycarbonate group, a polyamide group, a polyol group (polyvinyl alcohol group or the like), and a modified polysaccharide group, and may include at least one functional group selected from the group consisting of an ether group, an ester group, a carbonate group, an amide group, and s modified polysaccharide group. The second segment may have, for example, at least one structural unit selected from the group consisting of structural units having an ether bond, an ester bond, a carbonate ester bond (carbonate bond), an amide group, a siloxane bond, a urethane bond, a urethane bond, and a urea bond, or may have at least one structural unit selected from the group consisting of structural units having an alkylene group, a substituted alkylene group, an oxymethylene group, an alkyleneimine group, and a modified polysaccharide group.

Examples of the polyether group include functional groups derived from polyalkylene glycols such as polyethylene glycol, polypropylene glycol, an ethylene oxide/propylene oxide copolymer, and polybutylene glycol. Note that when these polyether groups are included in the second segment, the polyether group may be directly linked to a hetero element (O, N, and S) in an ester group or a thioester group, or an amide group of a linker contained in the second segment described below as necessary. Alternatively, the polyether group may be directly linked to a hetero element (O, N, and S) in an ester group or a thioester group, or an amide group of the polypeptide skeleton contained in the first segment. In this case, the entire polyether group is easily separated from the linker or the first segment, and as a result, the biodegradation rate of the polyether group can be increased.

Examples of the polyester group include functional groups derived from polyesters such as polylactic acid, poly(3-hydroxybutanoic acid), a polyhydroxybutanoic acid/hydroxyvaleric acid copolymer, a polyhydroxybutanoic acid/4-hydroxybutanoic acid copolymer, a polyhydroxybutanoic acid/hydroxyhexanoic acid copolymer, polytrimethylene terephthalate, a butanediol/long-chain dicarboxylic acid copolymer, polyethylene terephthalate, polybutylene succinate, a polybutylene succinate adipate copolymer, a polybutylene adipate terephthalate copolymer, polycaprolactone, and poly(trimethylene furandicarboxylate) (PTF). Among the polyesters described above, the polyester group is preferably a functional group derived from being classified into a biomass plastic such as polycaprolactone or a biodegradable plastic.

Examples of the polycarbonate group include functional groups derived from polycarbonates having an aliphatic hydrocarbon chain as a main skeleton, such as 1,6-hexanediol polycarbonate, 1,5-pentanediol polycarbonate, and 1,10 decanediol carbonate.

Examples of the polyamide group include functional groups derived from polyamides such as nylon 3, nylon 4, nylon 5, nylon 6, nylon 11, and nylon 610. Among the polyamides described above, the polyamide group is preferably a functional group derived from being classified into a biomass plastic or a biodegradable plastic.

Examples of the polyol group (polyvinyl alcohol group or the like) include functional groups derived from polyols (polyvinyl alcohols and the like) such as polyvinyl alcohol and an ethylene-vinyl alcohol copolymer. Among the polyols described above, the polyol group is preferably a functional group derived from being classified into a biomass plastic or a biodegradable plastic.

Examples of the modified polysaccharide group include functional groups derived from compounds obtained by chemically modifying cellulose, starch, chitin, chitosan, and the like. Examples of the chemically modified compound include cellulose acetate, ethyl cellulose, starch acetate, hydroxypropylated starch, carboxymethyl chitin, and carboxymethyl chitosan.

The second segment may further contain a linker (reactive functional group) in addition to the molecular group having the plasticizing function for the polypeptide skeleton. In this case, the molecular group and the polypeptide skeleton may be bonded via the linker. The linker may include, for example, at least one selected from the group consisting of a structural unit represented by the following Formula (1), structural units represented by the following General Formulas (2a) to (6), a structural unit represented by the following Formula (7), structural units represented by the following General Formulas (8a) to (9), structural units represented by the following General Formulas (10) to (11b), and structural units represented by the following General Formulas (13) to (16), may include at least one selected from the group consisting of a structural unit represented by the following Formula (1), structural units represented by the following General Formulas (2a) to (6), a structural unit represented by the following Formula (7), and structural units represented by the following General Formulas (8a) to (9), and preferably, from the viewpoint of improving biodegradability, may include at least one selected from the group consisting of a structural unit represented by the following Formula (1) and structural units represented by the following General Formulas (2a), (3a), (4a), (4b), and (10). The linker may have a plurality of structural units described above. That is, the linker may have only the structural unit described above, or may have a plurality of structural units described above (a plurality of structural units described above may be covalently bonded to the main body portion of the linker). The main body portion of the linker preferably has a relatively small molecular weight. In a case where the linker has three or more structural units described above, for example, three first segments can be bonded to one second segment, and a network structure can also be introduced into the synthetic polymer by such selection. In addition, a synthetic polymer in which some of the second segments that are plural are bonded to each other can be obtained by selecting a raw material used for producing the synthetic polymer (adjusting a functional group of a compound containing a polypeptide skeleton and a compound containing a molecular group having a plasticizing function for a polypeptide skeleton). In a case where the linker has a plurality of structural units described above, at least one of the plurality of structural units may be bonded to the polypeptide skeleton, and at least one of the plurality of structural units may be bonded to the molecular group. In a case where the linker has only the structural unit described above, the peptide skeleton is bonded to one of the two bonds described in the formula representing the structural unit representing the linker, and the molecular group is bonded to the other. For example, in a case where the linker is represented by the following Formula (1), preferably, the molecular group is bonded to nitrogen (N), and the other is bonded to a polypeptide skeleton. In a case where the linker has a plurality of structural units described above, for example, in a case of a structural unit represented by the following Formula (1), nitrogen (N) is preferably bonded to the main body of the linker, and the other is bonded to the polypeptide skeleton and the molecular group.

[Chem. 60]

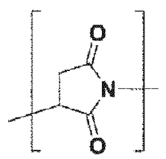

(1)

[Chem. 61]

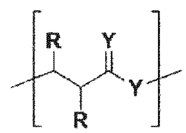

(2a)

[Chem. 62]

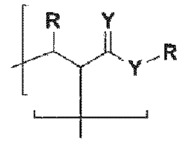

(2b)

[Chem. 63]

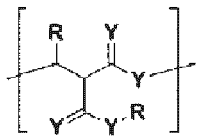

(3a)

[Chem. 64]

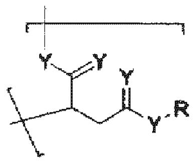

(4a)

[Chem. 65]

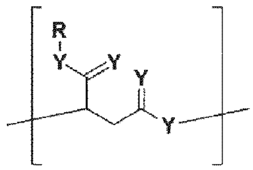

(4b)

[Chem. 66]

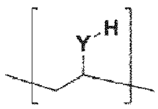

(5)

[Chem. 67]

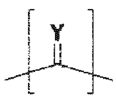

(6)

[Chem. 68]

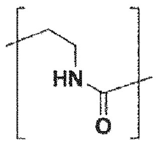

(7)

[Chem. 69]

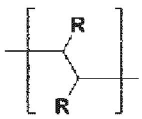

(8a)

[Chem. 70]

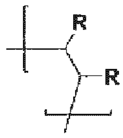

(8b)

[Chem. 71]

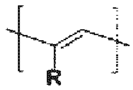

(9)

[Chem. 72]

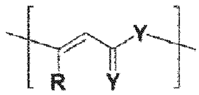

(10)

[Chem. 73]

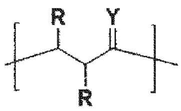

(11a)

[Chem. 74]

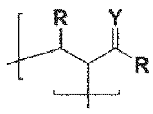

(11b)

[Chem. 75]

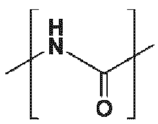

(13)

[Chem. 76]

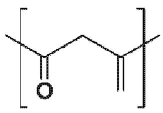

(14)

[Chem. 77]

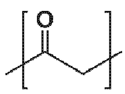

(15)

[Chem. 78]

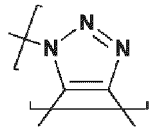

(16)

In General Formulas (2a), (2b), (3a), (4a), (4b), (5), (6), (10), (11a), and (11b), Y's each independently represent an oxygen atom, a sulfur atom, or $NR^1$. In $NR^1$, $R^1$ represents hydrogen, a hydrocarbon group, an aromatic group, a carbonyl group, or a sulfonyl group. In General Formulas (2a), (2b), (3a), (4a), (4b), (8a), (8b), (9), (10), (11a), and (11b), R's each independently represent hydrogen, a hydrocarbon group, or an aromatic group.

A molecular weight of the second segment (the molecular group having the plasticizing function for the polypeptide skeleton) is, for example, 200 to 500,000, 300 to 400,000, 350 to 350,000, 400 to 300,000, 500 to 200,000, 600 to 1,000,000, 700 to 50,000, 800 to 10,000, 900 to 7,500, or 100 to 5,000. This is because when the molecular weight of the molecular weight of the second segment is a low value of less than 200, localization of the molecular group having the plasticizing function in the three-dimensional structure of the molecule becomes difficult, and thus a weight ratio of the molecular group having the plasticizing function that is required to be introduced to exhibit a certain level of function is required to be increased more than necessary. As a result, there is a possibility that it is required to increase a reaction time in the production of the synthetic polymer or to increase a required reaction temperature. On the other hand, when the molecular weight of the molecular weight of the second segment exceeds 500,000, the molecular weight becomes excessively large, and the binding reactivity of the second segment to the first segment may be reduced. Note that the molecular weight of the second segment is a weight average molecular weight, and is generally determined by a known method using GPC.

The molecular weight of the second segment (the molecular group having the plasticizing function for the polypeptide skeleton) with respect to the molecular weight of the first segment (the polypeptide skeleton) can be appropriately adjusted according to the use of the synthetic polymer and the like. The molecular weight of the second segment (when two or more second segments are bonded to one first segment, the total molecular weight) is, for example, preferably 1 to 10,000, more preferably 1.5 to 9,000, still more preferably 2 to 8,000, still more preferably 3 to 7,000, still more preferably 5 to 5,000, still more preferably 7 to 3,000, and further still more preferably 10 to 2,000, based on the molecular weight of the first segment of 100. When the molecular weight of the second segment with respect to the molecular weight of the first segment is less than 1, plasticity (flexibility) may be insufficient in a molded body obtained using the synthetic polymer (molding material). On the other hand, when the molecular weight of the second segment with respect to the molecular weight of the first segment exceeds 10,000, plasticity is excessively increased, and rigidity of the molded body may be reduced. Within the above range, it is possible to produce a molded body having excellent flexibility. In addition, the molecular weight of the second segment and the molecular weight of the molecular group having the plasticizing function for the polypeptide skeleton may be, for example, 1.4 or more, 1.5 or more, 1.6 or more, 1.7 or more, 1.8 or more, 1.9 or more, 2.0 or more, 5.0 or more, 10 or more, 20 or more, 30 or more, or 40 or more, when the molecular weight of the first segment or the molecular weight of the polypeptide skeleton contained in the first segment is 100 (preferably, when the molecular weight of the polypeptide skeleton contained in the first segment is 100). Note that an upper limit value thereof is not particularly limited, and may be, for example, 100 or less, 80 or less, 70 or less, 60 or less, or 50 or less. Further, the molecular weight of the second segment is preferably within a range of 1 to 70, more preferably 1.5 to 60, still more preferably 1.5 to 50, and particularly preferably 2.0 to 50, when the molecular weight of the first segment is 100. In a case where a ratio of the molecular weight of the first segment (the polypeptide skeleton) to the molecular weight of the second segment (the molecular group having the plasticizing function for the polypeptide skeleton) is a value within the above range, for example, in a molded body obtained using a synthetic polymer, it can be expected to enhance the flexibility, stretchability, or the like of the entire synthetic polymer while sufficiently maintaining the characteristics (for example, high mechanical strength) of the first segment due to containing of a polypeptide skeleton. Note that a ratio of the molecular weight of the second segment when the molecular weight of the first segment is 100 is determined as a weight average molecular weight.

A content ratio of the first segment (the polypeptide skeleton) and the second segment (the molecular group having the plasticizing function for the polypeptide skeleton) in the synthetic polymer can be appropriately adjusted depending on the use of the synthetic polymer and the like. Such a content ratio of the first segment when the second segment is 100 in terms of mass ratio may be, for example, 50 or more, 60 or more, 70 or more, 80 or more, 90 or more, 100 or more, 110 or more, 150 or more, 200 or more, 250 or more, 300 or more, 300 or more, 400 or more, 450 or more, 500 or more, 550 or more, or 600 or more. An upper limit value of such a value is not particularly limited, and may be 1,000 or less, 900 or less, 800 or less, or 700 or less. Since the content ratio of the first segment and the second segment in the synthetic polymer is set to a value within the above range, wasteful use of the second segment is suppressed,

and the production cost of the synthetic polymer can be suppressed. Note that a reduction of the content ratio of the second segment to the first segment in the synthetic polymer can be advantageously realized by, for example, reducing the molecular weight of the first segment.

In the description of the above embodiment, the linker has been described as a constituent element of the second segment, but may be treated separately from the second segment. In addition, the linker can be treated as a constituent element of the first segment. Note that a molecular weight of the linker moiety is smaller than those of the polypeptide skeleton and the molecular group, but in a case where the linker is treated separately from the second segment, as for the molecular weight of the second segment, a range obtained by subtracting the molecular weight of the linker moiety is treated as a preferred range of the molecular weight of the second segment. Similarly, in a case where the linker moiety is a constituent element of the first segment, as for the molecular weight of the first segment, a range obtained by adding the molecular weight of the linker moiety is treated as a preferred range of the molecular weight of the first segment.

[Molding material and molded body]

An embodiment of the molding material contains the synthetic polymer described above. Since the molding material contains the synthetic polymer described above, a molded body having excellent flexibility can be produced. The molding material may contain other components in addition to the synthetic polymer described above. Examples of the other components include general-purpose polymers such as polyester, polyamide, and polysaccharides, and inorganic salts. In a case where the molding material contains other components, a content of the synthetic polymer may be, for example, 70 mass% or more, 80 mass% or more, or 90 mass% or more based on the total amount of the molding material.

An embodiment of the molded body contains the synthetic polymer described above. The molded body has excellent flexibility because it contains the synthetic polymer described above.

[Production method of synthetic polymer]

The synthetic polymer described above can be produced, for example, by introducing a functional group (for example, at least one functional group selected from the group consisting of a polyether group, a polyester group, a polycarbonate group, a polyamide group, a polyol group, and a modified polysaccharide group) including the molecular group into the first segment by generating a thioether group using a 1,4-addition reaction between a thiol group (also referred to as a mercapto group or a sulfhydryl group) of cysteine constituting a polypeptide skeleton and a carbon-carbon double bond of maleimide or a maleic acid derivative. The synthetic polymer described above can also be produced by applying an example of the production method described above, for example, by forming a thioether bond using the 1,4-addition reaction of a maleimide group described above, with a compound containing a polypeptide skeleton having cysteine, a compound containing a molecular group having a plasticizing function for the polypeptide skeleton and having a thiol group, and a compound having two or more maleimide groups. The synthetic polymer described above can also be produced, for example, by utilizing an addition reaction to an epoxy group or an isocyanate group, a substitution reaction to an $\alpha$-halocarbonyl group, or a Huisgen cyclization reaction between an alkynyl group and an azide group.

More generally, an embodiment of the production method of a synthetic polymer includes a step of obtaining a synthetic polymer by reacting a compound containing a polypeptide skeleton with at least one of compounds represented by the following General Formulas (1A) to (16A). The step may be a step of obtaining a synthetic polymer by reacting a compound containing a polypeptide skeleton with a compound represented by any one of the following General Formulas (1A) to (12A). The polypeptide skeleton includes at least one selected from the group consisting of a thiol group, an amino group, a hydroxy group, a guanidino group, a carboxy group, a phenoxy group, an indole group, an amide group, an azide group, and an alkynyl group, and preferably includes at least one selected from the group consisting of a thiol group, an amino group, a hydroxy group, a guanidino group, a carboxy group, a phenoxy group, an indole group, and an amide group. The reaction between the compound containing the polypeptide skeleton and the compound represented by any one of the following General Formulas (1A) to (12A) and (14A) may be a Michael addition reaction.

[Chem. 79]

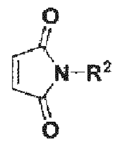

(1A)

[Chem. 80]

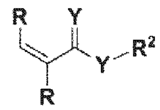

(2A)

[Chem. 81]

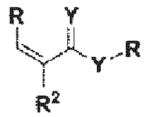

(2B)

[Chem. 82]

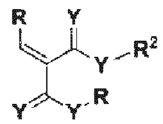

(3A)

[Chem. 83]

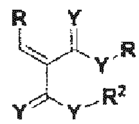

(3B)

[Chem. 84]

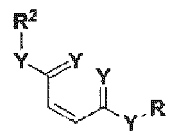

(4A)

[Chem. 85]

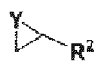

(5A)

[Chem. 86]

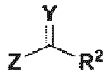 **(6A)**

[Chem. 87]

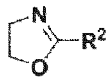 **(7A)**

[Chem. 88]

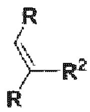 **(8A)**

[Chem. 89]

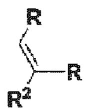 **(8B)**

[Chem. 90]

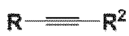 **(9A)**

[Chem. 91]

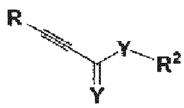 **(10A)**

[Chem. 92]

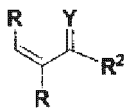 **(11A)**

[Chem. 93]

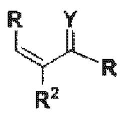 **(11B)**

[Chem. 94] **Z-$R^2$ (12A)**

[Chem. 95] **OCN-$R^2$ (13A)**

[Chem. 96]

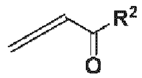 (14A)

[Chem. 97]

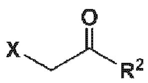 (15A)

[Chem. 98] **$N_3$-$R^2$ (16A)**

In General Formulas (1A), (2A), (2B), (3A), (3B), (4A), (5A), (6A), (7A), (8A), (8B), (9A), (10A), (11A), (11B), and (12A), $R^2$ represents a molecular group having a plasticizing function for the polypeptide skeleton. As the molecular group having the plasticizing function for the polypeptide skeleton, the examples described in the above description of the synthetic polymer can be applied.

In General Formulas (2A), (2B), (3A), (3B), (4A), (5A), (6A), (8B), (9A), (10A), (11A), and (11B), Y's each independently represent an oxygen atom, a sulfur atom, or $NR^1$. In $NR^1$, $R^1$ represents hydrogen, a hydrocarbon group, an aromatic group, a carbonyl group, or a sulfonyl group, and in General Formulas (2A), (2B), (3A), (3B), (4A), (5A), (8A), (8B), (9A), (10A), (11A), and (11B), R's each independently represent hydrogen, a hydrocarbon group, or an aromatic group. In General Formulas (6A) and (12A), Z represents a halogen atom, a sulfonic acid ester group, or a fluorine-containing carboxylic acid ester group. In General Formula (15A), X represents a halogen atom.

The number of molecular groups (second segments) having a plasticizing function for a polypeptide skeleton to be introduced into one compound containing a polypeptide skeleton (one first segment) is equal to or less than the total number of functional groups of a thiol group, an amino group, a hydroxy group, a guanidino group, a carboxy group, a phenoxy group, an indole group, an amide group, an azide group, and an alkynyl group (preferably, a thiol group, an amino group, a hydroxy group, a guanidino group, a carboxy group, a phenoxy group, an indole group, and an amide group) derived from an amino acid sequence constituting a polypeptide skeleton, and may be, for example, 1 or more, 2 or more, or 4 or more, and may be 30 or less, 25 or less, 20 or less, 15 or less, 10 or less, or 8 or less. The number of molecular groups (second segments) having a plasticizing function for a polypeptide skeleton to be introduced into one compound containing a polypeptide skeleton (one first segment) can be adjusted within the above range, and may be, for example, 1 to 30, 1 to 25, 1 to 20, 1 to 15, 1 to 10, 1 to 8, or 2 to 8.

In the production method of a synthetic polymer, for example, a compound containing a polypeptide skeleton and at least one of compounds represented by General Formulas (1A) to (16A) (preferably, compounds represented by General Formulas (1A) to (12A)) may be mixed in a solvent. In addition, in a case where a reaction is difficult to proceed, addition of an acid and a base, heating, and the like may be performed. The reaction temperature is usually 0 to 150°C, and may be, for example, 50 to 150°C, 70 to 120°C, 90 to 100°C, or may be 10 to 110°C, 20 to 100°C, or 25 to 90°C. The solvent to be used may be any solvent that can dissolve the compound containing the polypeptide skeleton and the compounds represented by General Formulas (1A) to (16A), and does not inhibit the progress of a desired reaction. Examples of the solvent include hexafluoroisopropyl alcohol (HFIP), dimethylsulfoxide (DMSO), dimethylformamide (DMF), dimethylacetamide (DMA), acetic acid, and formic acid.

The product (crude product containing a synthetic polymer) obtained by the above reaction may be purified by, for example, reprecipitation, normal phase column chromatography, reverse phase column chromatography, size exclusion chromatography, washing with a solvent, or the like.

An embodiment of the production method of a synthetic polymer includes a step of obtaining a synthetic polymer obtained by reacting a compound containing a polypeptide skeleton with a compound containing a molecular group having a plasticizing function for the polypeptide skeleton, and at least a compound having two or more structural units selected from the group consisting of structural units represented by General Formulas (2-1A) to (2-16A). The step may be a step of obtaining a synthetic polymer obtained by reacting a compound containing a polypeptide skeleton with a compound containing a molecular group having a plasticizing function for the polypeptide skeleton, and at least a compound having two or more functional groups selected from the group consisting of structural units represented by General Formulas (2-1A) to (2-12A).

The polypeptide skeleton includes at least one selected from the group consisting of a thiol group, an amino group, a hydroxy group, a guanidino group, a carboxy group, a phenoxy group, an indole group, an amide group, an

azide group, and an alkynyl group, and preferably includes at least one selected from the group consisting of a thiol group, an amino group, a hydroxy group, a guanidino group, a carboxy group, a phenoxy group, an indole group, and an amide group. The compound containing a molecular group includes at least one selected from the group consisting of a thiol group, an amino group, a hydroxy group, a guanidino group, a carboxy group, a phenoxy group, an indole group, an amide group, an azide group, and an alkynyl group, and preferably includes at least one selected from the group consisting of a thiol group, an amino group, a hydroxy group, a guanidino group, a carboxy group, a phenoxy group, an indole group, and an amide group. The functional group of the polypeptide skeleton and the functional group of the compound containing the molecular group can be selected according to the type of the functional group of at least the compound having two or more functional groups selected from the group consisting of structural units represented by General Formulas (2-1A) to (2-16A), and may be the same as or different from each other. In a case where the functional group of the polypeptide skeleton is different from the functional group of the compound containing a molecular group, the molecular structure of the synthetic polymer can be more easily controlled.

[Chem. 99]

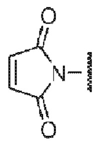

(2-1A)

[Chem. 100]

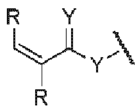

(2-2A)

[Chem. 101]

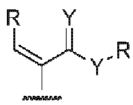

(2-2B)

[Chem. 102]

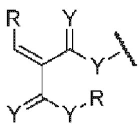

(2-3A)

[Chem. 103]

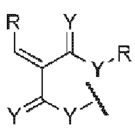

(2-3B)

[Chem. 104]

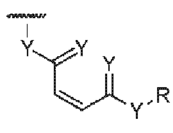

(2-4B)

[Chem. 105]

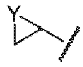

(2-5A)

[Chem. 106]

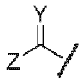

(2-6A)

[Chem. 107]

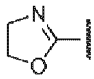

(2-7A)

[Chem. 108]

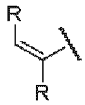

(2-8A)

[Chem. 109]

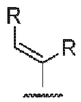

(2-8B)

[Chem. 110]

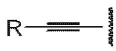

(2-9A)

[Chem. 111]

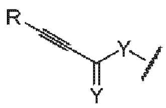

(2-10A)

[Chem. 112]

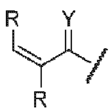

(2-11A)

[Chem. 113]

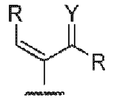

(2-11B)

[Chem. 114]

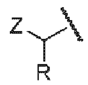

(2-12A)

[Chem. 115]

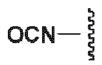

(2-13A)

[Chem. 116]

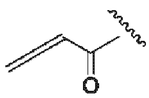

(2-14A)

[Chem. 117]

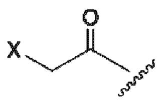

(2-15A)

[Chem. 118]

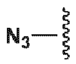

(2-16A)

In General Formulas (2-2A), (2-2B), (2-3A), (2-3B), (2-4A), (2-5A), (2-6A), (2-10A), (2-11A), and (2-11B), Y's each independently represent an oxygen atom, a sulfur atom, or $NR^1$. $R^1$ represents hydrogen, a hydrocarbon group, an aromatic group, a carbonyl group, or a sulfonyl group. In General Formulas (2-2A), (2-2B), (2-3A), (2-3B), (2-4A), (2-8A), (2-8B), (2-9A), (2-10A), (2-11A), (2-11B), and (2-12A), R's each independently represent hydrogen, a hydrocarbon group, or an aromatic group. In General Formulas (2-6A) and (2-12A), Z represents a halogen atom, a sulfonic acid ester group, or a fluorine-containing carboxylic acid ester group. In General Formula (2-15A), X represents a halogen atom.

The reaction between the compound containing the polypeptide skeleton and the compound containing a molecular group having a plasticizing function for the polypeptide skeleton and the compound having two or more specific functional groups described above may be, for example, a Michael addition reaction or the like.

In the production method of a synthetic polymer according to the present embodiment, a compound containing a polypeptide skeleton having a specific functional group, and a functional group that has a plasticizing function for a polypeptide skeleton and reacts with a compound containing a molecular group having a specific functional groups are separated as compounds having two or more specific structural units. By adopting such a production method, a reaction solution can be prepared without proceeding a reaction between a compound containing a polypeptide skeleton having a specific functional group and a compound having a plasticizing function for a polypeptide skeleton and having a molecular group having a specific functional group. Since the reaction does not proceed even when mixing is performed, it is possible to take time, for example, until a sufficiently uniform state is obtained. By blending a compound having two or more specific structural units after preparing a uniform reaction solution, the reaction system can be more easily controlled by starting the reaction, and a more uniform synthetic polymer can be prepared. Such a method is particularly

useful when solubility in a solvent or the like is low depending on the type of polypeptide skeleton or molecular group.

Hereinafter, several embodiments will be described, but the present disclosure is not limited to the embodiments. In addition, the description contents of the embodiments described above can be applied to each other.

## Examples

Hereinafter, the present disclosure will be described more specifically based on Examples and the like. However, the present disclosure is not limited to the following Examples.

[Production of artificial fibroin]

(1) Preparation of expression vector

Artificial fibroins having the amino acid sequence (PRT27) set forth in SEQ ID NO: 72, the artificial fibroin having the amino acid sequence (PRT17) set forth in SEQ ID NO: 62, and the artificial fibroin having the amino acid sequence (PRT18) set forth in SEQ ID NO: 63 were designed. Note that a value of an average hydropathy index of the artificial fibroin having the amino acid sequence (PRT27) set forth in SEQ ID NO: 72 is 0.44, a value of an average hydropathy index of the artificial fibroin having the amino acid sequence (PRT17) set forth in SEQ ID NO: 62 is 0.45, and a value of an average hydropathy index of the artificial fibroin having the amino acid sequence (PRT18) set forth in SEQ ID NO: 63 is 0.46.

The amino acid sequence (PRT17) set forth in SEQ ID NO: 62 is obtained by inserting one cysteine residue into an REP located at position 1 on each of the N-terminal side and the C-terminal side of a domain sequence in the amino acid sequence (PRT27) set forth in SEQ ID NO: 72. The total number of cysteine residues in the artificial fibroin having the amino acid sequence set forth in SEQ ID NO: 62 is 2. The amino acid sequence (PRT18) set forth in SEQ ID NO: 63 is obtained by inserting one cysteine residue into an REP located at positions 1 and 2 on each of the N-terminal side and the C-terminal side of a domain sequence in the amino acid sequence (PRT27) set forth in SEQ ID NO: 72. The total number of cysteine residues in the artificial fibroin having the amino acid sequence set forth in SEQ ID NO: 63 is 4.

Each of nucleic acids encoding the artificial fibroins having the amino acid sequences set forth in SEQ ID NO: 62, SEQ ID NO: 63, and SEQ ID NO: 72 was synthesized. In the nucleic acid, an NdeI site was added to the 5' end and an EcoRI site was added downstream of the stop codon. The nucleic acid was cloned into a cloning vector (pUC118). Thereafter, the nucleic acid was enzymatically cleaved by treatment with NdeI and EcoRI, and then, recombined into the protein expression vector pET-22b(+), thereby obtaining an expression vector.

(2) Production of protein

*Escherichia coli* BLR (DE3) was transformed with the obtained expression vector. The transformed *Escherichia coli* was cultured in 2 mL of an LB culture medium containing ampicillin for 15 hours. The culture solution was added to a 100 mL seed culture medium containing ampicillin (Table 4) so that $OD_{600}$ was 0.005. The culture solution was maintained at a temperature of 30°C, and put in a culture flask (for about 15 hours) until $OD_{600}$ reached 5, thereby obtaining a seed culture solution.

[Table 4]

| Seed culture medium | |
|---|---|
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture medium was added to a jar fermenter to which a 500 mL production medium (Table 5) was added so that $OD_{600}$ was 0.05. Culture was performed while maintaining the culture solution temperature at 37°C and controlling the pH to be constant at 6.9. Further, the culture solution was maintained to have a 20% dissolved oxygen concentration of the saturated dissolved oxygen concentration.

[Table 5]

| Production medium | |
|---|---|
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADECANOL (ADEKA Corporation, LG-295S) | 0.1 (mL/L) |

Immediately after glucose in the growing medium was completely consumed, a feed solution (455 g/1 L of glucose, 120 g/1 L of Yeast Extract) was added at a speed of 1 mL/min. Culture was performed while maintaining the culture solution temperature at 37°C and controlling the pH to be constant at 6.9. The culturing was performed for 20 hours while a dissolved oxygen concentration in the culture solution was maintained at 20% of the saturated dissolved oxygen concentration. The expression of the artificial fibroin was then induced by adding 1 M isopropyl-β-thiogalactopyranoside (IPTG) to the culture solution so that the final concentration was 1 mM. The culture solution was centrifuged for 20 hours after the addition of IPTG, and bacterial cells were collected. SDS-PAGE was conducted using the bacterial cells prepared from the culture solutions obtained before the addition of IPTG and after the addition of IPTG. The expression of the target artificial fibroin which depended on the addition of IPTG was confirmed by the appearance of a band of the size of the target artificial fibroin.

(3) Purification of protein

Bacterial cells that were collected 2 hours after the addition of IPTG were washed with 20 mM Tris-HCl buffer (pH 7.4). The washed bacterial cells were suspended in 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the precipitate reached a high level of purity. The washed precipitate was suspended in a 8 M guanidine buffer (8 M guanidine hydrochloride, 10 mM sodium dihydrogen phosphate, 20 mM NaCl, 1 mM Tris-HCl, pH 7.0) so that the precipitate reached a concentration of 100 mg/mL, and then, the precipitate was dissolved by being stirred with a stirrer at 60°C for 30 minutes. After the dissolving, the resultant was dialyzed with water using a dialysis tube (cellulose tube 36/32, available from Sanko Junyaku Co., Ltd.). A white coagulation protein obtained after dialysis was collected by centrifugation, and water was removed in a freeze dryer to recover freeze-dried powder, thereby obtaining powdered artificial fibroins (PRT17, PRT18, and PRT27). It was confirmed that a disulfide bond was formed between molecules of artificial fibroin into which a cysteine residue was inserted. It was confirmed that a dimer, a trimer, and a tetramer were contained in the artificial fibroin in amounts of 13.8 parts by mass, 2.1 parts by mass, and 0.8 parts by mass, respectively, with respect to 100 parts by mass of the monomer. The formation of disulfide bonds was measured by SDS-PAGE.

(Example 1)

A synthetic polymer was prepared using methoxy polyethylene glycol maleimide (manufactured by Sigma-Aldrich Inc.: second segment) having a number average molecular weight of 5,000. Specifically, in a glass container, 2,500 mg of artificial fibroin (first segment) having the amino acid sequence (PRT18) set forth in SEQ ID NO: 63 prepared as described above and having a number average molecular weight of 50,000 and 500 mg of the methoxy polyethylene glycol maleimide were weighed, 50 mL of hexafluoroisopropyl alcohol (manufactured by Central Glass Co., Ltd., HFIP) was further added as a solvent, and the mixture was stirred to dissolve the artificial fibroin, thereby obtaining a reaction solution. Thereafter, the reaction was performed by stirring the reaction solution at room temperature (25°C) for 20 hours.

When 20 hours had elapsed, the solvent was distilled off, and the crude product was washed three times with methanol (manufactured by Nacalai Tesque Inc.). 50 mL of methanol was used for one washing. Unreacted products such as methoxy polyethylene glycol maleimide were removed by a washing operation. After the washing operation, drying was performed under reduced pressure to obtain a product (synthetic polymer). An increase in molecular weight

was confirmed by SDS-PAGE for the product, and it was confirmed that a polyethylene glycol chain was introduced into artificial fibroin. It was confirmed that 1 to 4 second segments were bonded to one first segment in the obtained synthetic polymer. Fig. 6 illustrates results of SDS-PAGE. In Fig. 6, Lanes 1 and 7 are standard samples for specifying the molecular weight, Lanes 2 and 3 are artificial fibroins having the amino acid sequence (PRT18) set forth in SEQ ID NO: 63, and Lane 4 is the synthetic polymer prepared in Example 1.

(Example 2)

A synthetic polymer was prepared in the same manner as that of Example 1, except that methoxy polyethylene glycol maleimide (manufactured by Sigma-Aldrich Inc.) having a number average molecular weight of 10,000 was used, the blending amount of methoxy polyethylene glycol maleimide was 20 mg, the amount of artificial fibroin was 50 mg, 1 mL of hexafluoroisopropyl alcohol (Central Glass Co., Ltd., HFIP) was added as a solvent, and 1 mL of methanol was used for one washing of the crude product. An increase in molecular weight was confirmed by SDS-PAGE for the product, and it was confirmed that a polyethylene glycol chain was introduced into artificial fibroin. It was confirmed that 1 to 4 second segments were bonded to one first segment in the obtained synthetic polymer. In Fig. 6, Lane 5 is the constituent polymer prepared in Example 2.

(Example 3)

A synthetic polymer was prepared in the same manner as that of Example 2, except that methoxy polyethylene glycol maleimide (manufactured by Sigma-Aldrich Inc.) having a number average molecular weight of 750 was used, and the blending amount of methoxy polyethylene glycol maleimide was 1.5 mg. An increase in molecular weight was confirmed by SDS-PAGE for the product, and it was confirmed that a polyethylene glycol chain was introduced into artificial fibroin. It was confirmed that 1 to 4 second segments were bonded to one first segment in the obtained synthetic polymer. In Fig. 6, Lane 6 is the constituent polymer prepared in Example 3.

(Comparative Example 1)

For comparison, the same experiment was performed using artificial fibroin without cysteine. That is, in a glass container, 20 mg of artificial fibroin (number average molecular weight: 100,000) having the amino acid sequence (PRT27) set forth in SEQ ID NO: 72 prepared as described above and 4 mg of methoxy polyethylene glycol maleimide (manufactured by Central Glass Co., Ltd., HFIP) having a number average molecular weight of 5,000 were weighed, 1 mL of hexafluoroisopropanol (manufactured by Sigma-Aldrich Inc.) was further added as a solvent, and the mixture was stirred to dissolve the artificial fibroin, thereby obtaining a reaction solution. Thereafter, the reaction was performed by stirring the reaction solution at room temperature (25°C) for 20 hours.

When 20 hours had elapsed, the solvent was distilled off, and the crude product was washed three times with methanol (manufactured by Nacalai Tesque Inc.). 1 mL of methanol was used for one washing. Unreacted products such as methoxy polyethylene glycol maleimide were removed by a washing operation. After the washing operation, drying was performed under reduced pressure to obtain a product. It was confirmed by SDS-PAGE for the product that there was no change in molecular weight, and it was confirmed that the reaction between artificial fibroin and methoxy polyethylene glycol maleimide did not proceed. Fig. 7 illustrates results of SDS-PAGE. In Fig. 7, Lane 1 is a standard sample for specifying the molecular weight, 6 Lane is a product, and Lane 7 is the artificial fibroin having the amino acid sequence (PRT27) set forth in SEQ ID NO: 72.

(Comparative Example 2)

A mixture of artificial fibroin (number average molecular weight: 100,000) having the amino acid sequence (PRT27) set forth in SEQ ID NO: 72 prepared as described above and polyethylene oxide was prepared. Specifically, 10 g of the artificial fibroin and polyethylene oxide (number average molecular weight: 4,000) were blended in a container so as to have a weight of 5 mass% with respect to 100 mass% of the total amount of the artificial fibroin, and dry-mixing was performed under a condition of a rotation speed of 1,000 times/min using a bead mill (manufactured by NIPPON COKE & ENG. COMPANY, LIMITD, Attritor) for 10 minutes, thereby preparing a mixture.

(Comparative Example 3)

A mixture was prepared in the same manner as that of Comparative Example 2, except that polyethylene oxide having a number average molecular weight of 20,000 was used as the polyethylene oxide.

(Comparative Example 4)

A mixture of artificial fibroin (number average molecular weight: 100,000) having the amino acid sequence (PRT27) set forth in SEQ ID NO: 72 prepared as described above and polyethylene oxide monostearate (40E.O., number average molecular weight: 2,044) was prepared. Polyethylene oxide monostearate was blended so as to have a weight of 10 mass% with respect to 100 mass% of the total amount of the artificial fibroin, mixing was performed in ethanol (99.5%) for 15 hours, and drying was performed in an oven at 60°C for 5 hours, thereby preparing a mixture.

<Evaluation of molded body>

Molded bodies were prepared using the synthetic polymers prepared in Examples 1 to 3 and the mixtures prepared in Comparative Examples 2, 3, and 4, respectively, by heat and pressure molding under the following conditions. A pressure molding machine 10 illustrated in Fig. 8 was used for molding. Fig. 8 is a schematic cross-sectional view of the pressure molding machine. The pressure molding machine 10 illustrated in Fig. 8 includes a mold 2 capable of heating in which a through-hole is formed, and an upper pin 4 and a lower pin 6 capable of moving up and down within the through-hole of the mold 2. A sample is introduced into a gap formed by inserting the upper pin 4 or the lower pin 6 into the mold 2, and the composition is compressed by the upper pin 4 and the lower pin 6 while the mold 2 is heated, such that a molded body can be obtained.

A step of obtaining a molded body will be described with reference to Fig. 9. Fig. 9(a), 9(b), and 9(c) are schematic cross-sectional views of the pressure molding machine before introduction of a composition, after the introduction of the composition, and in a state where the composition is heated and pressurized, respectively. As illustrated in Fig. 9(a), the composition is introduced into the through-hole in a state where only the lower pin 6 is inserted into the through-hole of the mold 2, and as illustrated in Fig. 9 (b), the upper pin 4 is inserted into the through-hole of the mold 2 and lowered to start heating the mold 2, and a sample 8a before heating and pressurization is heated and pressurized in the through-hole. The upper pin 4 is lowered until the pressurizing force reaches a predetermined pressurizing force, and heating and pressurization are continued until the sample reaches a predetermined temperature in the state illustrated in Fig. 9(c) to obtain a molded body 8b after heating and pressurization. In some cases, the temperature of the mold 2 may be lowered using a cooler (for example, a spot cooler), and when the molded body 8b reaches a predetermined temperature, the upper pin 4 or the lower pin 6 may be removed from the mold 2 to obtain a molded body. Regarding the pressurization, the upper pin 4 may be lowered in a state where the lower pin 6 is fixed, but both the lowering of the upper pin 4 and the lifting of the lower pin 6 may be performed.

Specifically, first, 0.6 g of the synthetic polymer or the mixture was weighed and introduced into the through-hole of the mold 2 (in Example 1, a cylindrical mold having a circular through-hole having a diameter of 14 mm was used, and in Comparative Examples 2, 3, and 4, a cylindrical mold having a rectangular through-hole with a cross section of 35 mm × 25 mm was used) of the pressure molding machine 10 illustrated in Fig. 8. At this time, the sample was added so that the thickness was uniform. After all the samples were introduced, heating of the mold 2 was started, and the samples were pressurized by inserting the upper pin 4 and the lower pin 6 into the through-hole using a hand press machine (manufactured by NPa SYSTEM CO., LTD, trade name: NT-100H-V09). At this time, the pressurization condition of the sample was controlled to be 50 MPa. When 5 minutes had elapsed after the temperature of the sample reached 150°C, the heating was stopped, the sample was cooled with a spot cooler (TRUSCO NAKAYAMA CORPORATION, trade name: TS-25EP-1), and the sample was taken out when the temperature of the sample reached 50°C, and deburring was performed. Thereafter, a disk-shaped molded body (in Comparative Examples 2, 3, and 4, a rectangular parallelepiped molded body having a length of 35 mm × a width of 25 mm × a thickness of 3.0 mm) having a diameter of 14 mm × a thickness of 3.0 mm was obtained. Note that all the moisture contents in the molding material and the mixture used for molding were 7 to 9 mass% when measured with a heat-drying type moisture meter.

The obtained molded body was subjected to visual observation for appearance and finger touch observation for bleed-out, and was evaluated according to the following criteria. The results are shown in Table 6. In addition, the appearance of the molded body is illustrated in each of Figs. 10, 11, and 12.

[Evaluation criteria of appearance]

A: The appearance is uniform.
B: The appearance is uniform, but turbidity is observed.
C: The appearance is not uniform.

[Evaluation criteria of bleed-out]

A: There is no stickiness.
B: There is stickiness immediately after molding, but there is no stickiness after wiping off.
C: Stickiness remains even after wiping off, or stickiness occurs again.

[Table 6]

| | Appearance | Bleed-out |
|---|---|---|
| Example 1 | A | A |
| Example 2 | A | - |
| Example 3 | A | - |
| Comparative Example 2 | B | A |
| Comparative Example 3 | C | A |
| Comparative Example 4 | B | C |

In Table 6, "-" means that measurement is not performed. As can be seen from the results shown in Table 6 and illustrated in Figs. 10, 11, and 12, since the first segment and the second segment were bonded directly to each other, phase separation or the like was not confirmed in Examples 1 to 3. On the other hand, in Comparative Examples 1 to 4, it was confirmed that phase separation or bleed-out occurred in the mixture of the artificial fibroin and the flexible polymer.

(Example 4)

A film containing the synthetic polymer prepared in Example 1 and the artificial fibroin (number average molecular weight: 100,000) having the amino acid sequence (PRT27) set forth in SEQ ID NO: 72 prepared as described above was molded. Specifically, 4.4 g of dimethyl sulfoxide was weighed in a container, 0.15 g of the artificial fibroin was weighed therein, and the mixture was stirred under nitrogen under a condition of 120°C for 15 minutes to dissolve the artificial fibroin. 0.15 g of the artificial fibroin was further added, and the mixture was stirred under nitrogen under a condition of 120°C for 30 minutes, thereby preparing a solution in which a total of 0.30 g of the artificial fibroin was dissolved. In the solution, 0.15 g of the synthetic polymer was weighed and stirred under nitrogen under a condition of 90°C for 15 minutes to dissolve the synthetic polymer. 0.15 g of the synthetic polymer was further added, and the mixture was stirred under nitrogen under a condition of 90°C for 30 minutes, thereby preparing a dope solution in which 0.30 g of each of the artificial fibroin and the synthetic polymer was dissolved. In the dope solution, a mixing ratio was adjusted so that synthetic polymer:artificial fibroin:dimethyl sulfoxide was 6 mass%:6 mass%:88 mass%.

The dope solution was allowed to stand at 80°C for 30 minutes to defoam the dope solution. A film was molded from the dope solution after defoamation using a coater (manufactured by Imoto machinery Co., LTD.). First, a PET film (150 mm × 200 mm) as a release member was placed on a coater, and a liquid film of the dope solution was provided in the form of the PET film under the conditions of an applicator thickness of 0.5 mm, a coating speed of 20 m/min, and a temperature of 40°C. Thereafter, the liquid film of the dope solution was allowed to stand together with the PET film in an air blowing oven, drying was performed under a condition of 100°C for 15 minutes, transfer to a vacuum oven was further performed, vacuum-drying was performed under a condition of 80°C for 15 hours, and absolute drying was performed, thereby preparing a film-shaped molded body (film) formed of a synthetic polymer and artificial fibroin.

The prepared film was clear and colorless, and flexibility was observed. It was confirmed that the film was not cracked even when a bending stress was applied.

(Example 5)

2,300 mg of the artificial fibroin (number average molecular weight: 50,000, first segment) having the amino acid sequence (PRT17) set forth in SEQ ID NO: 62 prepared as described above and 900 mg of polyethylene glycol bismaleimide (manufactured by Biochempeg Scientific Inc., HO022022-20K, second segment) having a number average molecular weight of 20,000 were dissolved in 115 mL of a solvent (DMSO, manufactured by Kishida Chemical Co., Ltd.)

in an eggplant flask, 12 μL of triethylamine (manufactured by Nacalai Tesque Inc.) was added, and a reaction was performed at 80°C for 17 hours while stirring the mixture. 300 mL of ethyl acetate (manufactured by Kanto Chemical Co.) and 300 mL of hexane (manufactured by Kanto Chemical Co.) were added, and the mixture was allowed to stand for 2 hours to precipitate a product. Further, centrifugation (1,000 g × 10 minutes) was performed to remove the supernatant. In order to remove unreacted polyethylene glycol bismaleimide, ethyl acetate was added again, and centrifugation and supernatant removal were repeated three times. Further, drying was performed under reduced pressure to obtain a product. The prepared synthetic polymer has a structure in which a plurality of blocks in which an N-substituted maleimide having a polyethylene glycol chain (corresponding to a second segment) is bonded to a molecular chain terminal of artificial fibroin (corresponding to a first segment) are repeated. That is, the synthetic polymer is a molecule in which three segments are bonded in the order of the second segment, the first segment, and the second segment.

A film containing the synthetic polymer prepared as described above was molded. Specifically, 4.55 g of dimethyl sulfoxide was weighed in a container, 0.45 g of the synthetic polymer was added thereto, and the mixture was stirred under nitrogen under a condition of 90°C for 30 minutes, thereby preparing a dope solution in which the synthetic polymer was dissolved. The stirring rate was set to 100 rpm. In the dope solution, a mixing ratio was adjusted so that synthetic polymer:dimethyl sulfoxide was 9 mass%:91 mass%.

A film-shaped molded body (film) was prepared in the same manner as that of Example 4 using the dope solution. It was confirmed that the prepared film was clear and colorless, flexibility thereof was superior to that of the film of Example 4. It was confirmed that the film was not cracked even when a bending stress was applied.

(Example 6)

1,424 mg of the artificial fibroin (number average molecular weight: 50,000, first segment) having the amino acid sequence (PRT17) set forth in SEQ ID NO: 62 prepared as described above and 1,076 mg of methoxy polyethylene glycol maleimide (manufactured by Biochempeg Scientific Inc., MF001022-20K, second segment) having a number average molecular weight of 20,000 were dissolved in 71 mL of a solvent (DMSO, manufactured by Kishida Chemical Co., Ltd.) in an eggplant flask, 7.5 μL of triethylamine (manufactured by Nacalai Tesque Inc.) was added, and a reaction was performed at 80°C for 17 hours while stirring the mixture. 210 mL of ethyl acetate (manufactured by Kanto Chemical Co.) and 210 mL of hexane (manufactured by Kanto Chemical Co.) were added, and the mixture was allowed to stand for 17 hours to precipitate a product. Further, centrifugation (1,000 g × 10 minutes) was performed to remove the supernatant. In order to remove unreacted methoxy polyethylene glycol maleimide, ethyl acetate was added again, and centrifugation and supernatant removal were repeated three times. Further, drying was performed under reduced pressure to obtain a synthetic polymer. The prepared synthetic polymer has a structure in which an N-substituted maleimide having a polyethylene glycol chain (corresponding to a second segment) is bonded to both ends of the molecular chain of artificial fibroin (corresponding to a first segment). That is, the synthetic polymer is a molecule in which three segments are bonded in the order of the second segment, the first segment, and the second segment.

A film containing the synthetic polymer prepared as described above was molded. Specifically, 4.30 g of dimethyl sulfoxide was weighed in a container, 0.70 g of the synthetic polymer was added thereto, and the mixture was stirred under nitrogen under a condition of 90°C for 30 minutes, thereby preparing a dope solution in which the synthetic polymer was dissolved. The stirring rate was set to 100 rpm. In the dope solution, a mixing ratio was adjusted so that synthetic polymer:dimethyl sulfoxide was 14 mass%:86 mass%.

A film-shaped molded body (film) was prepared in the same manner as that of Example 4 using the dope solution. It was confirmed that the prepared film was clear and colorless, flexibility thereof was superior to that of the film of Example 4. It was confirmed that the film was not cracked even when a bending stress was applied.

(Example 7)

Polyethylene glycol (manufactured by Biochempeg scientific Inc., number average molecular weight: 20,000) having maleimide groups at both ends was reacted with artificial fibroin having two cysteine residues to prepare a synthetic polymer. Specifically, 4.022 g of artificial fibroin (number average molecular weight: 50,000) having the amino acid sequence (PRT17) set forth in SEQ ID NO: 62 prepared as described above, 1.564 g of polyethylene glycol having maleimide groups at both ends, and 201 mL of dimethyl sulfoxide as a solvent were weighed in an eggplant flask, and the mixture was stirred at 80°C for 48 hours to be dissolved and react, thereby obtaining a solution of a synthetic polymer. To the obtained solution, 603 mL of ethyl acetate and 603 mL of hexane were added, the mixture was stirred for 2 hours, centrifugation was performed, and then washing was further performed three times with ethyl acetate. Thereafter, drying was performed with an evaporator, and finally, vacuum drying was performed at room temperature to obtain a powder of a synthetic polymer.

(Example 8)

Polyethylene glycol (manufactured by Biochempeg scientific Inc., number average molecular weight: 20,000) having maleimide groups at both ends was reacted with artificial fibroin having two cysteine residues to prepare a synthetic polymer. Specifically, 348 mg of artificial fibroin (number average molecular weight: 50,000) having the amino acid sequence (PRT17) set forth in SEQ ID NO: 62 prepared as described above was weighed in a stainless steel container, 2.11 mL of dimethyl sulfoxide was added as a solvent, and the mixture was stirred and dissolved, thereby obtaining a fibroin solution. In addition, 132 mg of polyethylene glycol having maleimide groups at both ends was weighed in a glass container, 1.09 mL of dimethyl sulfoxide was added as a solvent, and the mixture was stirred and dissolved, thereby obtaining a polyethylene glycol solution. The obtained fibroin solution and polyethylene glycol solution were mixed while stirring the mixture, the mixture was reacted at 100°C for 5 minutes while stirring the mixture, and the mixture was further allowed to stand and react at 100°C for 10 minutes, thereby preparing a synthetic polymer. In Example 8, the synthetic polymer was subjected to film molding described below without being dried in a state in which the synthetic polymer was dissolved in dimethyl sulfoxide.

(Example 9)

Polyethylene glycol (manufactured by Biochempeg scientific Inc., number average molecular weight: 20,000) having maleimide groups at both ends was reacted with artificial fibroin having two cysteine residues and artificial fibroin having four cysteine residues to prepare a synthetic polymer. Specifically, 336 mg of artificial fibroin (number average molecular weight: 50,000, the total number of cysteine residues: 2) having the amino acid sequence (PRT17) set forth in SEQ ID NO: 62 prepared as described above, 44 mg of artificial fibroin (number average molecular weight: 53,100, the total number of cysteine residues: 4) having the amino acid sequence (PRT18) set forth in SEQ ID NO: 63 prepared as described above, and 160 mg of polyethylene glycol having maleimide groups at both ends were weighed in a glass container, 4.42 mL of dimethyl sulfoxide was added as a solvent, and the components described above were dissolved under stirring, thereby obtaining a reaction solution. The obtained reaction solution was allowed to react under stirring at 100°C for 10 minutes, and then was allowed to stand and react at 100°C for 5 minutes, thereby preparing a synthetic polymer. In Example 9, the synthetic polymer was subjected to film molding described below without being dried in a state in which the synthetic polymer was dissolved in dimethyl sulfoxide.

(Example 10)

Polyethylene glycol (manufactured by Biochempeg scientific Inc., number average molecular weight: 20,000) having maleimide groups at both ends was reacted with 2,2'-(ethylenedioxy)diethanethiol having two thiol groups and artificial fibroin having two cysteine residues to prepare a synthetic polymer. Specifically, 300 mg of artificial fibroin (number average molecular weight: 50,000) having the amino acid sequence (PRT17) set forth in SEQ ID NO: 62 prepared as described above and 284 mg of polyethylene glycol having maleimide groups at both ends were weighed in a glass container, 3.42 mL of dimethyl sulfoxide was added as a solvent, and the mixture was stirred to dissolve the respective components, thereby obtaining a reaction solution. The obtained reaction solution was stirred and allowed to react at 100°C for 15 minutes, 1.55 mg of 2,2'-(ethylenedioxy)diethanethiol was further added, and the mixture was stirred at 100°C for 10 minutes to react, thereby preparing a synthetic polymer. In Example 10, the synthetic polymer was subjected to film molding described below without being dried in a state in which the synthetic polymer was dissolved in dimethyl sulfoxide.

(Example 11)

Polyethylene glycol (manufactured by Biochempeg scientific Inc., number average molecular weight: 1,000) having maleimide groups at both ends was reacted with polyethylene glycol (manufactured by Biochempeg scientific Inc., number average molecular weight: 2,000) (that is, having thiol groups at both ends) having cysteine residues at both ends and artificial fibroin having two cysteine residues, thereby preparing a synthetic polymer. Specifically, 378 mg of artificial fibroin (number average molecular weight: 50,000) having the amino acid sequence (PRT17) set forth in SEQ ID NO: 62 prepared as described above, 79 mg of polyethylene glycol having maleimide groups at both terminals, and 143 mg of polyethylene glycol having cysteine residues at both ends were weighed in a vial bottle, 4.40 mL of dimethyl sulfoxide was added as a solvent, and the mixture was stirred and dissolved, thereby obtaining a reaction solution. The reaction solution was allowed to react under stirring at 100°C for 10 minutes, and was further allowed to stand and react at 100°C for 10 minutes, thereby preparing a synthetic polymer. In Example 11, the synthetic polymer was subjected to film molding described below without being dried in a state in which the synthetic polymer was dissolved in dimethyl sulfoxide.

(Comparative Example 5)

For comparison with the synthetic polymers prepared in Examples 7 to 11, a similar operation was performed using artificial fibroin (molecular weight: 100,000) having the amino acid sequence (PRT27) set forth in SEQ ID NO: 72 prepared as described above. Specifically, 650 mg of artificial fibroin (molecular weight: 100,000) having the amino acid sequence (PRT27) set forth in SEQ ID NO: 72 prepared as described above was weighed in a stainless steel container, 3.955 mL of dimethyl sulfoxide was added as a solvent, and the mixture was stirred, thereby obtaining a dispersion. The obtained dispersion was stirred and dissolved at 120°C for 30 minutes, and then allowed to stand at 100°C for 10 minutes. In Comparative Example 5, the artificial fibroin dissolved in dimethyl sulfoxide was subjected to film molding as described below without drying.

<Evaluation of molded body>

Film-shaped molded bodies were prepared under the following conditions using the synthetic polymers prepared in Examples 7 to 11, respectively. As for the synthetic polymer prepared in Example 7, first, 530 mg of a powder of the synthetic polymer was added to 3.53 mL of dimethyl sulfoxide, and the mixture was stirred at 90°C for 40 minutes to dissolve the powder, thereby preparing a dope solution. Next, the dope solution was allowed to stand at 80°C for 30 minutes, and the defoamed solution was subjected to film molding. The synthetic polymers prepared in Examples 8 to 11 were subjected to film molding while being dissolved in dimethyl sulfoxide as dope solutions without drying. In addition, the artificial fibroin of Comparative Example 5 was similarly subjected to film molding as a dope solution while being dissolved in dimethyl sulfoxide without drying.

First, a PET film (150 mm × 200 mm) was prepared as a release member, and a liquid film of the dope solution was provided on the PET film so as to have a thickness of 0.4 mm with an applicator. Thereafter, the liquid film was allowed to stand together with the PET film in an air blowing oven, drying was performed under a condition of 60°C for 30 minutes, transfer to a vacuum oven was further performed, vacuum-drying was performed under a condition of 80°C for 15 hours, and absolute drying was performed, thereby preparing a film-shaped molded body (film) formed of a synthetic polymer or artificial fibroin.

Each of the prepared films was subjected to a tensile test to evaluate the elastic modulus, tensile strength, elongation at break, and toughness. The results are shown in Table 7. Table 7 also shows the thickness of the film in the absolute dry state. Note that the elongation at break shown in Table 7 was expressed as a relative value based on the results obtained in Comparative

Example 5.

[Table 7]

| | Film thickness [pm] | Elastic modulus | Elongation at break | Toughness |
|---|---|---|---|---|
| Example 7 | 26 | 73 | 1292 | 639 |
| Example 8 | 27 | 76 | 5400 | 2927 |
| Example 9 | 22 | 63 | 8520 | 3120 |
| Example 10 | 28 | 24 | 22332 | 7190 |
| Example 11 | 59 | 38 | 436 | 217 |
| Comparative Example 5 | 49 | 100 | 100 | 100 |

As shown in Table 7, it can be seen that in the films obtained in Examples 7 to 11, the elongation at break was extremely large and the elastic modulus was suppressed to be low in comparison to the film obtained in Comparative Example 5. It can be confirmed from this that the films obtained in Examples 7 to 11 have excellent flexibility. In addition, the films obtained in Examples 7 to 11 have sufficiently excellent toughness.

From the results of Examples 7 and 8, it was confirmed that a film having an excellent elastic modulus, elongation at break, and toughness was prepared by directly molding a film from a solution after reaction rather than preparing a film after once isolating a synthetic polymer as a powder and dissolving the synthetic polymer again, and from the results of Examples 8, 10, and 11, it was confirmed that a film having flexibility to be obtained was prepared by preparing a second segment having flexibility, and the tendency of the flexibility becomes remarkable by lengthening the second

segment.

(Example 12)

2,177 mg of the artificial fibroin (number average molecular weight: 50,000, first segment) having the amino acid sequence (PRT17) set forth in SEQ ID NO: 62 prepared as described above and 823 mg of polyethylene glycol bismaleimide (Biochempeg Scientific Inc., HO022022-20K: second segment) having a number average molecular weight of 20,000 were dissolved in 12,000 mg of a dimethyl sulfoxide solution (DMSO, manufactured by Kishida Chemical Co., Ltd.) having a lithium chloride concentration of 2 M, and the mixture was stirred under nitrogen at 100°C for 4 hours to react, thereby preparing a dope solution in which the synthetic polymer was dissolved. The stirring rate was set to 100 rpm. In the dope solution, a mixing ratio was adjusted so that synthetic polymer:dimethyl sulfoxide was 20 mass%:80 mass%. A viscosity of the obtained dope at 100°C was 10,900 mPa•s. The synthetic polymer in the prepared dope solution has a structure in which a plurality of blocks in which an N-substituted maleimide having a polyethylene glycol chain (corresponding to a second segment) is bonded to a molecular chain terminal of artificial fibroin (corresponding to a first segment) are repeated.

Fibers were prepared by dry-wet spinning under the following conditions using the dope solution. The dope solution at 100°C was extruded together with nitrogen gas from a nozzle (nozzle diameter: 0.1 mm) by a gear pump, passed through a first coagulation bath (first bath), a second coagulation bath (second bath), and a stretching bath (third bath), and then wound up by a heating type roller set at 60°C. A stretch ratio in the stretching bath was set to 8.5 times. The first bath was a methanol bath at 0°C, the second bath was a methanol bath at 25°C, and the third bath was a water bath at 54°C. It was confirmed that the obtained fibers had sufficient stretchability. The appearance of the obtained fibers is illustrated in Fig. 13.

Examples described above have been shown as examples for the case where the second segment has a maleimide group. Hereinafter, some examples are also shown for confirmation in the case of using another functional group instead of the maleimide group and in the case of changing the bonding type between the first segment and the second segment.

(Example 13)

A synthetic polymer was prepared using poly(ethylene glycol) glycidyl ether (manufactured by Merck & Co., Inc.: second segment) having a number average molecular weight of 6,000. Specifically, in a glass container, 27 mg of the artificial fibroin (first segment) having a number average molecular weight of 50,000 and having the amino acid sequence (PRT17) set forth in SEQ ID NO: 62 prepared as described above and 3.1 mg of the poly(ethylene glycol) glycidyl ether were weighed. Further, 0.14 μL of triethylamine (manufactured by Nacalai Tesque Inc.) and 1.73 mL of an N,N-dimethylformamide solution of lithium chloride (lithium chloride concentration: 2 mass%) as a solvent were added to obtain a reaction solution. Note that as lithium chloride, a product manufactured by FUJIFILM Wako Pure Chemical Corporation was used, and as N,N-dimethylformamide, a product manufactured by Kanto Chemical Co. was used.

The reaction solution described above was allowed to react at 50°C or 80°C for 3 hours or 20 hours. After the reaction, 6 mL of RO water was added to precipitate the product, and the product was centrifuged to remove the solvent. Further, washing was performed three times with RO water and twice with acetone, and the washing liquid was removed by centrifugation each time. Note that 15 mL of a solvent (RO water or acetone) was used for one washing. By such a washing operation, unreacted poly(ethylene glycol) diglycidyl ether and the like were removed. After the washing, a synthetic polymer was obtained by drying under reduced pressure. By GPC measurement for the obtained synthetic polymer, it was confirmed that the molecular weight of the synthetic polymer as a product was higher than that of the artificial fibroin before the reaction, and it was confirmed that a covalent bond was formed between the artificial fibroin and poly(ethylene glycol) diglycidyl ether. The results of GPC are illustrated in Fig. 14.

(Example 14)

Polyethylene glycol (manufactured by Nacalai Tesque Inc.) having a number average molecular weight of 20,000 was reacted with hexamethylene diisocyanate (manufactured by Tokyo Chemical Industry Co., Ltd.) to prepare a polymer compound corresponding to the second segment. A synthetic polymer was prepared by reacting the polymer compound with artificial fibroin.

Specifically, 197 mg of the polyethylene glycol was weighed in a glass container, 0.847 μL of N,N-dimethylformamide as a solvent was added thereto, and the mixture was heated to 40°C to be dissolved. The mixture was returned to 25°C, and 3.2 μL of the hexamethylene diisocyanate was added to prepare a reaction solution. The reaction was performed by stirring the reaction solution at 40°C or 60°C for 4 hours.

In addition, 42.9 mg of the artificial fibroin (first segment) having a number average molecular weight of 10,000

and having the amino acid sequence (PRT27) set forth in SEQ ID NO: 72 was weighed in another glass container, 1.47 mL of an N,N-dimethylformamide solution of lithium chloride (lithium chloride concentration: 2 mass%) as a solvent was added thereto, and the mixture was heated at 100°C for 10 minutes to be dissolved, thereby preparing a solution. In addition, apart from this, 42.9 mg of the artificial fibroin (first segment) having a number average molecular weight of 50,000 and having the amino acid sequence (PRT17) set forth in SEQ ID NO: 62 was weighed in another glass container, 1.47 mL of an N,N-dimethylformamide solution of lithium chloride (lithium chloride concentration: 2 mass%) as a solvent was added thereto, and the mixture was heated at 60°C for 10 minutes to be dissolved, thereby preparing a solution. Each of the two types of artificial fibroin solutions was cooled to 30°C.

A solution prepared by adding 16 mg of lithium chloride to a solution containing a polymer compound corresponding to the second segment was added to each of the two types of artificial fibroin solutions in an amount of 86 mg to obtain two types of reaction solutions. Thereafter, the reaction was performed by stirring each of the reaction solutions at 30°C for 20 hours.

After the reaction, washing was performed in the same manner as that of Example 13 to remove unreacted polymer compounds and the like. After the washing, drying was performed under reduced pressure, thereby obtaining a synthetic polymer containing the artificial fibroin having the amino acid sequence (PRT27) set forth in SEQ ID NO: 72 and a synthetic polymer containing the artificial fibroin having the amino acid sequence (PRT17) set forth in SEQ ID NO: 62. By GPC measurement for the two types of the obtained synthetic polymers, it was confirmed that both the molecular weights of the two types of the synthetic polymers as products were higher than that of the artificial fibroin before the reaction, and it was confirmed that a covalent bond was formed between the artificial fibroin and the polymer compound (the reaction product of polyethylene glycol and hexamethylene diisocyanate). The results of GPC are illustrated in Fig. 15. Fig. 15(a) illustrates the results of the synthetic polymer containing the artificial fibroin having the amino acid sequence (PRT27) set forth in SEQ ID NO: 72, and Fig. 15(b) illustrates the results of the synthetic polymer containing the artificial fibroin having the amino acid sequence (PRT17) set forth in SEQ ID NO: 62.

(Example 15)

Polyethylene glycol (manufactured by Nacalai Tesque Inc.) having a number average molecular weight of 20,000 was reacted with 3-butynoic acid (manufactured by Tokyo Chemical Industry Co., Ltd.) to prepare a polymer compound corresponding to the second segment. A synthetic polymer was prepared by reacting the polymer compound with artificial fibroin.

Specifically, 1 g of the polyethylene glycol, 25 mg of 3-butynoic acid, and 10.3 mg of p-toluenesulfonic acid (manufactured by Kishida Chemical Co., Ltd.) as an acid catalyst were weighed in a glass container, and 10 mL of toluene (manufactured by Kishida Chemical Co., Ltd.) as a solvent was added, thereby obtaining a reaction solution. The reaction was performed by stirring the reaction solution at 90°C for 20 hours. At a point in time when 20 hours had elapsed, 50 mL of cyclopentyl methyl ether (manufactured by FUJIFILM Wako Pure Chemical Corporation) was added, and the mixture was allowed to stand for 2 hours for precipitation. The precipitate was collected by filtration and washed three times with cyclopentyl methyl ether to obtain a powder. 15 mL of a solvent (cyclopentyl methyl ether) was used for one washing. Unreacted products such as 3-butynoic acid were removed by a washing operation. This precipitation and purification operation were repeated twice, and drying was performed under reduced pressure, thereby obtaining a polymer compound powder corresponding to the second segment.

Next, in a glass container, 40 mg of artificial fibroin (first segment) having the amino acid sequence (PRT17) set forth in SEQ ID NO: 62 prepared as described above and having a number average molecular weight of 50,000, 16 mg of the polymer compound, 0.22 μL or 1.67 μL of triethylamine (manufactured by Nacalai Tesque Inc.) as a base catalyst, and 2.8 mL of an N,N-dimethylformamide solution of lithium chloride (lithium chloride concentration: 2 mass%) or dimethyl sulfoxide as a solvent were added to prepare a reaction solution. The reaction was performed by stirring the reaction solution at 25°C or 80°C for 20 hours.

After the reaction, washing was performed in the same manner as that of Example 13 to remove unreacted polymer compounds and the like. After the washing, a synthetic polymer was obtained by drying under reduced pressure. By GPC measurement for the obtained synthetic polymer, it was confirmed that the molecular weight of the synthetic polymer as a product was more increased than the artificial fibroin before the reaction, and it was confirmed that a covalent bond was formed between the artificial fibroin and the polymer compound (the reaction product of polyethylene glycol and 3-butynoic acid). The results of GPC are illustrated in Fig. 16.

(Example 16)

Polyethylene glycol (manufactured by Nacalai Tesque Inc.) having a number average molecular weight of 20,000 was reacted with methyl bromoacetate (manufactured by Tokyo Chemical Industry Co., Ltd.) to prepare a polymer compound corresponding to the second segment. A synthetic polymer was prepared by reacting the polymer compound

with artificial fibroin. The reaction was performed by stirring the reaction solution at 90°C for 20 hours.

Specifically, 300 mg of the polyethylene glycol, 8.3 μL of methyl bromoacetate, and 3.1 mg of p-toluenesulfonic acid (manufactured by Kishida Chemical Co., Ltd.) as an acid catalyst were weighed in a glass container, and 3 mL of toluene (manufactured by Kishida Chemical Co., Ltd.) as a solvent was added, thereby obtaining a reaction solution. The reaction was performed by stirring the reaction solution at 90°C for 20 hours. At a point in time when 20 hours had elapsed, 50 mL of cyclopentyl methyl ether (manufactured by FUJIFILM Wako Pure Chemical Corporation) was added, and the mixture was allowed to stand for 2 hours for precipitation. The precipitate was collected by filtration and washed three times with cyclopentyl methyl ether precipitation. The precipitate was collected by filtration and washed three times with cyclopentyl methyl ether to obtain a powder. 5 mL of a solvent (cyclopentyl methyl ether) was used for one washing. Unreacted products such as methyl bromoacetate were removed by a washing operation. This precipitation and purification operation were repeated twice, and drying was performed under reduced pressure, thereby obtaining a polymer compound powder corresponding to the second segment.

Next, in a glass container, 36 mg of artificial fibroin (first segment) having the amino acid sequence (PRT17) set forth in SEQ ID NO: 62 prepared as described above and having a number average molecular weight of 50,000, 13.7 mg of the polymer compound, 0.19 μL of triethylamine (manufactured by Nacalai Tesque Inc.) as a base catalyst, and 2.9 mL of an N,N-dimethylformamide solution of lithium chloride (lithium chloride concentration: 2 mass%) as a solvent were added to prepare a reaction solution. The reaction was performed by stirring the reaction solution at 50°C for 20 hours.

After the reaction, washing was performed in the same manner as that of Example 13 to remove unreacted polymer compounds and the like. After the washing, a synthetic polymer was obtained by drying under reduced pressure. By GPC measurement for the obtained synthetic polymer, it was confirmed that the molecular weight of the synthetic polymer as a product was more increased than the artificial fibroin before the reaction, and it was confirmed that a covalent bond was formed between the artificial fibroin and the polymer compound (the reaction product of polyethylene glycol and methyl bromoacetate). The results of GPC are illustrated in Fig. 17.

(Example 17)

Polyethylene glycol (manufactured by FUJIFILM Wako Pure Chemical Corporation) having a number average molecular weight of 400, p-toluenesulfonic acid monohydrate (manufactured by Kishida Chemical Co., Ltd.), and sodium azide (manufactured by Nacalai Tesque Inc.) were reacted to prepare a polymer compound corresponding to the second segment. In addition, artificial fibroin was reacted with propargyl bromide (manufactured by Tokyo Chemical Industry Co., Ltd.) to prepare artificial fibroin (first segment) having an alkynyl group corresponding to the first segment, and the artificial fibroin was reacted with the polymer compound, thereby preparing a synthetic polymer.

Specifically, 200 mg of the polyethylene glycol, 210 mg of p-toluenesulfonyl chloride, and 348 μL of triethylamine (manufactured by Nacalai Tesque Inc.) as a base catalyst were weighed in a glass container to prepare a reaction solution. The reaction was performed by stirring the reaction solution at 25°C for 5 hours. After 5 hours had elapsed, 9 mL of 1 N hydrochloric acid (manufactured by Kanto Chemical Co.) and 9 mL of ethyl acetate (manufactured by Kanto Chemical Co.) were added, and the mixture was transferred to a separatory funnel, and extraction was performed with ethyl acetate three times. Furthermore, the ethyl acetate layer was washed with a sodium hydrogen carbonate solution (manufactured by Kanto Chemical Co.) and saturated saline (manufactured by Kanto Chemical Co.), and dehydration was performed with sodium sulfate (manufactured by Nacalai Tesque Inc.). Thereafter, drying was performed under reduced pressure to obtain a reaction product.

Next, 20 mg of the reaction product, 5.5 mg of sodium azide, 0.5 mg of tetrabutylammonium iodide (manufactured by Tokyo Chemical Industry Co., Ltd.) as a catalyst, and 1 mL of dimethyl sulfoxide (manufactured by Kishida Chemical Co., Ltd.) as a solvent were added to a glass container to prepare a reaction solution. The reaction was performed by stirring the reaction solution at 80°C for 20 hours. At a point in time when 20 hours had elapsed, 3 mL of a saturated ammonium chloride solution (manufactured by Kanto Chemical Co.) was added, and the mixture was transferred to a separatory funnel, and extraction was performed with ethyl acetate three times. Further, the mixture was dehydrated with sodium sulfate (manufactured by Nacalai Tesque Inc.). Thereafter, drying was performed under reduced pressure to obtain a polymer compound corresponding to the second segment.

Next, 30 mg of artificial fibroin (first segment) having the amino acid sequence (PRT17) set forth in SEQ ID NO: 62 prepared described above and having a number average molecular weight of 50,000, 0.9 μL of propargyl bromide, and 1 mL of dimethyl sulfoxide (manufactured by Kishida Chemical Co., Ltd.) as a solvent were added to a glass container, thereby preparing a reaction solution. The reaction was performed by stirring the reaction solution at 25°C for 20 hours. At a point in time when 20 hours had elapsed, 3 mL of ethyl acetate (manufactured by Kanto Chemical Co.) was added to precipitate a product. The precipitate was collected by filtration and washed three times with ethyl acetate. 5 mL of a solvent (ethyl acetate) was used for one washing. Unreacted products such as propargyl bromide were removed by a washing operation. After the washing operation, drying was performed under reduced pressure to obtain artificial fibroin

(first segment) having an alkynyl group corresponding to the first segment.

In a glass container, 0.4 mg of the polymer compound, 30 mg of artificial fibroin (first segment) having an alkynyl group corresponding to the first segment, 0.17 mg of copper(I) iodide (manufactured by Nacalai Tesque Inc.) as a catalyst, and 1 mL of dimethyl sulfoxide (manufactured by Kishida Chemical Co., Ltd.) as a solvent were weighed to prepare a reaction solution. The reaction was performed by stirring the reaction solution at 80°C for 20 hours. At a point in time when 20 hours had elapsed, 3 mL of ethyl acetate was added to precipitate a product. The precipitate was collected by filtration and washed three times with ethyl acetate. 5 mL of a solvent (ethyl acetate) was used for one washing. An unreacted polymer compound and the like were removed by a washing operation. After the washing, a synthetic polymer was obtained by drying under reduced pressure. By GPC measurement for the obtained synthetic polymer, it was confirmed that the molecular weight of the synthetic polymer as a product was more increased than the artificial fibroin before the reaction, and it was confirmed that a covalent bond was formed between the artificial fibroin having an alkynyl group and the polymer compound (the reaction product of polyethylene glycol and sodium azide). The results of GPC are illustrated in Fig. 18.

(Reference Example 1: Evaluation of hydrophobicity (solubility) of structural protein)

The solubility in a 9 M lithium bromide aqueous solution of each of the artificial fibroin and silk fibroin (value of average hydropathy index: 0.21) was evaluated. As the silk fibroin, "Silk Powder IM" manufactured by KB SEIREN, LTD. was used. As the artificial fibroin, artificial fibroin (number average molecular weight: 50,000) having the amino acid sequence (PRT17) set forth in SEQ ID NO: 62 prepared as described above and artificial fibroin (number average molecular weight: 100,000) having the amino acid sequence (PRT27) set forth in SEQ ID NO: 72 prepared as described above were used. Specifically, the solubility was evaluated by adding predetermined amounts of silk fibroin and artificial fibroin to a 9 M lithium bromide aqueous solution, respectively, and stirring the mixture at 60°C for 30 minutes, and then the appearance of the aqueous solution was observed. The results are shown in Table 8. In Table 8, "A" indicates that the added fibroin was completely dissolved and a solution having a set concentration could be prepared, and "B" indicates that a residue of fibroin was observed. That is, it was confirmed that artificial fibroin was highly hydrophobic fibroin that was not dissolved in an amount equivalent to 5 mass% even in a 9 M lithium bromide aqueous solution at 60°C.

[Table 8]

| | | Set value of fibroin concentration [mass%] | | | | | | | | |
|---|---|---|---|---|---|---|---|---|---|---|
| | | 1 | 5 | 20 | 30 | 31 | 32 | 33 | 34 | 35 |
| Silk fibroin | | - | A | A | A | B | B | B | B | B |
| Artificial fibroin | PRT17 | B | B | - | B | - | - | - | - | - |
| | PRT27 | - | B | - | B | - | - | - | - | - |

(Reference Example 2: Evaluation of hydrophobicity (hot water resistance) of structural protein)

The artificial fibroin and silk fibroin were subjected to a hot water resistance test to evaluate the hot water resistance. As the silk fibroin, "Silk Powder IM" manufactured by KB SEIREN, LTD. was used. As the artificial fibroin, artificial fibroin (number average molecular weight: 50,000) having the amino acid sequence (PRT17) set forth in SEQ ID NO: 62 prepared as described above was used. A dispersion was prepared by adding 50 mg of fibroin and 950 mg of RO water as a solvent to a glass container so that the content of fibroin was 5 mass%. The dispersion was stirred at 100°C for 5 hours. After 5 hours had elapsed, the solvent was removed by centrifugation. The solvent was removed and then washed twice with acetone, and the acetone was removed by centrifugation. After the washing operation, drying was performed under reduced pressure to obtain a treated powder. The degree of decomposition was confirmed by GPC measurement for the treated powder. The results of GPC are illustrated in Fig. 19. As is apparent from Fig. 19, a decrease in molecular weight of silk fibroin was confirmed as compared with that before treatment, and such a decrease in molecular weight was not observed in the artificial fibroin. From this result, it is presumed that the artificial fibroin had lower hydrophilicity to RO water than the silk fibroin. In other words, it can be said that the artificial fibroin has high hydrophobicity. Note that, in Fig. 19, the results of the artificial fibroin (number average molecular weight: 50,000) having the amino acid sequence (PRT17) set forth in SEQ ID NO: 62 are referred to as PRT100.

(Reference Example 3: Evaluation of hydrophobicity (contact angle) of structural protein)

For artificial fibroin and silk fibroin, the contact angle with respect to water was measured and evaluated. As

the silk fibroin, "Silk Powder IM" manufactured by KB SEIREN, LTD. was used. As the artificial fibroin, artificial fibroin (number average molecular weight: 50,000) having the amino acid sequence (PRT17) set forth in SEQ ID NO: 62 prepared as described above and artificial fibroin (number average molecular weight: 100,000) having the amino acid sequence (PRT27) set forth in SEQ ID NO: 72 prepared as described above were used. Specifically, for the measurement of the contact angle, a dispersion was prepared by first adding 30 mg of fibroin and 970 mg of dimethyl sulfoxide as a solvent to a glass container so that a content of the fibroin was 3 mass%. The dispersion was stirred at 100°C for 15 minutes to prepare a dimethyl sulfoxide solution. Next, a masking tape having a thickness of 100 um was attached onto the slide glass to form a shim, and the dimethyl sulfoxide solution prepared as described above was cast to form a liquid film. Thereafter, each slide glass was transferred into a vacuum oven (manufactured by TOKYO RIKAKIKAI CO, LTD., product name: VOS-210C), allowed to stand, and dried under reduced pressure at 80°C to form a fibroin film. 2 μL of RO water was added dropwise to the film, and the contact angle of water after 5 seconds was measured using a contact angle meter (manufactured by Kyowa Interface Science Co., Ltd., product name: DMs-061). The measurement was performed on 6 samples for each fibroin, and the average value thereof was used for evaluation. The results are shown in Table 9 and are illustrated in Fig. 20. As shown in Table 9, it was confirmed that the artificial fibroin had a larger water contact angle and higher hydrophobicity than the silk fibroin. In Fig. 20, the results for silk fibroin are shown on the left, the results for artificial fibroin having the amino acid sequence (PRT17) set forth in SEQ ID NO: 62 are shown on the right, and the results for artificial fibroin having the amino acid sequence (PRT27) set forth in SEQ ID NO: 72 are shown in the center.

[Table 9]

| | | Water contact angle [°] (average value) |
|---|---|---|
| Silk fibroin | | 54.9 |
| Artificial fibroin | PRT17 | 70.0 |
| | PRT27 | 72.4 |

Industrial Applicability

According to the present disclosure, it is possible to provide a synthetic polymer capable of producing a molded body having excellent flexibility as a polymer material having a peptide skeleton. According to the present disclosure, it is possible to provide a molding material and a molded body each containing the synthetic polymer.

According to the present disclosure, the physical properties and functions of the synthetic polymer can be adjusted by selecting the second segment to be introduced into the first segment. As a result, the physical properties and functions of the molded body prepared using the synthetic polymer can be adjusted.

Reference Signs List

2 Mold
4 Upper pin
6 Lower pin
8a Sample
8b Molded body
10 Pressure molding machine

## Claims

**1.** A synthetic polymer comprising:

a first segment containing a polypeptide skeleton; and
one or a plurality of second segments bonded directly to the first segment,
wherein the second segment contains a molecular group having a plasticizing function for the polypeptide skeleton.

2. The synthetic polymer according to claim 1, wherein the polypeptide skeleton is a hydrophobic polypeptide skeleton.

3. The synthetic polymer according to claim 2, wherein an average hydropathy index of the hydrophobic polypeptide skeleton is 0.22 or more.

4. The synthetic polymer according to any one of claims 1 to 3, wherein a molecular weight of the second segment is a value within a range of 1 to 70 when a molecular weight of the first segment is 100.

5. The synthetic polymer according to any one of claims 1 to 4, wherein the second segment contains a skeleton derived from at least one selected from the group consisting of polyether, polyester, polycarbonate, polyamide, polyol, polyolefin, polyacetal, polyketal, poly(meth)acrylate, silicone, polyurethane, polyalkyleneimine, a phenolic resin, a urea resin, a melamine resin, and polysaccharides.

6. The synthetic polymer according to any one of claims 1 to 5, wherein the second segment includes at least one selected from the group consisting of a polyether group, a polyester group, a polycarbonate group, a polyamide group, a polyol group, and a modified polysaccharide group.

7. The synthetic polymer according to any one of claims 1 to 6, wherein the synthetic polymer contains a plurality of first segments, and
some of the second segments are bonded to two or more first segments to form a network structure.

8. The synthetic polymer according to any one of claims 1 to 6, wherein the synthetic polymer contains a plurality of first segments, and
the first segment and the second segment are alternatively bonded.

9. The synthetic polymer according to any one of claims 1 to 8, wherein the second segment contains a plurality of molecular groups, and the plurality of molecular groups are linked to each other.

10. The synthetic polymer according to any one of claims 1 to 9, wherein the second segment further contains a linker, and the molecular group and the polypeptide are bonded to each other via the linker.

11. The synthetic polymer according to claim 10, wherein the linker includes at least one selected from the group consisting of a structural unit represented by the following Formula (1), structural units represented by the following General Formulas (2a) to (6), a structural unit represented by the following Formula (7), structural units represented by the following General Formulas (8a) to (9), structural units represented by the following General Formulas (10) to (11b), and structural units represented by the following General Formulas (13) to (16):

[Chem. 1]

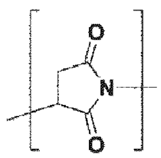

(1)

[Chem. 2]

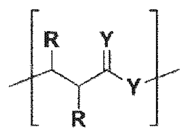

(2a)

[Chem. 3]

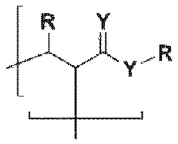

(2b)

[Chem. 4]

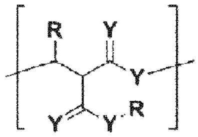

(3a)

[Chem. 5]

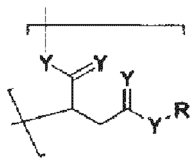

(4a)

[Chem. 6]

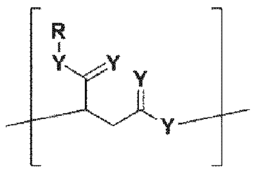

(4b)

[Chem. 7]

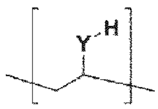

(5)

[Chem. 8]

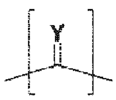

(6)

[Chem. 9]

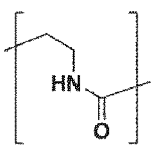

(7)

[Chem. 10]

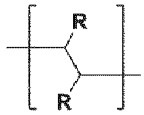

(8a)

[Chem. 11]

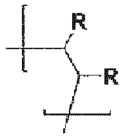

(8b)

[Chem. 12]

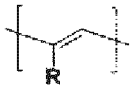

(9)

[Chem. 13]

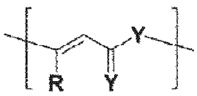

(10)

[Chem. 14]

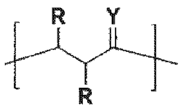

(11a)

[Chem. 15]

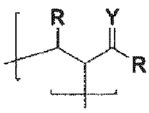

(11b)

[Chem. 16]

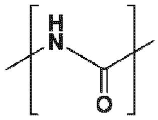

(13)

[Chem. 17]

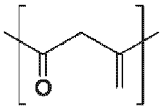

(14)

[Chem. 18]

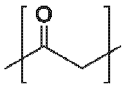

(15)

[Chem. 19]

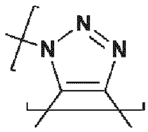

(16)

,

in General Formulas (2a), (2b), (3a), (4a), (4b), (5), (6), (10), (11a), and (11b), Y's each independently represent an oxygen atom, a sulfur atom, or $NR^1$, and $R^1$ represents hydrogen, a hydrocarbon group, an aromatic group, a carbonyl group, or a sulfonyl group, and
in General Formulas (2a), (2b), (3a), (4a), (4b), (8a), (8b), (9), (10), (11a), and (11b), R's each independently represent hydrogen, a hydrocarbon group, or an aromatic group.

**12.** The synthetic polymer according to claim 11, wherein the linker includes at least one selected from the group consisting of a structural unit represented by Formula (1) and structural units represented by General Formulas (2a), (3a), (4a), (4b), and (10).

**13.** The synthetic polymer according to any one of claims 1 to 12, wherein the polypeptide skeleton includes a skeleton derived from an artificial protein.

**14.** The synthetic polymer according to claim 13, wherein the artificial protein includes an artificial structural protein.

**15.** The synthetic polymer according to claim 14, wherein the artificial structural protein includes artificial fibroin.

**16.** The synthetic polymer according to claim 15, wherein the artificial fibroin includes artificial modified spider silk fibroin.

**17.** A molding material comprising the synthetic polymer according to any one of claims 1 to 16.

**18.** A molded body comprising the synthetic polymer according to any one of claims 1 to 16.

**19.** A production method of a synthetic polymer, the method comprising:

a step of obtaining a synthetic polymer by reacting a compound containing a polypeptide skeleton with at least one of compounds represented by the following General Formulas (1A) to (16A),
wherein the polypeptide skeleton includes at least one selected from the group consisting of a thiol group, an amino group, a hydroxy group, a guanidino group, a carboxy group, a phenoxy group, an indole group, an amide group, an azide group, and an alkynyl group:

[Chem. 20]

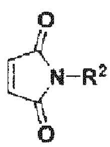

(1A)

[Chem. 21]

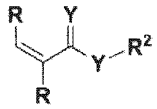

(2A)

[Chem. 22]

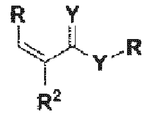

(2B)

[Chem. 23]

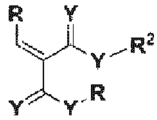

(3A)

[Chem. 24]

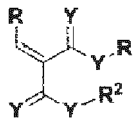

(3B)

[Chem. 25]

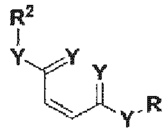

(4A)

[Chem. 26]

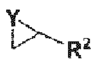

(5A)

[Chem. 27]

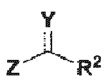

(6A)

[Chem. 28]

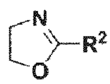

(7A)

[Chem. 29]

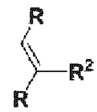

(8A)

[Chem. 30]

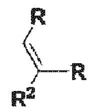

(8B)

[Chem. 31]

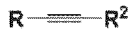

(9A)

[Chem. 32]

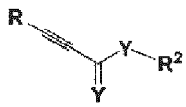

(10A)

[Chem. 33]

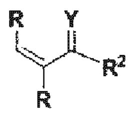

(11A)

[Chem. 34]

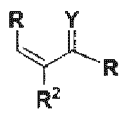

(11B)

[Chem. 35] **$Z\text{-}R^2$ (12A)**

[Chem. 36] **$OCN\text{-}R^2$ (13A)**

[Chem. 37]

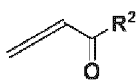

(14A)

[Chem. 38]

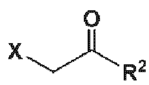

(15A)

[Chem. 39] **$N_3$-$R^2$ (16A)**,

in General Formulas (1A), (2A), (2B), (3A), (3B), (4A), (5A), (6A), (7A), (8A), (8B), (9A), (10A), (11A), (11B), and (12A), $R^2$ represents a molecular group having a plasticizing function for the polypeptide skeleton,
in General Formulas (2A), (2B), (3A), (3B), (4A), (5A), (6A), (8B), (9A), (10A), (11A), and (11B), Y's each independently represent an oxygen atom, a sulfur atom, or $NR^1$, and $R^1$ represents hydrogen, a hydrocarbon group, an aromatic group, a carbonyl group, or a sulfonyl group,
in General Formulas (2A), (2B), (3A), (3B), (4A), (5A), (8A), (8B), (9A), (10A), (11A), and (11B), R's each independently represent hydrogen, a hydrocarbon group, or an aromatic group,
in General Formulas (6A) and (12A), Z represents a halogen atom, a sulfonic acid ester group, or a fluorine-containing carboxylic acid ester group, and
in General Formula (15A), X represents a halogen atom.

**20.** The production method according to claim 19, wherein the reaction between the compound containing the polypeptide skeleton and the compound represented by any one of General Formulas (1A) to (12A) and (14A) is a Michael addition reaction.

**21.** The production method according to claim 20, wherein the polypeptide skeleton is a hydrophobic polypeptide skeleton.

**22.** The production method according to claim 21, wherein an average hydropathy index of the hydrophobic polypeptide skeleton is 0.22 or more.

**23.** The production method according to any one of claims 19 to 22, wherein a molecular weight of the molecular group having the plasticizing function for the polypeptide skeleton is a value within a range of 1 to 70 when a molecular weight of the polypeptide skeleton is 100.

**24.** The production method according to any one of claims 19 to 23, wherein the compound containing the polypeptide skeleton contains an artificial protein.

**25.** The production method according to claim 24, wherein the artificial protein includes an artificial structural protein.

**26.** A production method of a synthetic polymer, the method comprising:

a step of obtaining a synthetic polymer by reacting a compound containing a polypeptide skeleton with a compound containing a molecular group having a plasticizing function for the polypeptide skeleton, and at least a compound having two or more structural units selected from the group consisting of structural units represented by the following General Formulas (2-1A) to (2-16A),
wherein the polypeptide skeleton includes at least one selected from the group consisting of a thiol group, an amino group, a hydroxy group, a guanidino group, a carboxy group, a phenoxy group, an indole group, an amide group, an azide group, and an alkynyl group, and
the compound containing the molecular group includes at least one selected from the group consisting of a thiol group, an amino group, a hydroxy group, a guanidino group, a carboxy group, a phenoxy group, an indole group, an amide group, an azide group, and an alkynyl group:

[Chem. 40]

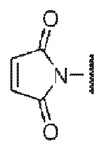

(2-1A)

[Chem. 41]

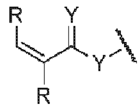

(2-2A)

[Chem. 42]

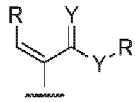

(2-2B)

[Chem. 43]

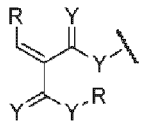

(2-3A)

[Chem. 44]

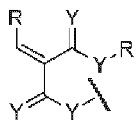

(2-3B)

[Chem. 45]

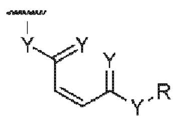

(2-4B)

[Chem. 46]

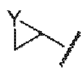

(2-5A)

[Chem. 47]

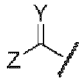

(2-6A)

[Chem. 48]

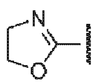

(2-7A)

[Chem. 49]

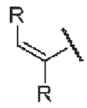

(2-8A)

[Chem. 50]

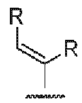

(2-8B)

[Chem. 51]

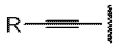

(2-9A)

[Chem. 52]

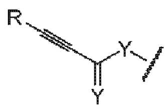

(2-10A)

[Chem. 53]

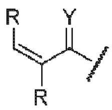

(2-11A)

[Chem. 54]

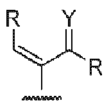

(2-11B)

[Chem. 55]

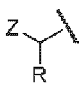

(2-12A)

[Chem. 56]

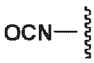

(2-13A)

[Chem. 57]

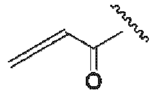

(2-14A)

[Chem. 58]

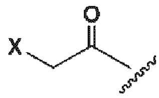

(2-15A)

[Chem. 59]

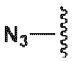

(2-16A)

,

in General Formulas (2-2A), (2-2B), (2-3A), (2-3B), (2-4A), (2-5A), (2-6A), (2-10A), (2-11A), and (2-11B), Y's each independently represent an oxygen atom, a sulfur atom, or $NR^1$, and $R^1$ represents hydrogen, a hydrocarbon group, an aromatic group, a carbonyl group, or a sulfonyl group,
in General Formulas (2-2A), (2-2B), (2-3A), (2-3B), (2-4A), (2-8A), (2-8B), (2-9A), (2-10A), (2-11A), (2-11B), and (2-12A), R's each independently represent hydrogen, a hydrocarbon group, or an aromatic group,
in General Formulas (2-6A) and (2-12A), Z represents a halogen atom, a sulfonic acid ester group, or a fluorinecontaining carboxylic acid ester group, and
in General Formula (2-15A), X represents a halogen atom.

**27.** The production method according to claim 26, wherein the polypeptide skeleton is a hydrophobic polypeptide skeleton.

**28.** The production method according to claim 27, wherein an average hydropathy index of the hydrophobic polypeptide skeleton is 0.22 or more.

**29.** The production method according to any one of claims 26 to 28, wherein a molecular weight of the molecular group having the plasticizing function for the polypeptide skeleton is a value within a range of 1 to 70 when a molecular weight of the polypeptide skeleton is 100.

FIG. 1

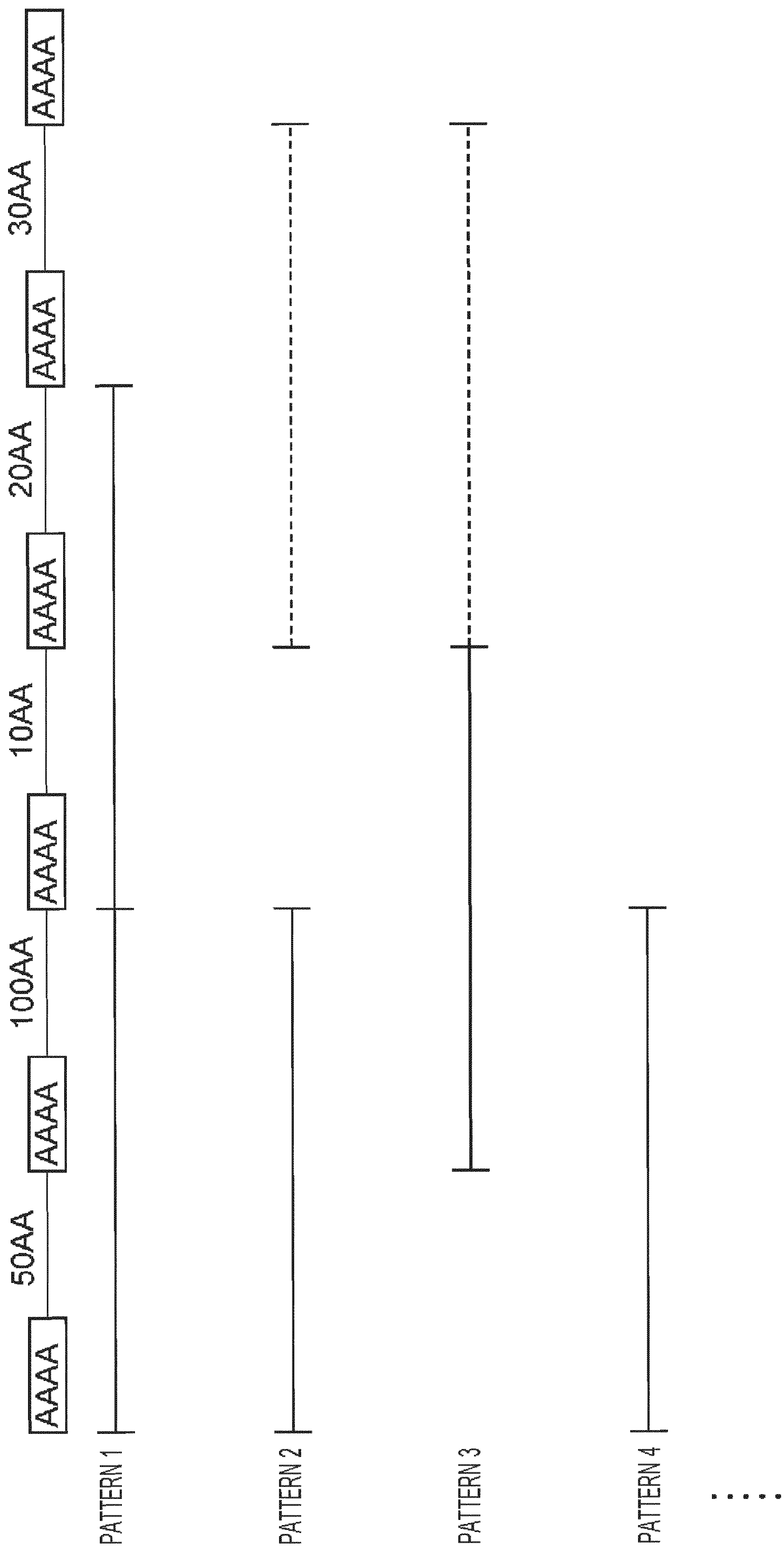
AAAA
50AA
AAAA
100AA
AAAA
10AA
AAAA
20AA
AAAA
30AA
AAAA
PATTERN 1
PATTERN 2
PATTERN 3
PATTERN 4
.....

FIG. 2

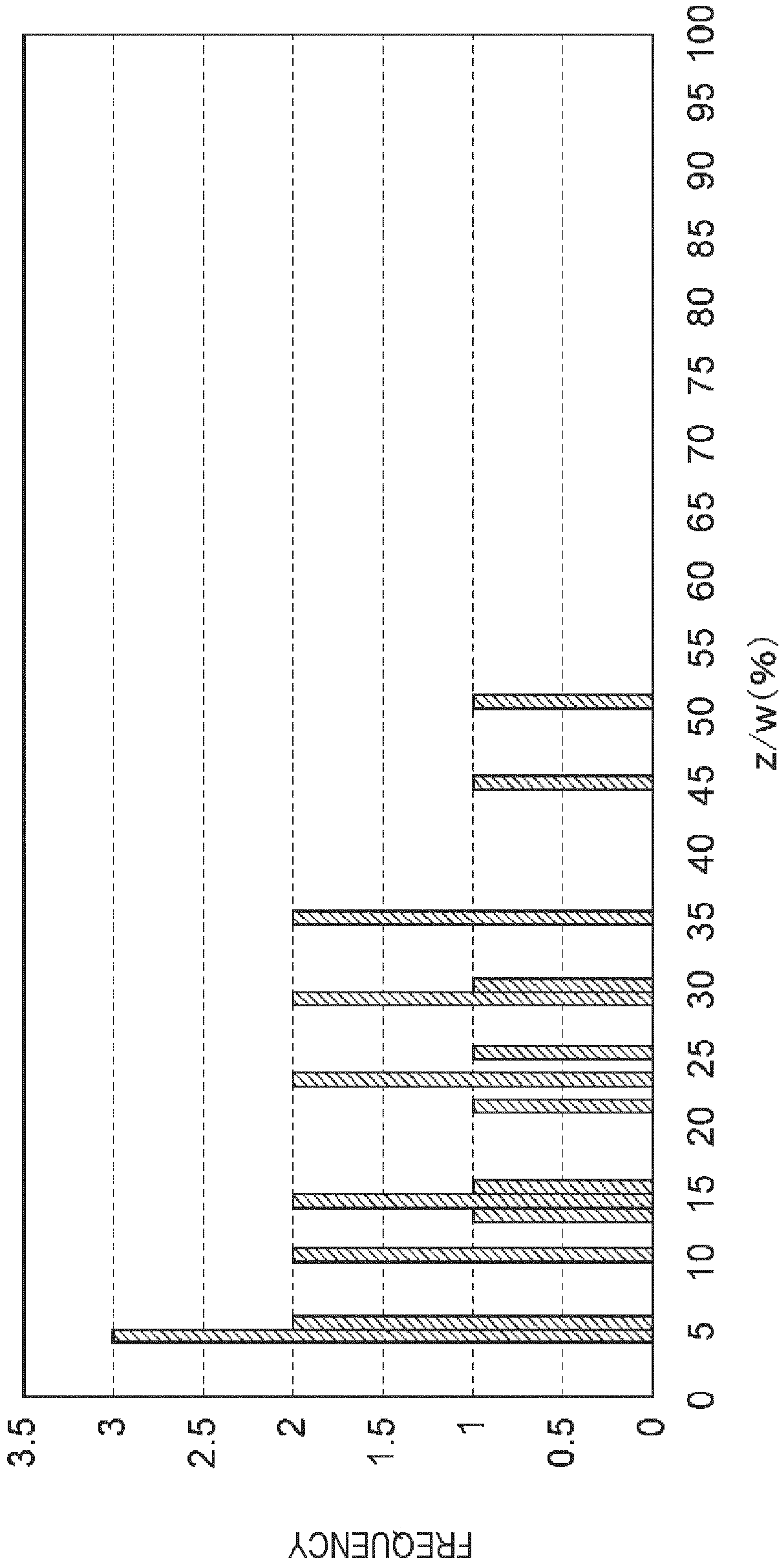
FREQUENCY
0
0.5
1
1.5
2
2.5
3
3.5
z/w(%)
0
5
10
15
20
25
30
35
40
45
50
55
60
65
70
75
80
85
90
95
100

FIG. 3

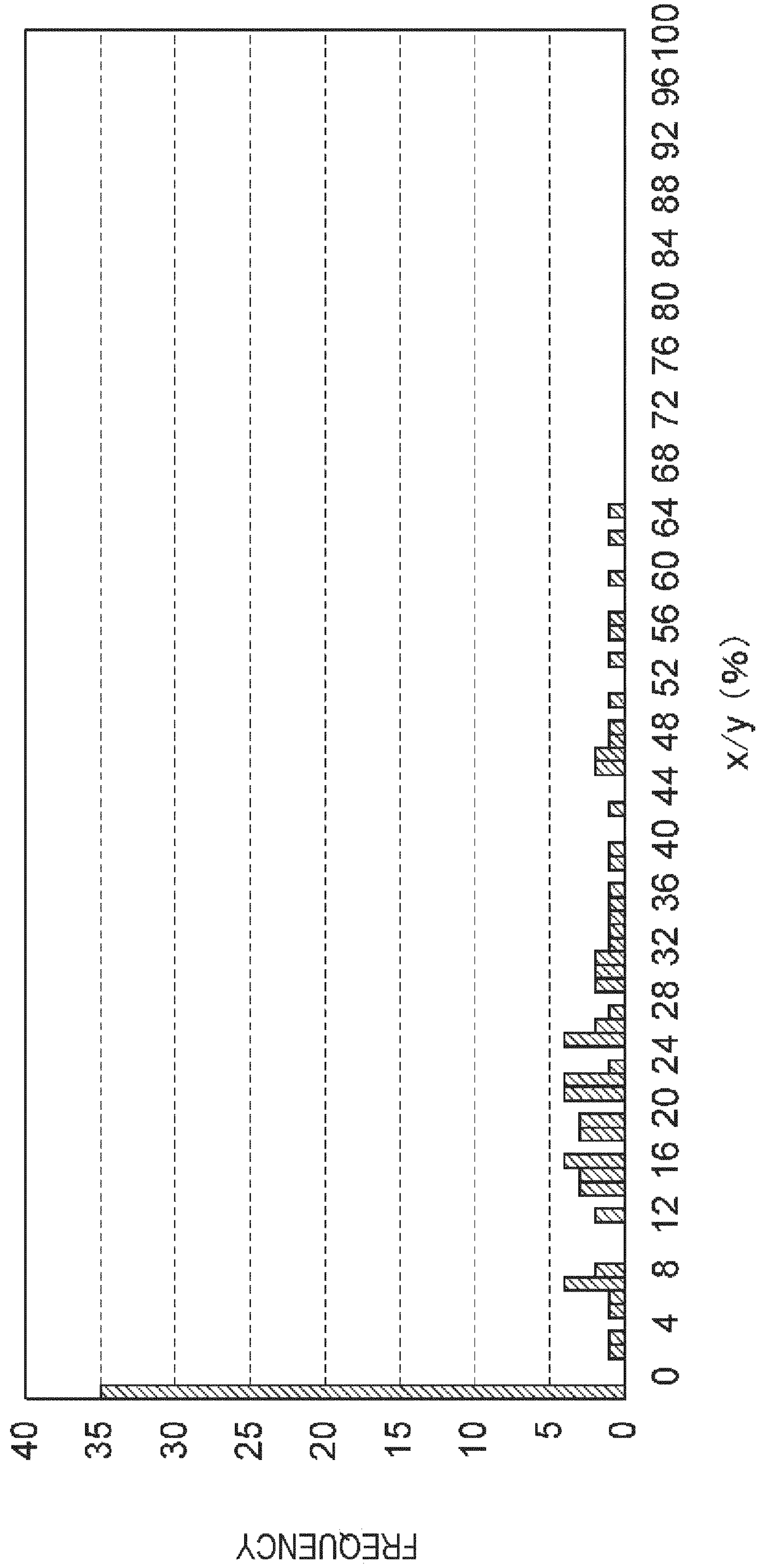
FREQUENCY
0
5
10
15
20
25
30
35
40
x/y (%)
0 4 8 12 16 20 24 28 32 36 40 44 48 52 56 60 64 68 72 76 80 84 88 92 96 100

FIG. 4

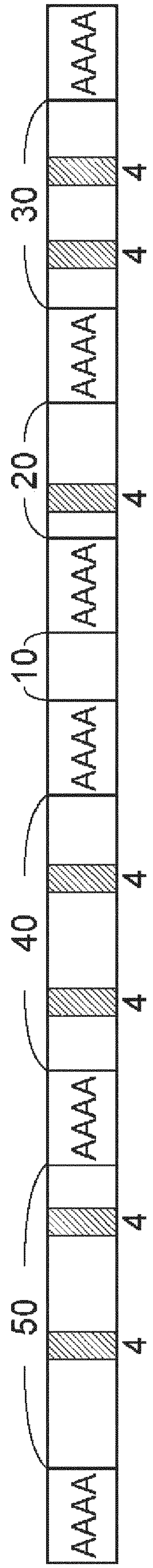

FIG. 5

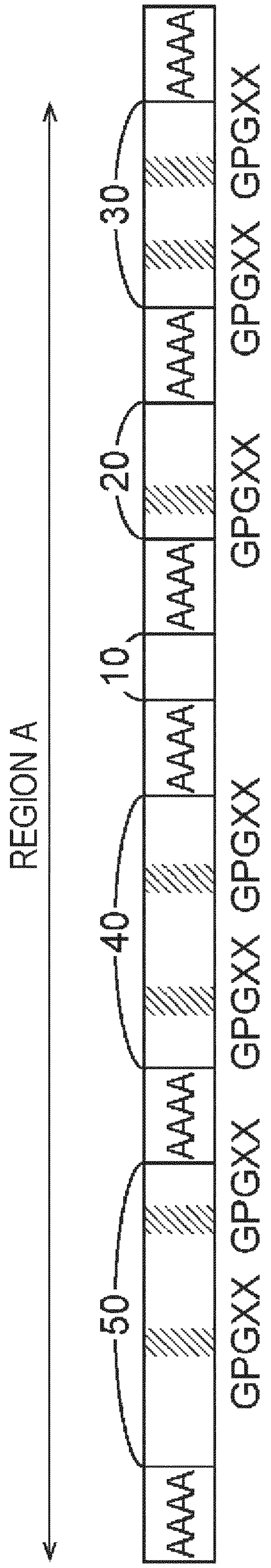
REGION A
50
40
10
20
30
AAAA
GPGXX GPGXX
AAAA
GPGXX GPGXX
AAAA
AAAA
GPGXX
AAAA
GPGXX GPGXX
AAAA

FIG. 6

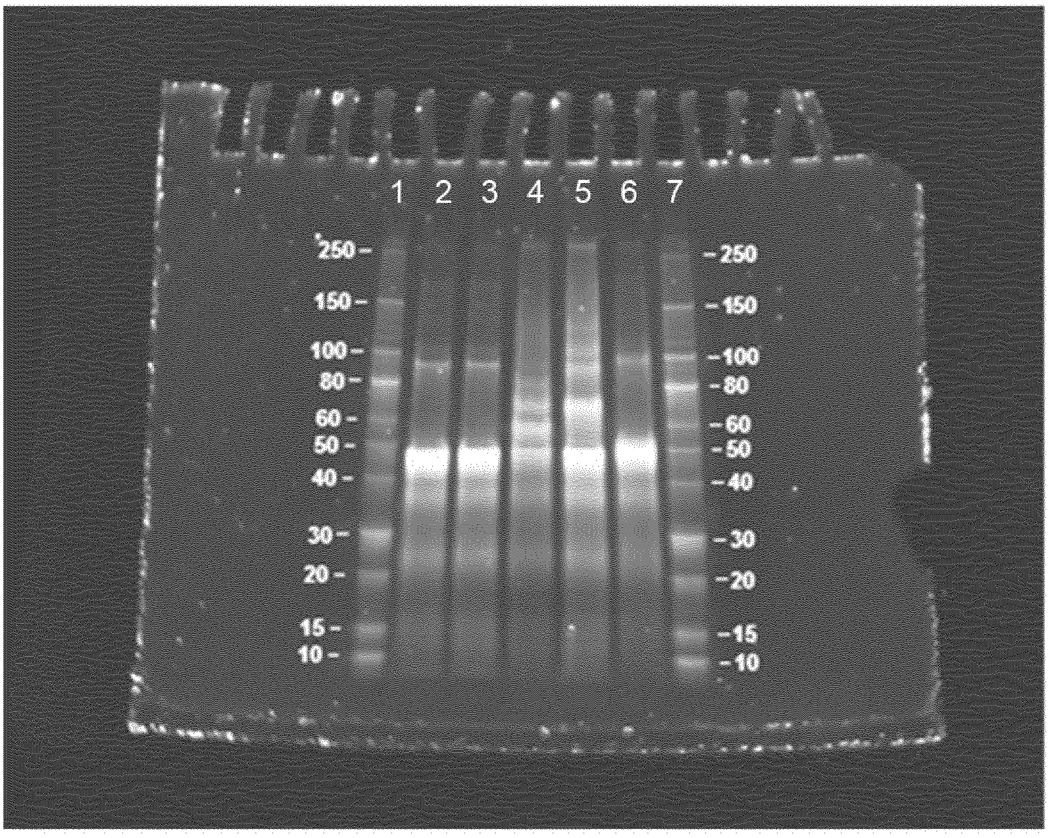
1
2
3
4
5
6
7
250
150
100
80
60
50
40
30
20
15
10
250
150
100
80
60
50
40
30
20
15
10

FIG. 7

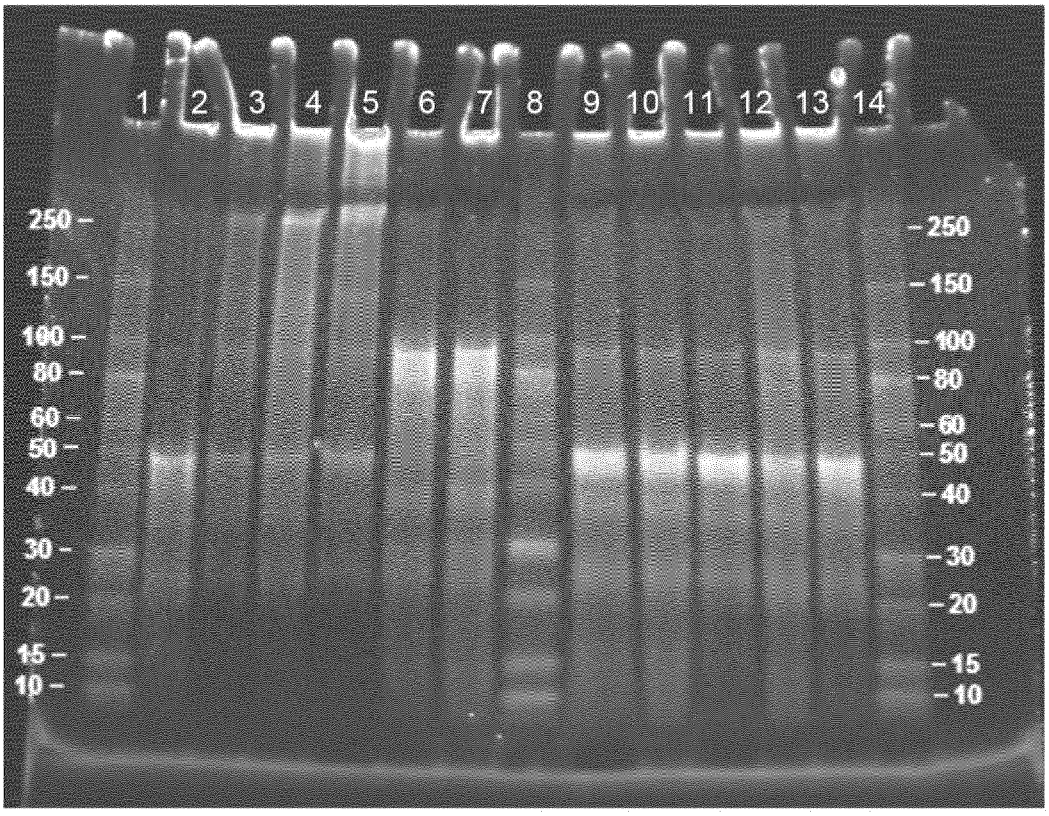
1
2
3
4
5
6
7
8
9
10
11
12
13
14
250 -
150 -
100 -
80 -
60 -
50 -
40 -
30 -
20 -
15 -
10 -
- 250
- 150
- 100
- 80
- 60
- 50
- 40
- 30
- 20
- 15
- 10

FIG. 8

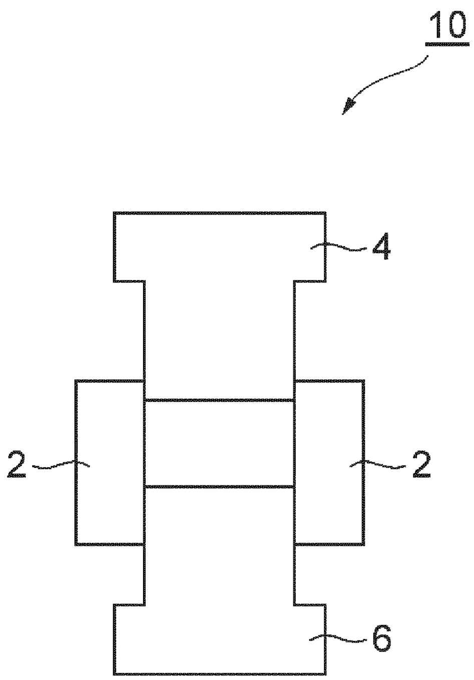

FIG. 9

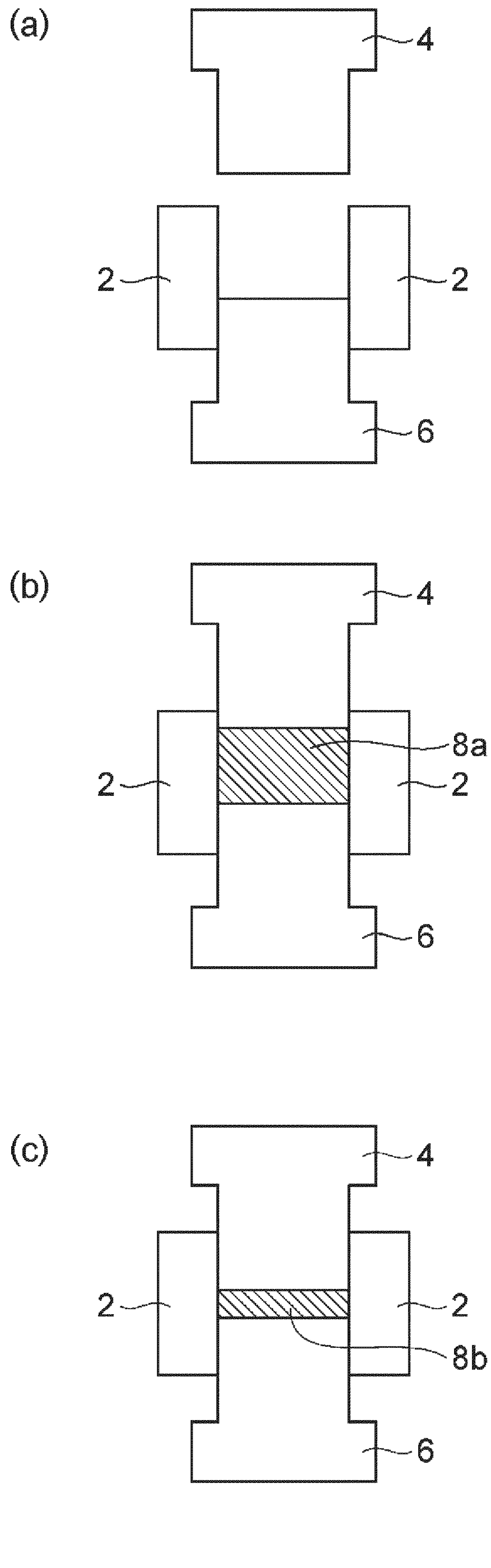

FIG. 10

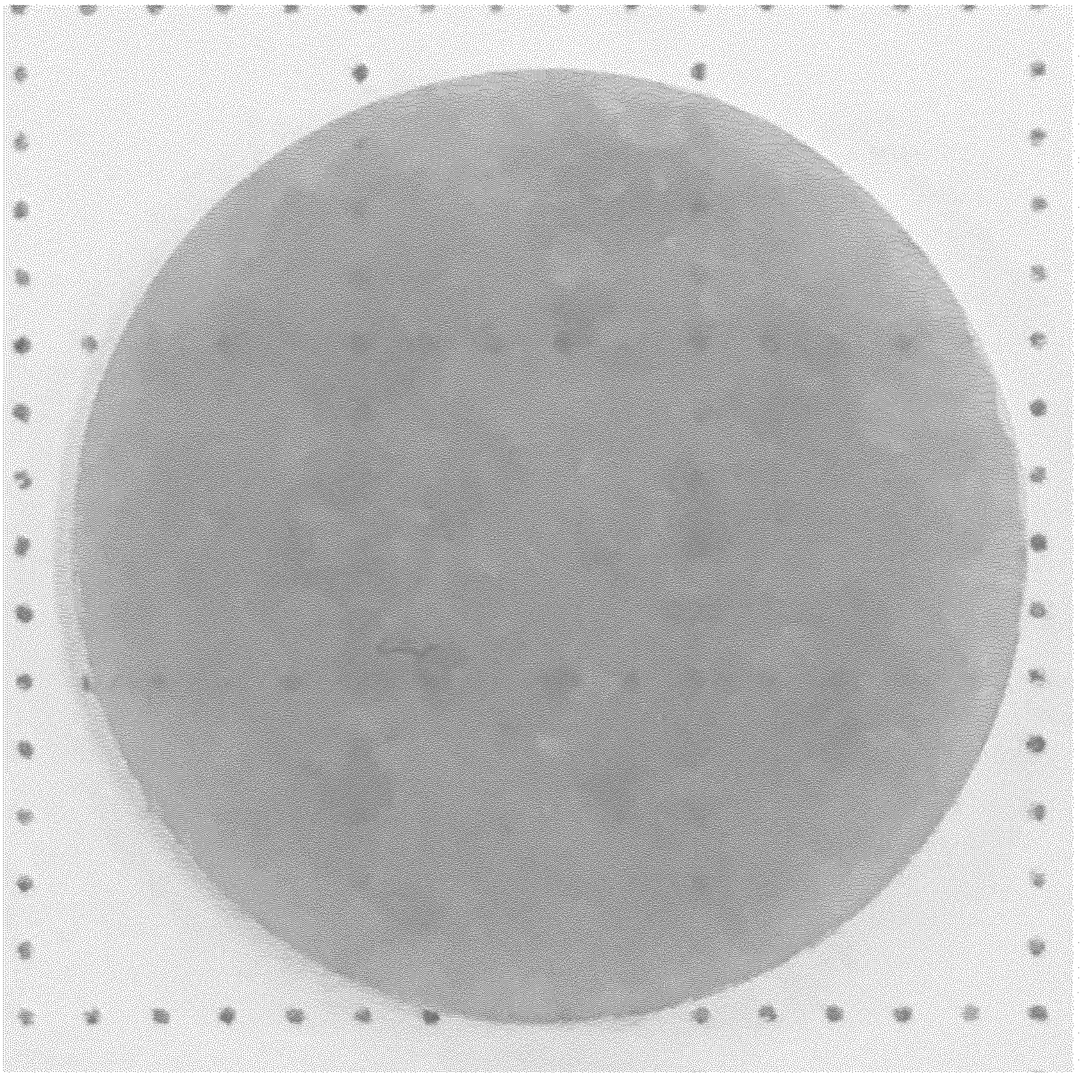

FIG. 11

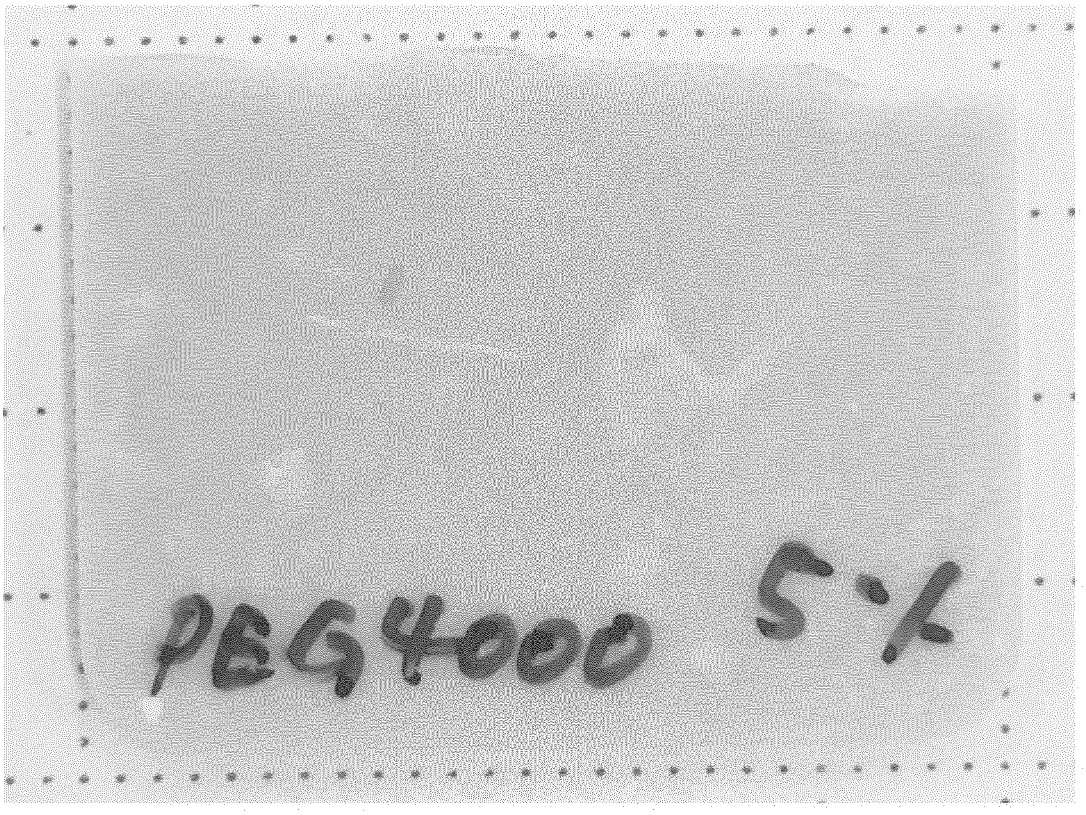

FIG. 12

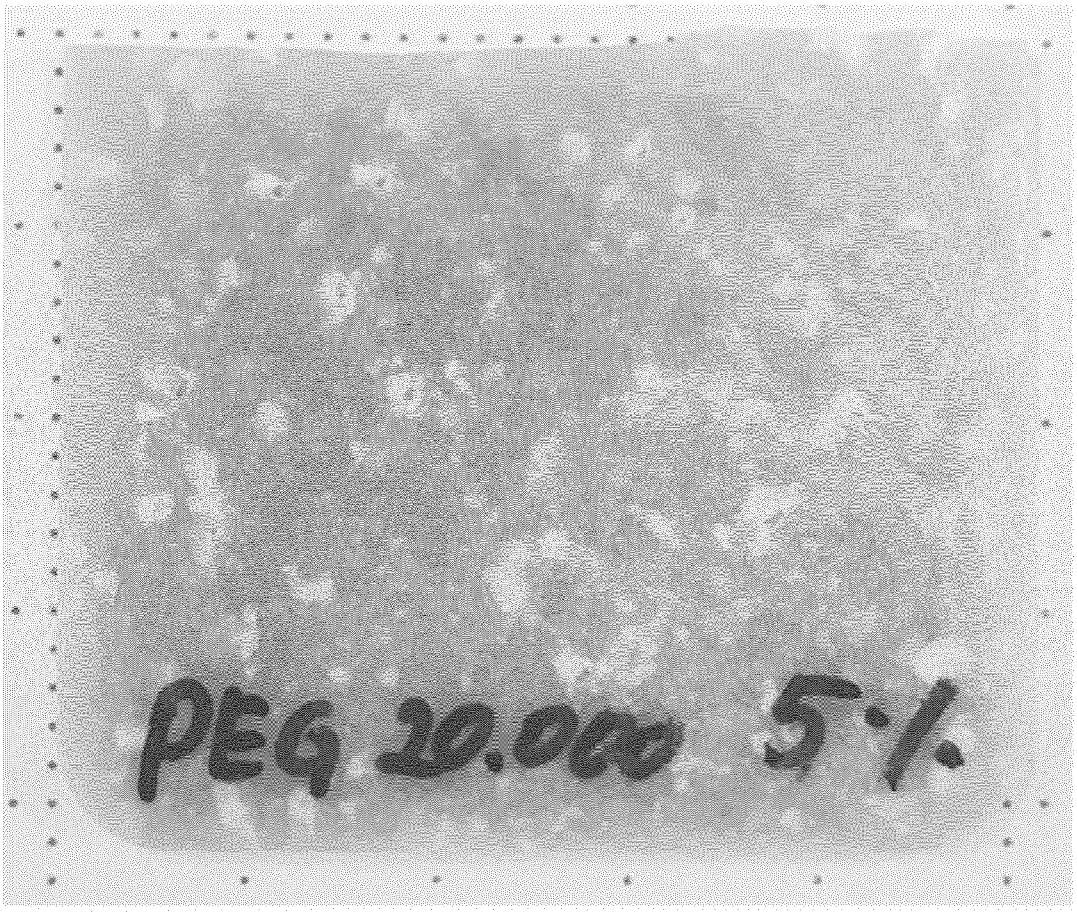

FIG. 13

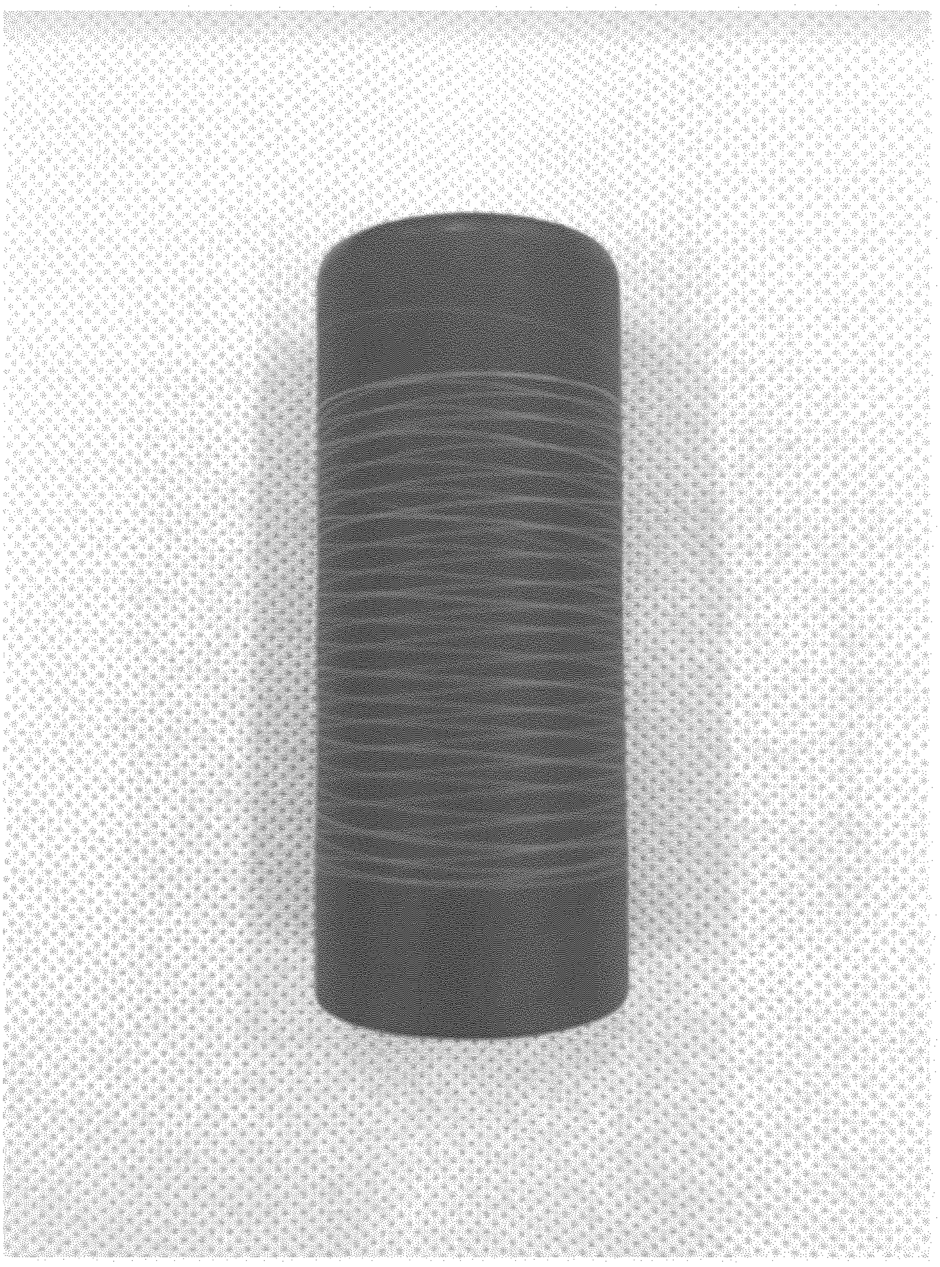

FIG. 14

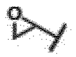 EPOXIDE

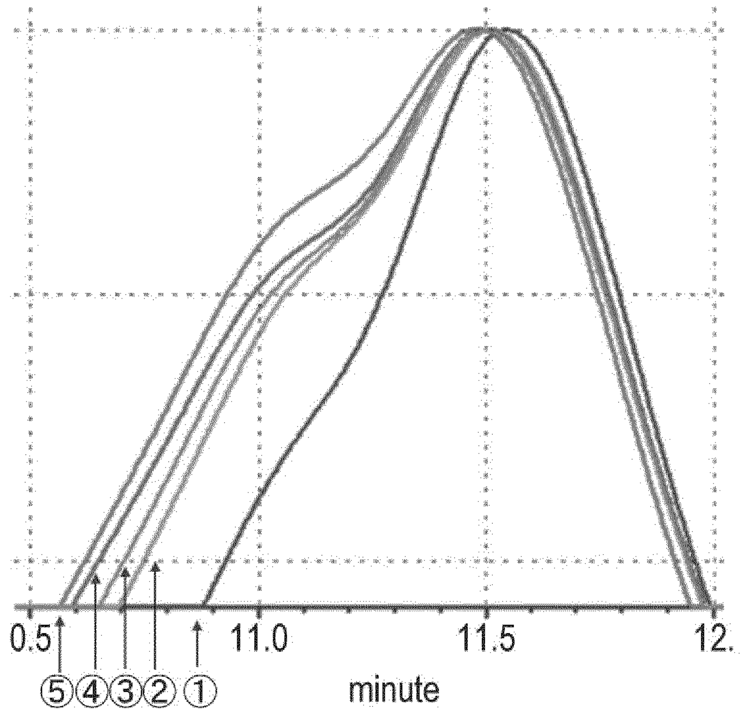


| | REACTION TEMPERATURE, TIME |
|---|---|
| ① | CONTROL |
| ② | 50°C, overnight |
| ③ | 80°C, overnight |
| ④ | 50°C, 3h |
| ⑤ | 80°C, 3h |

FIG. 15

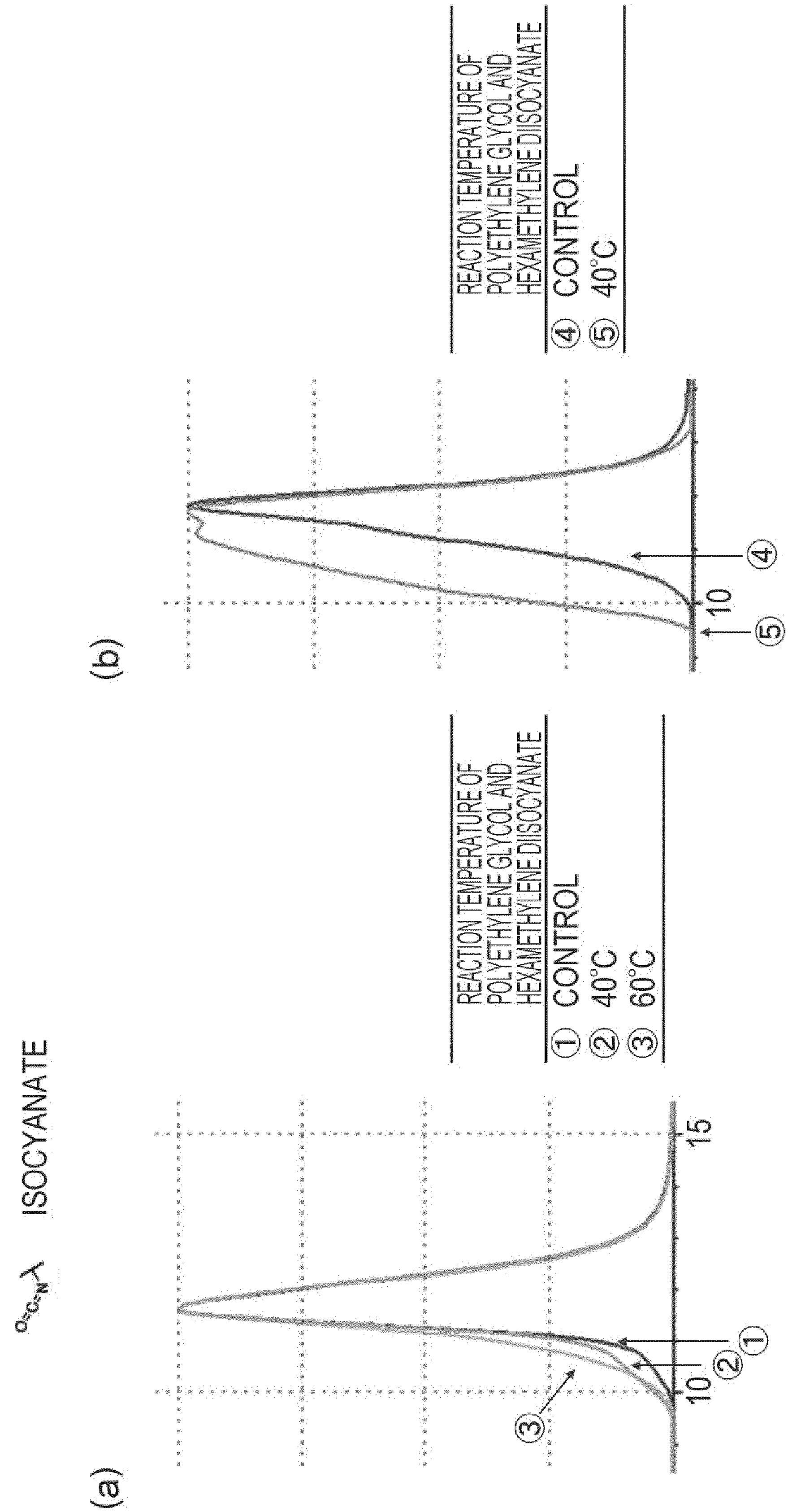
(a)
ISOCYANATE
10
15
①
②
③
REACTION TEMPERATURE OF POLYETHYLENE GLYCOL AND HEXAMETHYLENE DIISOCYANATE
① CONTROL
② 40°C
③ 60°C
(b)
10
④
⑤
REACTION TEMPERATURE OF POLYETHYLENE GLYCOL AND HEXAMETHYLENE DIISOCYANATE
④ CONTROL
⑤ 40°C

# FIG. 16

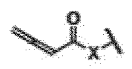 ALLENYL ESTER · AMIDE DERIVATIVE

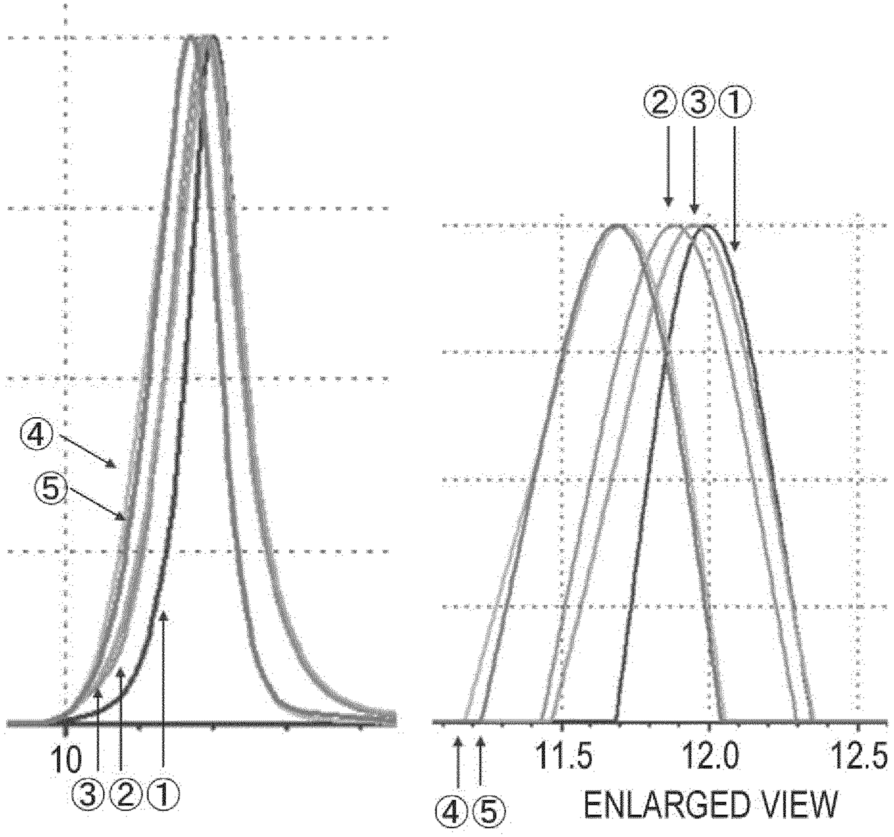


| | REACTION TEMPERATURE, SOLVENT | TEA |
|---|---|---|
| ① | CONTROL | |
| ② | 80°C, DMSO | 0.22μL |
| ③ | 25°C, DMF/2%LiCl | 0.22μL |
| ④ | 80°C, DMF/2%LiCl | 0.22μL |
| ⑤ | 25°C, DMF/2%LiCl | 1.67 μL |

FIG. 17

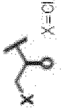
X=Cl, Br, I

A-HALOCARBONYL DERIVATIVE

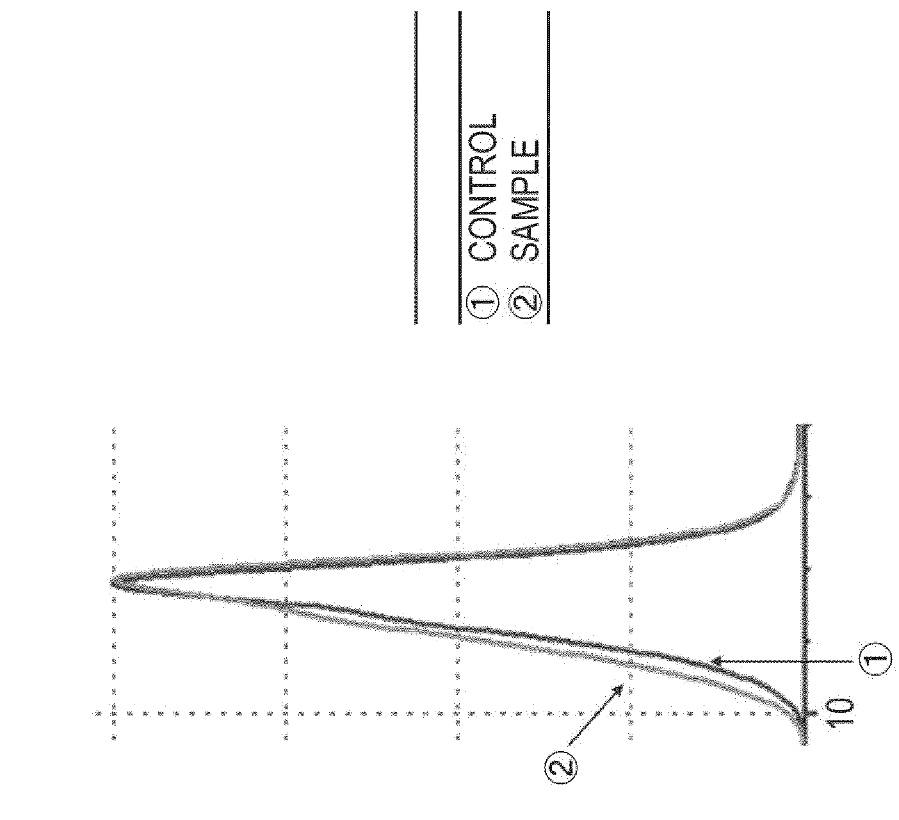

# FIG. 18

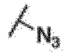 AZIDE (INDUCTION FROM SH, NH2, OH, AND THE LIKE) 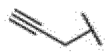 ALKYNE 〈Huisgen REACTION〉

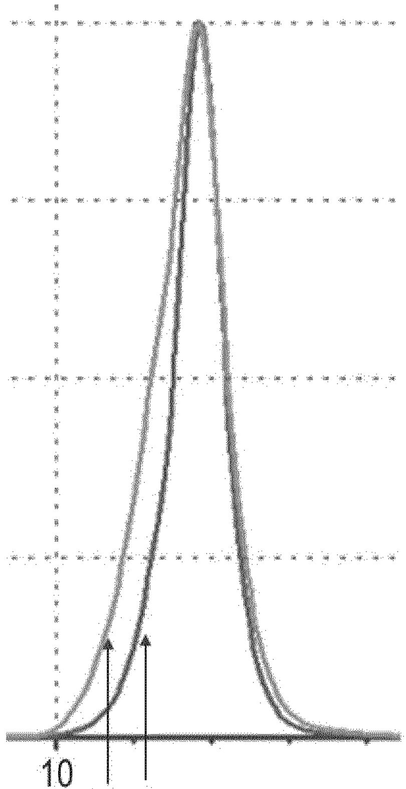


① CONTROL
② SAMPLE

FIG. 19

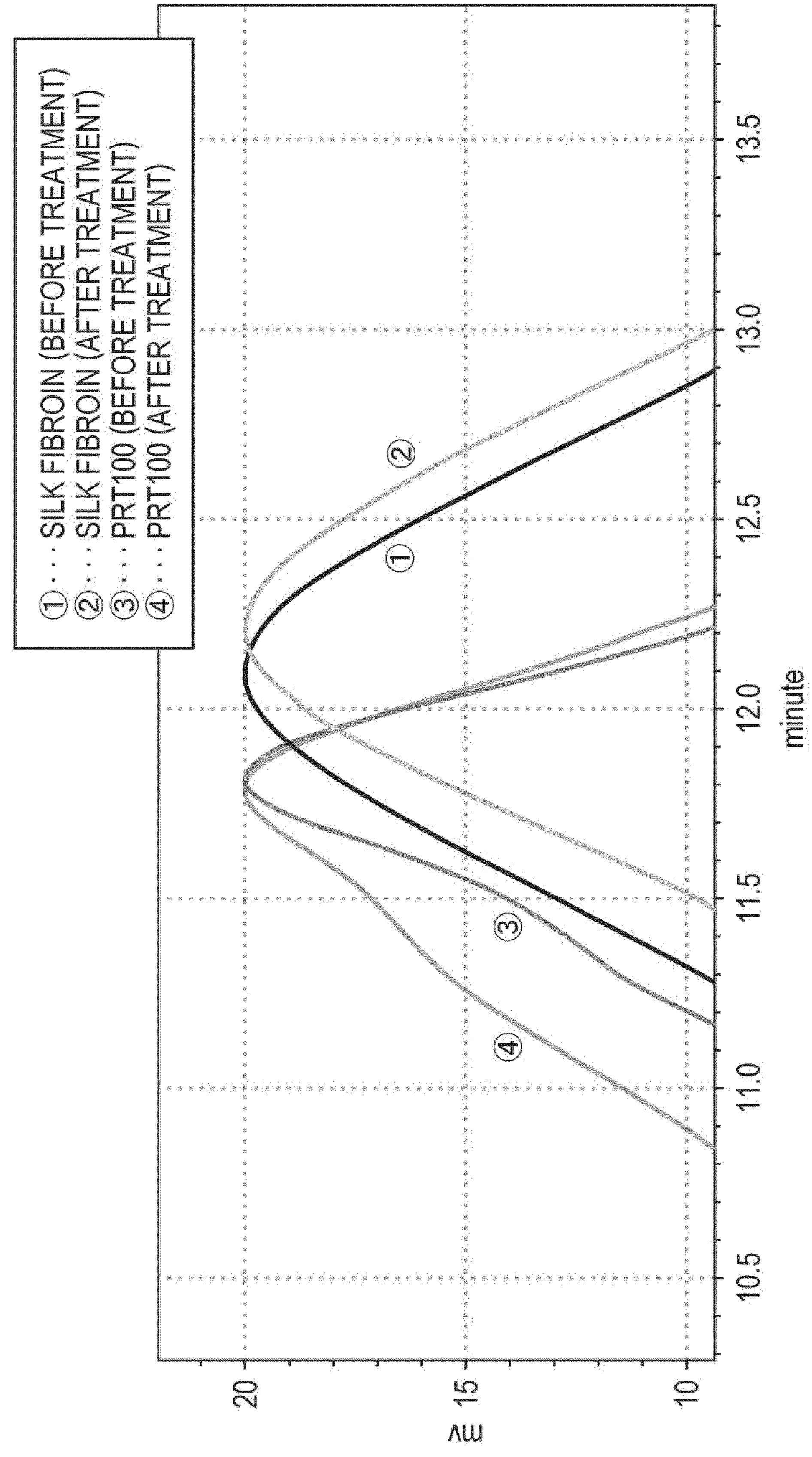

① · · · SILK FIBROIN (BEFORE TREATMENT)
② · · · SILK FIBROIN (AFTER TREATMENT)
③ · · · PRT100 (BEFORE TREATMENT)
④ · · · PRT100 (AFTER TREATMENT)
①
②
③
④
mV
20
15
10
10.5
11.0
11.5
12.0
12.5
13.0
13.5
minute

FIG. 20

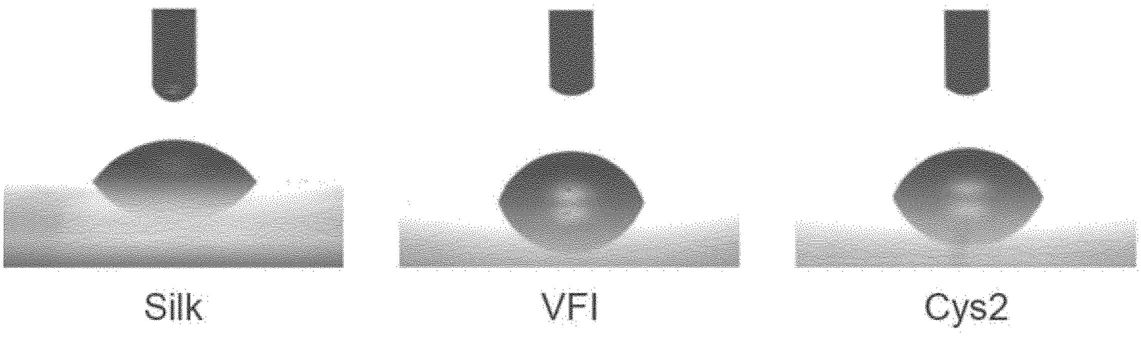

**INTERNATIONAL SEARCH REPORT**

International application No.
PCT/JP2021/010699

A. CLASSIFICATION OF SUBJECT MATTER
C07K 14/435(2006.01)i; C08G 81/00(2006.01)i; C08H 1/00(2006.01)i; C08L 89/00(2006.01)i; C08L 101/16(2006.01)i; C12N 15/12(2006.01)i; C12P 21/02(2006.01)i
FI: C07K14/435 ZNA; C12P21/02 C; C12N15/12; C08H1/00; C08L89/00; C08L101/16; C08G81/00

According to International Patent Classification (IPC) or to both national classification and IPC

B. FIELDS SEARCHED

Minimum documentation searched (classification system followed by classification symbols)
C07K14/435; C08G81/00; C08H1/00; C08L89/00; C08L101/16; C12N15/12; C12P21/02

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
CAplus/MEDLINE/EMBASE/BIOSIS(STN), JSTPlus/JMEDPlus/JST7580 (JDreamIII), UniProt/GeneSeq, PubMed, Japio-GPG/FX

C. DOCUMENTS CONSIDERED TO BE RELEVANT

| Category* | Citation of document, with indication, where appropriate, of the relevant passages | Relevant to claim No. |
|---|---|---|
| X | JP 2013-536737 A (TRUSTEES OF TUFTS COLLEGE) 26 September 2013 (2013-09-26) claims 2-3, example 1, paragraphs [0035], [0046], [0049] | 1-2, 4-21, 23-27, 29 |
| Y | claims 2-3, example 1, paragraphs [0035], [0046], [0049] | 1-29 |
| Y | WO 2019/022163 A1 (SPIBER INC., RIKEN) 31 January 2019 (2019-01-31) claims 1, 29, examples, paragraphs [0034], [0147] | 1-29 |
| A | JP 2009-505668 A (TECHNISCHE UNIVERSITÄT MÜNCHEN) 12 February 2009 (2009-02-12) entire text, all drawings | 1-29 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 May 2021 (27.05.2021) | 08 June 2021 (08.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2021/010699

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-169369 A (FUJI XEROX CO., LTD.) 29 June 2006 (2006-06-29) entire text, all drawings | 1-29 |
| A | ZHANG, H. et al., "Microbial production of amino acid-modified spider dragline silk protein with intensively improved mechanical properties.", Preparative Biochemistry and Biotechnology, 2016, vol. 46, no. 6, pp. 552-558, entire text, all drawings | 1-29 |

Form PCT/ISA/210 (continuation of second sheet) (January 2015)

**INTERNATIONAL SEARCH REPORT**
Information on patent family members

International application No.
PCT/JP2021/010699

| Patent Documents referred in the Report | Publication Date | Patent Family | Publication Date |
|---|---|---|---|
| JP 2013-536737 A | 26 Sep. 2013 | WO 2012/031144 A2<br>claims 2-3, example 1, paragraphs [0042], [0053], [0056]<br>US 2013/0287742 A1<br>EP 2611473 A1 | |
| WO 2019/022163 A1 | 31 Jan. 2019 | US 2020/0207817 A1<br>claims 1, 29, examples, paragraphs [0035], [0148]<br>EP 3660036 A1 | |
| JP 2009-505668 A | 12 Feb. 2009 | WO 2007/025719 A1<br>entire text, all drawings<br>US 2009/0123967 A1<br>EP 1919938 A1 | |
| JP 2006-169369 A | 29 Jun. 2006 | US 2006/0128943 A1<br>entire text, all drawings | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015178466 A
- JP S63248394 A
- JP S58110600 A
- JP S60221091 A
- JP 60221091 A
- US 4686191 A
- US 4939094 A
- US 5160735 A
- JP H231682 A
- JP H4278091 A
- JP H7170984 A
- JP H6133782 A
- JP 2002238569 A
- JP H2227075 A
- JP 5336963 A
- WO 9423021 A


### Non-patent literature cited in the description


- **KYTE J ; DOOLITTLE R.** A simple method for displaying the hydropathic character of a protein. *J. Mol. Biol.,* 1982, vol. 157, 105-132
- *Proc. Natl. Acad. Sci. USA,* 1972, vol. 69, 2110
- *Gene,* 1982, vol. 17, 107
- *Molecular & General Genetics,* 1979, vol. 168, 111
- **TAKAHASHI et al.** *J. Bacteriol.,* 1983, vol. 156, 1130-1134
- **TAKAGI et al.** *Agric. Biol. Chem.,* 1989, vol. 53, 3099-3100
- **OKAMOTO et al.** *Biosci. Biotechnol. Biochem.,* 1997, vol. 61, 202-203
- *Agric. Biol. Chem.,* 1984, vol. 48, 669
- *Agric. Biol. Chem.,* 1989, vol. 53, 277
- *Proc. Natl. Acad. Sci. USA,* 1985, vol. 82, 4306
- *J. Bacteriol.,* 1990, vol. 172, 2392
- *J. Bacteriol.,* 1987, 1239-1245
- **SHIGEZO UDAKA.** *Journal of the Agricultural Chemical Society of Japan,* 1987, vol. 61, 669-676
- *Appl. Microbiol. Biotechnol.,* 1989, vol. 30, 75-80
- *J. Biochem.,* 1992, vol. 112, 488-491
- *Appl. Environ. Microbiol.,* 1992, vol. 58, 525-531
- *J. Bacteriol.,* 1995, vol. 177, 745-749
- *Appl. Microbiol. Biotechnol.,* 1994, vol. 42, 358-363
- **MYERS, A. M. et al.** *Gene,* 1986, vol. 45, 299-310
- *Methods Enzymol.,* 1990, vol. 194, 182
- *Proc. Natl. Acad. Sci., USA,* 1984, vol. 81, 4889
- *J. Bacteriol.,* 1983, vol. 153, 163
- *Proc. Natl. Acad. Sci. USA,* 1978, vol. 75, 1929
- **COHEN et al.** *Proc. Natl. Acad. Sci. USA,* 1972, vol. 69, 2110
- *Mol. Gen. Genet.,* 1979, vol. 168, 111
- *J. Mol. Biol.,* 1971, vol. 56, 209
- Baculovirus Expression Vectors, A Laboratory Manual. W. H. Freeman and Company, 1992
- Current Protocols in Molecular Biology, Baculovirus Expression Vectors, A Laboratory Manual. W. H. Freeman and Company, 1992
- *Bio/Technology,* 1988, vol. 6, 47
- *Proc. Natl. Acad. Sci. USA,* 1987, vol. 84, 7413
- *Biosci. Biotechnol. Biochem.,* 1995, vol. 59, 1795-1797
- *Enzyme Microbiol Technol,* 1984, vol. 6, 386-389
- *Gene,* 1989, vol. 84, 329-334
- *BiosciBiotechnol Biochem,* 2000, vol. 64, 1416-1421
- *BiochemBiophys Res Commun,* 1983, vol. 112, 284-289
- *Gene,* 1983, vol. 26, 205-221
- *Mol Gen Genet,* 1999, vol. 261, 290-296
- *Proc Natl Acad Sci USA,* 1986, vol. 83, 4869-4873
- *Gene,* 1987, vol. 57, 21-26
- Molecular Cloning
- *Nature,* 1962, vol. 195, 788
- **PAULSON et al.** *J. Biol. Chem.,* 1989, vol. 264, 17619
- **LOWE et al.** *Proc. Natl. Acad. Sci. USA,* 1989, vol. 86, 8227
- *Genes Develop.,* 1990, vol. 4, 1288